# EUROPEAN PATENT APPLICATION

(11) **EP 4 018 821 A1**
(43) Date of publication of application: **29.06.2022**
(21) Application number: 20216347.3
(22) Date of filing: 22.12.2020
(51) Int. Cl.: A01H 1/00, A01H 5/00, A01H 6/46, C12N 15/82

(54) **METHODS FOR IDENTIFYING AND SELECTING MAIZE PLANTS WITH CYTOPLASMATIC MALE STERILITY RESTORER GENE**

(71) Applicant: KWS SAAT SE & Co. KGaA, 37574 Einbeck (DE)
(72) Inventor: KLOIBER-MAITZ, Monika, 37574 Einbeck (DE); KNAAK, Carsten, 37079 Göttingen (DE); DALL'OCCHIO, Hervé, 11170 Caux-et-Sauzens (FR); CASTELLE, Jean-Claude, 11170 Alzonne (FR)
(74) Representative: De Clercq & Partners

(57) **Abstract**

The present invention relates to a method for identifying a plant or plant part, such as a maize plant or plant part, comprising a novel restorer of fertility locus, in particular a cytoplasmic male sterility restorer of fertility locus. The invention further relates to molecular markers associated with such locus and the use of such markers in the identification of plants. The invention further relates to methods for generating plants or plant parts comprising the novel restorer of fertility locus.

SEQUENCE LISTING

<110>  KWS SAAT SE & Co. KGaA

<120>  METHODS FOR IDENTIFYING AND SELECTING MAIZE PLANTS WITH CYTOPLASMATIC
MALE STERILITY RESTORER GENE

<130>  KWS-024-EP-PRIO

<160>  208

<170>  PatentIn version 3.5

<210>  1
<211>  3221
<212>  DNA
<213>  Zea mays


<220>
<221>  misc_feature
<222>  (1547)..(1573)
<223>  n is a, c, g, or t

<400>  1
   agtggtacgg tggggtcgcg gagcttgcca gcttggtggc tgcaactcgc cagcctgatg      60
   gggcggcttc gcgcgtgctg ccgtctccac cgcttcctcg cgccgccgcc ggcacaagtg     120
   ccatcaccgg gacaatctct ggcccgcggg tccaacaccg ctaccgctca cgtagcgcct     180
   ttctcgaggc ttctctcgtc ggccgcggcc gttgcgcccc acgacacacg cgactcgggc     240
   ctcggcggtt cggcgtactg ggcctggatt cgcgcggcga ccgagtcggc cccggctcct     300
   tcgccgcccc aggaggagga ggacgagggc ccagcacgct acatccccgt caaagcctac     360
   ttcctctcca ccaggtaagc acgcagcgcc gctcccgcgc ttgttcgata ggtgtttgat     420
   gatttatcat cgaggaaaca ttcgggactt tcgggtgcta ccctactatt ctaaaaatgt     480
   ccatgccaat tggcactgcg aactaaaacg acgtgttttc aatttttcct cctcgcggtg     540
   acagcattga tctgaagagc atgcaagcgg agcacgggaa tgatattgta cctccgtcga     600
   cccgtttgct taactacatt gcgcttcggt actccgagtt cccacctgag attatggtta     660
   gttattacag tttagcgtca cttctttttt gacctgaaat tcatatcaaa atgaacaaat     720
   ctcagtcagc tttggaccag ttgcacatac tttgtaacat tgacctatgc tacgaattga     780
   tttccttgtg attttgattg atatttatgc agaacattgg agtgaaggat aacagatttt     840
   gctatcgcta tgtggttgtt ttccagtatg gttctgctgt gcttttcaac attgctgatc     900
   atgaagccga gtactatctt gatataatta ggaagcatgc ttcaggatgg cttccagaga     960
   tgagaaaaga tggtatgctc actgctaaac tgcgtcctgt ttactgcttt ttactggcct    1020
   ccatttttgt gttccttaat aatatttgca tggacagaaa acagtcagtt gccttctgtt    1080
   atattcaaag atgctatttg ttaactgtga tagaactcaa cttaaagaga attgttgcat    1140
   gcctagactg tgtgttttgt gcatttttgt ttagcagaaa taagttgcta ctgcttttcg 1200
   tccactgtga caaagcatat gccccctgga attgaactgt ttgactcttt gtaaagatgc 1260
   aggtttagag taagaccaac atcagctgat gccatttatt ttgcatgaac agattatgca 1320
   gtggttgaga aaccatcctt gacaacatgg atgaaaggag gacttgatta tatagttctt 1380
   aaaagtttag atacagacgg gattcgcata atttcaagcg ttcttggtca aagtatcgcc 1440
   cttgatcact atattcggca ggtactgaaa ctcaaagtta atgaacttat gtgttgtcac 1500
   agtatcatta aagctttgtc taaaagttct gaacttcaaa attatcnnnn nnnnnnnnnn 1560
   nnnnnnnnnn nnnttcactg aaattaatcg tgtcatggag aaaaccggta actttaccat 1620
   gcaaagaaaa aaactctttc aacttgtggg taaggccaat tctaatcttg cggatgttat 1680
   catcagactt ggtctttttg acaggtatgt ctactacact caagtactat acttcctatt 1740
   taggatggtt ccatttcttg tacaacatgt gtgaagttaa atattttatg gatcaaagaa 1800
   tgtgtcaatc ccatgtggag gattgtgaag gtgtgtgatt caatttgcta gacattgcac 1860
   gtctataagc cactgttatg cgtacatttg atattccctt ttggggttca catgtgggtt 1920
   ttgctgtttg tttggaaatt cttccaatac tttgaaaaaa tattgatgat tttgatcatt 1980
   gtacttagaa attagacatc actctatcac tatatcacaa tattaactat ttttataagt 2040
   attgtgtttg ctatccttgg agtatatcat attctcgttg gcttggctgc atgcatcccc 2100
   atgtttctgc tttgactgtc ttgttgcttt tatagtgcaa ttttatgtct tgattcctgc 2160
   ctattttttc cttttctttt ttgcttctcg atacattcac ctactgtcat ttggccatgt 2220
   tttttgctag gtcagaaatt gcttggaaaa atgcaaatta tgcacaaatt ctagaatatc 2280
   ttcgggagga atatgaactg aatcagcgtt ttggaagcct tgacttcaaa ctgaaatttg 2340
   tggaggtagt gctttgtgta ttcattctcc tatagtgcaa aagttgggtt ctttatcttt 2400
   caagttctca tttgcgctta cgatttatta ttgtaacagc ataacattca ctttcttcaa 2460
   gaggtcctcc aaaacagacg gtctgatctg ctggaatggg gtgtcataat actgctgatt 2520
   attgagattg ctatctcatt atatgaaatt gccaaggact ccatgatcga ttgagagctc 2580
   tcaacaacac ctctattcat cctcgatcat cccattatgc taactccaga ttactgtttt 2640
   aacgaaaaaa aggaaaagaa tgaccccagc ttccatattt tctgactatc catcttcacc 2700
   ctttggattt ctaaccttag atgaaaggaa gttgggactg gcgttctggc ttgagtttat 2760
   ttacatcctt taattctaca actccaaacg aaaagaagtt tggccaattt tcctatgtcc 2820
   aatgagttcc cgggcagcca gggttttcta atttgagtag aatctagttt tcctttcaat 2880
   attcatgcca ggtgttcttg atacatgttc ttggtgccac tttttagtat cttgatagga 2940
   ctgtcagctc tttttttggg ggcaaggctg acgccaggca gctgcaactg gacctgatgg 3000
   aaagatcatg aaagttttta tcaaatggct ttgtaatttg tacaaaaact tagcgtggga 3060
   aggttccctt tggtttaacc atactgaatt gctcgttcca ttttgcgtgg taattaacca 3120
   tgcatactgt aagtatgtgt agtacacttg atgcctatga gtgagagtga attagatatt 3180
   tcttctagtc aaaatctatg caataaataa actatctatg t 3221

<210> 2
<211> 3214
<212> DNA
<213> Zea mays

<400> 2
agtggtacgg tggggtcgcg gagcttgcca gctttgtggc tgcaactcgc cagcctgatg 60

gggcggcttc gcgcgtgctg ccgtctccac cgcttcctcg cgccgccgcc ggcacaagtg 120

ccatcaccgg gacaatctct ggcccgcggg tccaacaccg ctaccgctca cgtagcgcct 180

ttctcgaggc ttctctcgtc ggccgcggcc gttgcgcccc acgacacacg cgactcgggc 240

ctcggcggtt cggcgtactg ggcctggatt cgcgcggcga ccgagtcggc cccggctcct 300

tcgccgcccc aggaggagga ggacgagggc ccagcacgct acatccccgt caaagcctac 360

ttcctctcca ccaggtaagc acgcagcgcc gctcccgcgc ttgctcgata ggtgtttgat 420

gatttatcat cgaggaaaca ttcgggtgct accttactat tctaaaaatg tccatgccaa 480

ttggcactgc gaactaaaac gacgtgtttt caatttttcc tcctcgccgt gacagcattg 540

atctgaagag catgcaagcg gagcacggga atgatattgt acctccgtcg acccgtttgc 600

ttaactacat tgcgcttcgg tactccgagt tcccacctga gattatggtt agttattaca 660

gtttagcgtc acttcttttt tgacctgaaa ttcatatcaa aatgaacaaa tctcagtcag 720

ctttgaacca gttgcacata gacctatgct acgaattgat ttccttgtga ttttggttga 780

tatttatgca gaacattgga gtgaaggata acagattttg ctatcgctat gtggttgttt 840

tccagtatgg ttctgctgtg cttttcaaca ttgctgatca tgaagccgag tactatcttg 900

atataattag gaagcatgct tcaggatggc ttccagagat gagaaaagat ggtatgctca 960

ctgctaaact gcgtcctgtt tactgctttt tactggcctc catttttgtg ttccttaata 1020

atattttcat ggaccgaaaa cagtacagtt gccttctccc ttctgttata ttcaaagatg 1080

ctatttgtta actgtgatag aactcaactt aaagagaatt gttgcatgcc tagactgtgt 1140

gttttgtgca tttttgttta gcagaaataa gttgttactg cttttcgtcc actgtgacaa 1200

agcacatgcc ccctggaatt gaactgtttg actctttgta aagatgcagg tttagagtaa 1260

gaccaacatc agctgatgcc atttattttg catgaacaga ttatgcagtg gttgagaaac 1320

catccttgac aacatggatg aaaggaggac ttgattatat agttcttaaa agtttagata 1380

cagacgggat tcgcataatt tcaagcgttc ttggtcaaag tatcgccctt gatcactata 1440

ttcggcaggt actgaaactc aaagttaatg aacttatgtg ttgtcacagt atcattaaag 1500

ctttgtctaa aagttctgaa cttcaaaatt atcaattttg ataggtcgat gatatggttg 1560

aggaattcac tgaaattaat cgtgtcatgg agaaaaccgg taactttacc atgcaaagaa 1620

aaaaactctt tcaacttgtg ggtaaggcca attctaatct tgcggatgtt atcatcagac 1680

ttggtctttt tgacaggtat gtctactaca ctcaagtact atacttccta tttaggatgg 1740

ttccatttct tgtacagcat atgtgaagtt aaatatttta tggatcaaag aatgtgtcaa 1800

tcccatgtgg aggattgtga aggtgtgtga ttcaagttgc tagacatagc acgtctataa 1860

gccactgtta tgcgtacatt tgatattccc ttttggggtt cacatgtggg ttttgctgtt 1920

tgtttggaaa ttcttccaat actttgaaaa aatattgatg attttgatca ttgtacttag 1980

aaattagaca tcactctatc actatatcac aatattaact atttttataa gtattgtgtt 2040

tgctatcctt ggagtatatc atattcttgt tggcttggct acatgcatcc ccatgtttct 2100

gctttgactg tcttgttgct tttatagtgc aattttacgt cttgattcct gcctattttt 2160

ttccttttct tttttgcttc tcgatacatt cacctactgt catttgtcca tgttttttgc 2220

taggtcagaa attgcttgga aaaatgcaaa ttatgcacaa attctagaat atcttcggga 2280

ggaatatgaa ctgaatcagc gttttggaag ccttgacttc aaactgaaat ttgtggaggt 2340

agtgctttgt gtattcattc tctatagtgc aaaagttggg ttctttatct ttcaagttct 2400

catttgcgct tacgatttat tattgtaaca gcataacatt cactttcttc aagaggtcct 2460

ccaaaacaga cggtctgatc tgctggaatg gggtgtcata atactgctga ttattgagat 2520

tgctatctca ttatatgaaa ttgccaagga ctccatgatc gattgagagc tctgaacaac 2580

acctctattc atcctcgatc atcccattat gctaactcca gatccactgt tttaacaaaa 2640

aaaggaaaag aatgacccca gcttccatat tttctgacta tccatctcca ccctttggat 2700

ttctaacctt agatgaaagg aagttgggaa tggcgttctg gcttgagttt atttacatcc 2760

tttaattcta caactccaaa cgaaaagaag tttggccaat tttcctatgt ccaatgagtt 2820

cccgggcagc caggttttcc aatttgagta gaatctagtt ttcctttcaa tattcatgcc 2880

aggtgttctt gatacatgtt cttggtgcca ctttttagta tcttgatagg actgtcagct 2940

cttttttttt gggggcaagg ctgacgccag gcagctgcaa ctggacctga tggaaaaatc 3000

atgaaagttt ttatcaaatg gctttgtaat ttgtacaaat acttagcgtg ggaaggttcc 3060

atttggttta accatactga attgctcgtt ccattttgcg tggtaattaa ccatgcatac 3120

tgtaagtatg tgtagtacac ttgatgccta tgagtgagag tgaattagat atttcttcta 3180

gtcaaaatct atgcaataaa taaactatct gtgt 3214

<210> 3
<211> 1140
<212> DNA

<213>  Artificial Sequence

<220>
<223>  cDNA of Zm00001d043358 (genotype B73)

<400>  3
atggggcggc ttcgcgcgtg ctgccgtctc caccgcttcc tcgcgccgcc gccggcacaa      60

gtgccatcac cgggacaatc tctggcccgc gggtccaaca ccgctaccgc tcacgtagcg     120

cctttctcga ggcttctctc gtcggccgcg gccgttgcgc cccacgacac acgcgactcg     180

ggcctcggcg gttcggcgta ctgggcctgg attcgcgcgg cgaccgagtc ggccccggct     240

ccttcgccgc cccaggagga ggaggacgag ggcccagcac gctacatccc cgtcaaagcc     300

tacttcctct ccaccagcat tgatctgaag agcatgcaag cggagcacgg gaatgatatt     360

gtacctccgt cgacccgttt gcttaactac attgcgcttc ggtactccga gttcccacct     420

gagattatga acattggagt gaaggataac agattttgct atcgctatgt ggttgttttc     480

cagtatggtt ctgctgtgct tttcaacatt gctgatcatg aagccgagta ctatcttgat     540

ataattagga agcatgcttc aggatggctt ccagagatga gaaaagatga ttatgcagtg     600

gttgagaaac catccttgac aacatggatg aaaggaggac ttgattatat agttcttaaa     660

agtttagata cagacgggat tcgcataatt tcaagcgttc ttggtcaaag tatcgccctt     720

gatcactata ttcggcaggt cgatgatatg gttgaggaat tcactgaaat taatcgtgtc     780

atggagaaaa ccggtaactt taccatgcaa agaaaaaaac tctttcaact tgtgggtaag     840

gccaattcta atcttgcgga tgttatcatc agacttggtc tttttgacag gtcagaaatt     900

gcttggaaaa atgcaaatta tgcacaaatt ctagaatatc ttcgggagga atatgaactg     960

aatcagcgtt ttggaagcct tgacttcaaa ctgaaatttg tggagcataa cattcacttt    1020

cttcaagagg tcctccaaaa cagacggtct gatctgctgg aatggggtgt cataatactg    1080

ctgattattg agattgctat ctcattatat gaaattgcca aggactccat gatcgattga    1140


<210>  4
<211>  379
<212>  PRT
<213>  Zea mays

<400>  4

Met Gly Arg Leu Arg Ala Cys Cys Arg Leu His Arg Phe Leu Ala Pro
1               5                   10                  15


Pro Pro Ala Gln Val Pro Ser Pro Gly Gln Ser Leu Ala Arg Gly Ser
            20                  25                  30


Asn Thr Ala Thr Ala His Val Ala Pro Phe Ser Arg Leu Leu Ser Ser
        35                  40                  45

Ala Ala Ala Val Ala Pro His Asp Thr Arg Asp Ser Gly Leu Gly Gly
    50                  55                  60

Ser Ala Tyr Trp Ala Trp Ile Arg Ala Ala Thr Glu Ser Ala Pro Ala
65                  70                  75                  80

Pro Ser Pro Pro Gln Glu Glu Glu Asp Glu Gly Pro Ala Arg Tyr Ile
                85                  90                  95

Pro Val Lys Ala Tyr Phe Leu Ser Thr Ser Ile Asp Leu Lys Ser Met
            100                 105                 110

Gln Ala Glu His Gly Asn Asp Ile Val Pro Pro Ser Thr Arg Leu Leu
        115                 120                 125

Asn Tyr Ile Ala Leu Arg Tyr Ser Glu Phe Pro Pro Glu Ile Met Asn
    130                 135                 140

Ile Gly Val Lys Asp Asn Arg Phe Cys Tyr Arg Tyr Val Val Val Phe
145                 150                 155                 160

Gln Tyr Gly Ser Ala Val Leu Phe Asn Ile Ala Asp His Glu Ala Glu
                165                 170                 175

Tyr Tyr Leu Asp Ile Ile Arg Lys His Ala Ser Gly Trp Leu Pro Glu
            180                 185                 190

Met Arg Lys Asp Asp Tyr Ala Val Val Glu Lys Pro Ser Leu Thr Thr
        195                 200                 205

Trp Met Lys Gly Gly Leu Asp Tyr Ile Val Leu Lys Ser Leu Asp Thr
    210                 215                 220

Asp Gly Ile Arg Ile Ile Ser Ser Val Leu Gly Gln Ser Ile Ala Leu
225                 230                 235                 240

Asp His Tyr Ile Arg Gln Val Asp Asp Met Val Glu Glu Phe Thr Glu
                245                 250                 255

Ile Asn Arg Val Met Glu Lys Thr Gly Asn Phe Thr Met Gln Arg Lys
            260                 265                 270

Lys Leu Phe Gln Leu Val Gly Lys Ala Asn Ser Asn Leu Ala Asp Val
        275                 280                 285

Ile Ile Arg Leu Gly Leu Phe Asp Arg Ser Glu Ile Ala Trp Lys Asn
    290                 295                 300

Ala Asn Tyr Ala Gln Ile Leu Glu Tyr Leu Arg Glu Glu Tyr Glu Leu
305                 310                 315                 320

Asn Gln Arg Phe Gly Ser Leu Asp Phe Lys Leu Lys Phe Val Glu His
                325                 330                 335

Asn Ile His Phe Leu Gln Glu Val Leu Gln Asn Arg Arg Ser Asp Leu
            340                 345                 350

Leu Glu Trp Gly Val Ile Ile Leu Leu Ile Ile Glu Ile Ala Ile Ser
        355                 360                 365

Leu Tyr Glu Ile Ala Lys Asp Ser Met Ile Asp
    370                 375

<210>  5
<211>  3168
<212>  DNA
<213>  Zea mays

<400>  5
caaatcggta gtttcagttc cttcacttgt ctgcatcccc cttcccctaa ctccgccgcc      60

actgccgtcc gcgctcgccg tcgccccgaa cctatcgccc actgcatttt tccggcggag     120

gtatgcgatg aggctgctgc aagcggagga gctatttcgg aaggttctgg aggtcgggtc     180

gaagaataag gcggctcggc tgctggggct cgacgtcggc agcaagtacg tcggggtggc     240

catctccgat gagaagaaca gggtcgctat gcctctgagg taagcacctc caacaactgc     300

tgcccgcggc acactgtgct cgctgcgata agtgctatgt ttagaggatt tgattttctt     360

cctatgttaa cttgtgtact cactgtggta atccaacata agctcatatg tgccatctca     420

agatgcagat gcaggttgaa atatgaatgt gtgtgcaaaa acgttttttg gccaattggc     480

ctcctaaaat tatttcagtc caaatttttt ttggcccgct taaattgcag tggtaccaat     540

tcatatgtaa acttagtcaa gatagtattc aatgattgga tcgaggtgtt ttatttgtat     600

tctgcatatt gaagcgacag aaatggtgtg aattgtcagg tggtagatgt ggtgctatcc     660

tattatgatc ttcttttgga tattgtgtgt gcacctctga attggtgggg aaaggatatg     720

caacaacatc ctatggaatg catttggtct actcaaccac tttatttgca tcaattgttt     780

aaaactgcat gaataataaa aatgaaaagt acaaattatc atattggttg ggtaaatgga     840

agactccaat attctgtgga atttcatttc tttacatata tatttgattc aaggaccaca     900

cgtgtactca tccctatgtg tagtagatta gaaatagaga tgcaggtact cagaaaacac     960

tgctagacat ttaacagtac aagataagca tggctactga tccatctgtg tattcgtttt    1020

ttctcatgta taacatagat ttggtaaact gtgcgagtct gttacacccg accttcatcc    1080

gtttcccctt tgctcaatca ggctgggtca agcaggtcag actatatatc tgatagggtg 1140

ttttagtcta aacctttttt ctcttatccc ttgtgtactt ctctggagtt ttgtaataat 1200

ttcctctgtt gctgtcacac agttaagtct gagaagattc gaatatcttc ctttccttgt 1260

cccttgtgta cttgcctgga gttttgcaat aattgcctct gttgctgtca caaagttaag 1320

tctgagaaga ttcagattac aagcccaaca agataatttg ttgttttatg gatattggct 1380

atattcctaa aatctactta gtttctttat agccaaatct atatcttgtg aaaaaaatgt 1440

aacaactgca attcagacga cagtttacat tcatgtactt gttagaaaag gacatgtatc 1500

attatctgtg gctaagatcc ttattgaatg aatcttgcag tgttttgtgt cggacaaaaa 1560

caaacatcaa cttgatggca gatgatttca aaacattggt atgttgaaaa tacctgttca 1620

gtttctggta tcatataaat tttggatata tcaaaagtcg gtccttagat tgagtactca 1680

atatgctata cagcatcttt tcataacgaa tggttgtgca tcacaccatc attctctttt 1740

attaaattca ttttggtttc atttattgcc ccgaaatatt taggtttggc agttggcact 1800

gcaatttatt tgtagtaatg ctaagttcta tacgtagatc agcattttta actgattggt 1860

tgcagctcta tagttgttgc tttagtttat gtttaattgg aatgaaagat gtaatcacca 1920

atgccaactt tgtttactgg gataggtttc gatgtattcc atagctgggt tcgttgtggg 1980

ctatccattc aaaatctacg gtcaaccttg tgcaagtgta aagtgcccat gctcatgtac 2040

aattcccgtt tgttctactt aatctttttt ttaatgttct cttttgtctg taaatcaggc 2100

aatccaagta aggcatcttg ctggggaact ttgtaaaaca gggaaacttg atgatctgcc 2160

ctacgcatat tgggatgaaa atttcacctc aaaggtgaac tctgttttct tctcggaaat 2220

ttcttgctga tacatgagaa acaggattaa taggtggaaa gtgcattttt tctgctgaat 2280

gtggacgcta cgctacgtta cattacattg ttgtaacatg ctataggcat ggtatcagat 2340

tcgttaatag tgctgctcct tctatgcaca cgtttttact ctcctcccat ctctctttca 2400

agttcttcat ttcattagtg gagctctttg attgatcatt tgatctcctc cttgtggttt 2460

tccgcagtgt gtagaagccc tcttgcatcc cctgaatcta aaagacttgg acgatgccaa 2520

aacaatgaca gacaaatttg ctgcagtctg tatactccag gtgcgtactc ctaagtacca 2580

gggtgacttt gcgttactgc tgcagatttc atgtttttca actcttcgtg acagatggta 2640

catagtttat tcagttaaag tcatctgaag ctgctttagt gacactgacg acgatccatt 2700

actgaacagg gttatcttga caacatgaac agaaaactga gatccacaga taagtctgaa 2760

gcataaaggt ggcacctaag attccagttt gaactaaacc aaagagcaag ggaacctttt 2820

ttttttgata gaacaggagg ggagggcccc tactggaaat tttatttaaa atttgaaaag 2880

aaaacaaagt ttagaacaaa caagaagaca aattacaggg ggttaaggag agtttgtact 2940

gccattgttt tctactgcca ttgttgaagc caagtcggga tggcagaatc aaatctatcc 3000

aaagctctgt gaatgacaag gagcaattta tttgcaaact cctgcttgca atgttgcacc 3060

gttggatctt tgttttgaaa aagctaagca ttgcggcact ttcaaatgct ccaagtcatt 3120

tgaataataa cctccataga gaatgggaca ttgagttgct gttttagg 3168

<210> 6
<211> 3155
<212> DNA
<213> Zea mays

<400> 6
caaatcggta gtttcagttc cttcacttgt ctgcatcccc cttcccctaa ctccgccgcc 60

actgccgtcc gcgctcgccg tcgccccgaa cctatcgccc actgcatttt tccggcggag 120

gtatgcgatg aggctgctgc aagcggagga gctatttcgg aaggttctgg aggtcgggtc 180

gaagaataag gcggctcggc tgctggggct cgacgtcggc agcaagtacg tcggggtggc 240

catctccgat gagaagaaca gggtcgctat gcctctgagg taagcacctc caacaactgc 300

tgcccgcggc acactgtgct cgctgcgata agtgctatgt ttagaggatt tgattttctt 360

cctatgttaa cttgtgtact cactgtggta atccaacata agctcatatg tgccatctca 420

agatgcagat gcaggttgaa atatgaatgt gtgtgcaaaa acgttttttg gccaattggc 480

ctccttaaat tatttcagtc caaatttttt ttggcccgct taaattgcag tggtaccaat 540

tcatatgtaa acttagtcaa gatagtattc aatgattgga tcgaggtgtt ttatttgtat 600

tctgcatatt aaagcgacag aaatggtgtg aattgtcggg tggtagatgt ggtgctatcc 660

tattatgatc ttcttttgga tattgtgtat gcacctctga attggtgggg aaaggatatg 720

caacaacatc ctatggaatg catttggtct actcaaccac tttatttgca tcaattgttt 780

aaaactgcat gaataataaa aatgaaaagt ataaattatc atattggttg ggtaaatgga 840

agactccaat attctgtgga atttgatttc tttacatata tatttgattc aaggaccaca 900

agtgtactca tccctatgtg tagtagatta gaaatagaga tgcaggtact cagaaaacac 960

tgctagacat ttaacagtac aagataaaca tggctactga tccatctgtg tatttgtttt 1020

ttctcatgta taacatagat ttggtaaact gtgcgagtct gttacacccg accttcatcc 1080

gttttccctt tgctcaatca ggctgggtca agcaggtcag actatatatc tgatagggtg 1140

ttttagtcta aacctttttt ctcttatccc ttgtgtactt ctctggagtt ttgtaacaat 1200

ttcctctgtt gctgtcacac agttaagtct gagaagattc gaatatcttc ctttccttgt 1260

cccttgtgta cttgcctgga gttttgcaat aattgcctct gttgctgtca caaagttaag 1320

tctgagaaga ttcagattac aagctcaaca agataatttg ttgttttatg gatattggct 1380

atattcctaa aatctactta gtttctttat agccaaatct atatcttgtg aaaaaaatgt 1440

aacaactgca attcagacga tagtttacat tcatgtactt gttagaaaag gacatgtatc 1500

attatctgtg gctaagatcc ttattgaatg aatcttgcag tgttttgtgt cggacaaaaa 1560

caaacatcaa cttgatggca gatgatttca aaacattggt atgttgaaaa tacctgttca 1620

gtttctggta tcatataaat tttggatata tcaaaagtcg gtccttagat tgagtactca 1680

atatgctata cagcatcttt tcataacgaa tggttgtgca tcacaccatc attctctttt 1740

attaaattca ttttggtttc atttattgcc ccgaaatatt taggtttggc agttggcact 1800

gcaatttatt tgtagtaatg ctaagttcta tacgtagatc agcattttta actgattggt 1860

tgcagctcta tagttgttgc tttagtttat gtttaattgg aatgaaagat gtaatcacca 1920

atgccaactt tgtttattgg gataggtttc gatgtattcc atagctgggt tcgttgtggg 1980

ctatccattc aaaatctatg gtcaaccttg tgcaagtgta aagtgcccat gctcatgtac 2040

aattcccgtt tgttctactt aatctttttt ttaatgttct cttttgtctg taaatcaggc 2100

aatccaagta agtcttgctg gggaactttg taaaacaggg aaacttgatg atctgcccta 2160

cgcatattgg gatgaaaatt tcacctcaaa ggtgaactct gttttcttct cggaaatttc 2220

ttgctgatac atgagaaaca ggattaatag gtggaaagtg cattttttct gctgaatgtg 2280

gatgctacgt tacattacat tgttgtaaca tgctataggc atggtatcag attcgttaat 2340

agtgctgctc cttctatgca cacgttttta ctctcctccc atctctcttt gaagttcttc 2400

atttcattag tggagctctt tgattgatca tttgatctcc ttgtggtttt ccgcagtgtg 2460

tagaagccct cttgcatccc ctgaatctaa aagacttgga cgatgccaaa acaatgacag 2520

acaaatttgc tgcagtctgt atactccagg tgcgtactcc taagtaccag ggtgactttg 2580

cgttactgct gcagatttca tgtttttcaa ctcttcgtga cagatggtac atagtttatt 2640

cagttaaagt catctgaagc tgctttagtg acactgacga cgatccatta ctgaacaggg 2700

ttatcttgac aacatgaaca gaaaactgag atccacagat aagtctgaag cataaaggtg 2760

gcacctaaga ttccagtttg aactaaacca aagagcaagg gaaccttttt tttgatagaa 2820

caggagggga gggcccctac tgaaaatttt atttaaaatt tgaaaagaaa acaaagttta 2880

gaacaaacaa gaagacaaat tacagggggt tagggagagt ttgtactgcc attgttttct 2940

actgccattg ttgaagccaa gtcgggatgg cagaatcaaa tctacccaaa gctctgtgaa 3000

tgacaaggag caattcattt gcaaactcct gcttgcaatg ttgcaccgtt ggatctttgt 3060

tttgaaaaag ccaagcattg cggcacttcc aaatgctcaa agtcatgaga ataataacct 3120

ccatagagaa tgggacattg agttgctgtt ttagg 3155

<210> 7
<211> 525
<212> DNA
<213> Artificial Sequence

<220>
<223>  cDNA of Zm00001d043352 (genotype B73)

<400>  7
atgaggctgc tgcaagcgga ggagctattt cggaaggttc tggaggtcgg gtcgaagaat      60

aaggcggctc ggctgctggg gctcgacgtc ggcagcaagt acgtcggggt ggccatctcc     120

gatgagaaga acagggtcgc tatgcctctg agtgttttgt gtcggacaaa aacaaacatc     180

aacttgatgg cagatgattt caaaacattg gtttcgatgt attccatagc tgggttcgtt     240

gtgggctatc cattcaaaat ctatggtcaa ccttgtgcaa gtgcaatcca agtaagtctt     300

gctggggaac tttgtaaaac agggaaactt gatgatctgc cctacgcata ttgggatgaa     360

aatttcacct caaagtgtgt agaagccctc ttgcatcccc tgaatctaaa agacttggac     420

gatgccaaaa caatgacaga caaatttgct gcagtctgta tactccaggg ttatcttgac     480

aacatgaaca gaaaactgag atccacagat aagtctgaag cataa                     525

<210>  8
<211>  174
<212>  PRT
<213>  Zea mays

<400>  8

Met Arg Leu Leu Gln Ala Glu Glu Leu Phe Arg Lys Val Leu Glu Val
1               5                   10                  15

Gly Ser Lys Asn Lys Ala Ala Arg Leu Leu Gly Leu Asp Val Gly Ser
            20                  25                  30

Lys Tyr Val Gly Val Ala Ile Ser Asp Glu Lys Asn Arg Val Ala Met
        35                  40                  45

Pro Leu Ser Val Leu Cys Arg Thr Lys Thr Asn Ile Asn Leu Met Ala
    50                  55                  60

Asp Asp Phe Lys Thr Leu Val Ser Met Tyr Ser Ile Ala Gly Phe Val
65                  70                  75                  80

Val Gly Tyr Pro Phe Lys Ile Tyr Gly Gln Pro Cys Ala Ser Ala Ile
                85                  90                  95

Gln Val Ser Leu Ala Gly Glu Leu Cys Lys Thr Gly Lys Leu Asp Asp
            100                 105                 110

Leu Pro Tyr Ala Tyr Trp Asp Glu Asn Phe Thr Ser Lys Cys Val Glu
        115                 120                 125

Ala Leu Leu His Pro Leu Asn Leu Lys Asp Leu Asp Asp Ala Lys Thr
    130                 135                 140

Met Thr Asp Lys Phe Ala Ala Val Cys Ile Leu Gln Gly Tyr Leu Asp
145                 150                 155                 160

Asn Met Asn Arg Lys Leu Arg Ser Thr Asp Lys Ser Glu Ala
                165                 170

<210>  9
<211>  3645
<212>  DNA
<213>  Zea mays

<220>
<221>  misc_feature
<222>  (1562)..(1567)
<223>  n is a, c, g, or t

<400>  9
aacacgctct cagtctcact cccacggtcc cactcctcag gagcgtccag agtttaggtg      60

ctcgtggagt cgtggtggag gtggtaggtg tcgatggcct tcaagctggc caccaaggcc     120

gcggcggcgt cgcccgctgc tgctcaccgc gggggtctcg cccgggggcc ggagggtacg     180

agccgcgttg ccttcggacc agcgcctaga aacaaggggc tccgcgcggc caacaactcc     240

gcgacgcccg tggctaagta cgaacgctcc ttctcctggt tagaaattgt ttaatttgat     300

aaacgactat gacccttggt tccgcgtaag gtatgggggg ctggccctac atcagacttt     360

gtgtttgtgc gtggtcgtga tcgtggtgtg gtgtggtgtg gtaactctta tctaaagaga     420

tttaggattg ggtcggttct cagttgggga tctgtttggt gcgcttgagc ccttctgaac     480

ttgatcatcc atgtttttga tacttaaggc atccaatttg tagctactgc tctagttcta     540

ggatttggag aactccttgt cgaattgtcc cacagagcta atgattgtgc taacaaaatg     600

acatgatgct gattgttcta cgcctcttgc ttaataccct gtgaagttta attatttggt     660

ggtacacttg accttggaag tcatgacctc ccaaaagcat ggtgtagcat gtaggtacta     720

tatatctagg ttgttgcaac taaattgtgg aaatttccac acctctgatg gctcatatgt     780

tgtcattcct ttgtgacgaa gtcttcttga tttttgtgaa tatattccag atacgaaagt     840

ggaaccgtgg ggaggaaatt gacatggatg gctatatata ttaaccttgt tgctttttgc     900

acaaaggctt actgtcttat gtgatgctgg attactttgt ttttacgaaa cccgctttag     960

ttatactgaa ggaactgatc atttctttat cttaatggtg cacttttatt agggaagaga    1020

gggttgatcg aagtgaaata ttgacattgg atagcattag acaagttttg attagactag    1080

aagacagcat catattcggc cttttggaga gagcacagtt ttgttacaac gctgatacat    1140

atgatagcaa tgctttccac atggatggtt ttggcggctc tttggttgaa tatatggtta    1200

gagaaactga aaagctccat gcacaggtgg aattcatttg gacatttttt aagaacaaaa 1260

acagttgatc atggcatgca aaagaataac atacactatt gttttaacaa taggttggga 1320

gatacaagag cccagatgag caccctttct ttcctgagga tctgcctgag ccccggttgc 1380

cacctatgca gtacccaagg gtaatgcatg ttcttggttc tcaaatgctt ctcatgatga 1440

ctgcttttgt ttctttgtca ctgtaataga tataaaccat catttgcaat ctaatgtacc 1500

attttcttca tggaggtaga taaaagaaaa tgtgcttttg gatacagttg tttagcatcc 1560

gnnnnnncaa tatattgagc aatgctcagt tagtgaacaa catataggta gtgctctgtg 1620

acttgcagaa tgtactatca ttattgcaaa tgccacatcc cacagacctt agatacaagt 1680

gacggaaagt tctgtgttta ctactatata ttctactttc tccaatatat tttggaacaa 1740

gctgtacagc tctttattgt attgcgttga gtcaaaataa gtgatggggt tattttttta 1800

ttacaacata tgctattttg actttgagta aatccaaaag ctcctattga accttaggtg 1860

gagcagaggt gaaagtaaca ttatttgcat tttttcaagg ggatatccaa ttgatgatga 1920

tttactgctt tgcttcaatt taatcgttgg atggtttgac ctgcatgaat cttgtcatat 1980

tctacaagcg ctaacatagt gccatcctaa tccatgtgca acatgaaata tttgtttatg 2040

ctgcatttgg actatcatgt gttgcaagat tcataaatca taatgccaac aagttgtcct 2100

ggtattatga ctatgttagt acattttaga tggattcata cttcgctctt gtcctttgca 2160

taggttttgc atcccattgc cgattctatc aatatcaaca aagagatttg gaaaatgtat 2220

tttgatgaac ttcttccaag attggtgaaa aaaggaagtg atggtaatgc tggatccagt 2280

gctctttgtg acacgacctg cttgcaggta cagatgatat tcatgttaaa tcctacactc 2340

agttttatgg tctatttggt tcctttagtc cagggactaa agttgtccct accctgtttg 2400

gttccaagga ctaaaagtat tcaaaacaca ttaaatgact ataagaatac cgaaatgccc 2460

cttaccgttc taccgtcttt agttcaactg aaataaatga tggacaaaag gtagaattaa 2520

tatgttttag tcctttttag tcaccccttg aggggctagg gactaaaagg actaatcttt 2580

aatccctgga accaaacaga cccttagttc tttgggtgca catccgcttt tgttccagac 2640

ttccagtcaa ctaggtgtcc aggttataaa attccttttg ctctgtctgc cacaattttc 2700

attcagttaa aagaatatat ttgagcaaac aaagccagat caaatttgca tgaatcaatc 2760

catgcaagat tattttcata gtactggcct gatcattgct ggtaacttgt gcaattttgc 2820

atttttataa ttccctttct ctgtttttct tgttccatac tccaggcgct ctccaaaagg 2880

atccactatg ggaagtttgt ggcagaggct aagtttcagg agtccccgga agcttacatg 2940

ccagccataa tagctcaggt atgtgctttt gtttcaactc tagtatttct agtacaacta 3000

tttttcattt ttcttttgtt ttatttcaga gtttcataac aaaatcatat ttcgattcat 3060

ttgaagaaaa aaactgtcga tttcttgtca cgttttgcaa aatccaggac cgtgatcaac 3120

tcatgcacct tctcacatat gaaacggtgg agcgtgctat cgaacacagg gtggaagcca 3180

aagccaagat cttcgggcaa gaggtgaaca tcggtgtgga ggacaacggc agcccaccgg 3240

tgtacaagat cgttccgagc ttggtcgccg agctgtacag ctacagaatc atgccgctaa 3300

ccaaagaggt tcaaatcgcg tatttgctta ggaggctgga ttgattgtgt ttatgtagat 3360

gtaaattgcc agatcagaac tcctggcgtt aagccagaac attggtaaat accggttttg 3420

gtgtcaggat gcaaaatgta ccatgtgtgg aattgtgcca acaaacaagc ataatatcat 3480

gtggagttgt cgcgtagtcg cacctcgtgc gtactgccaa tggacaaagg ttgtagactg 3540

tatggaatga tggttcactt tctttggcgg atgccctttt gaatgggaat aaaattctct 3600

cattgtcgtt gaaatttata cagatccgct cacgtgccat cgaaa 3645

<210> 10
<211> 3745
<212> DNA
<213> Zea mays

<400> 10
aacacgctct cagtctcact cccacggtcc cactcctcag gagcgtccag agtttaggtg 60

ctcgtggagt cgtggtggag gtggtaggtg tcgatggcct tcaagctggc caccaaggcc 120

gcggcggcgt cgcccgctgc tgctcaccgc gggggtctcg cccgggggcc ggagggtacg 180

agccgcgttg ccttcggacc agcgcctaga aacaaggggc tccgcgcggc caacaactcc 240

gcgacgcccg tggctaagta cgaacgctcc ttctcctggt tagaaattgt ttaatttgat 300

aaacgactat gacccttggt tccgcgtaag gtatgggggg ctggccctac atcagacttt 360

gtgtttgtgc gtggtcgtga tcgtggtgtg gtgtggtgtg gtgtggtaac tcttatctaa 420

agagatttag gattgggtcg gttctcagtt ggggatctgt ttggtgcgct tgagcccttc 480

tgaacttgat catccatgtt tttgatactt aaggcatcca atttgtagct actgctctag 540

ttctaggatt tggaaaactc cttgtcgaat tgtcccacag agctaatgat tgtgcgaaca 600

aaatgacatg atgctgattg ttctacgcct cttgcttaat accctgtgaa gtttaattat 660

ttggtggtac acttgacctt ggaagtcatg acctcccaaa agcatggtgt agcatgtagg 720

tactatatat ctaggttgtt gcaactaaat tgtggaaatt tccacacctc tgatggctca 780

tatgttgtca ttcctttgtg acgaagtctt cttgattttt gtgaatatat tccagatacg 840

aaagtggaac cgtggggagg aaattgacat ggatggctat atatattaac tctgttgctt 900

tttgcacaaa ggcttactgt cttatgtgat gctggattgc tttgttttta cgaaacccgc 960

tctagttata ctgaaggaac tgatcatttc tttatcttaa tggtgcactt ttattaggga 1020

agagagggtt gatcgaagtg aaatattgac attggatagc attagacaag ttttgattag 1080

actagaagac agcatcatat ttggcctttt ggagagagca cagttttgtt acaatgctga 1140

tacatatgat agcaatgctt tccacatgga tggttttggc ggctctttgg ttgaatatat 1200

ggttagagaa actgaaaagc tccatgcaca ggtggaattc atttggacat tttttaagaa 1260

caaaaacagt tgatcatggc atgcaaaaga ataacatacg ctattgtttt taacataggt 1320

tgggagatac aagagcccag atgagcaccc tttctttcct gaggatctgc ctgagccccg 1380

gttgccacct atgcagtacc caagggtaat gcatgttctt ggttctcaaa tgcttctcat 1440

gatgactgct tttgtttctt tgtcaccgta agagatataa accatcattt gcaatctaat 1500

gtaccatttt cttcatggag gtagataaaa gaaaatgtgc ttttgaatac agttgtttag 1560

catccgaatt aaaaggcttg tacagccaat atattgagca attctcagtt agtgaacaac 1620

atataggtag tgttctgtga cttgcagaat gtactatcat tattgcaaat gccacatccc 1680

acagacctta gatacaagtg acggaaagtt ctgtgtttac tactatatat tttactttct 1740

ccaatatatt ttggaacaag ctgtacagct ctttattgta ttgcgttgag tcgaaataag 1800

tgatggggtt attttttttg tttttttatt acaacatatg ctcttttgac tttgagtaaa 1860

tccaaaagct cctattgaac cttaggtgga gcagaggtga aagtaacatt atttgcattt 1920

ttttcaaggg gatatccatt tgatgatgat ttactgcttt gcttcaattt aatcgttgga 1980

tggtttgaca ctttgacctg catgaatctt gtcatattct acaagcgcta acatagcgcc 2040

atcctaatcc atgtgcaaca tgaaatattt gtttatgctg catttggact atcatgcgtt 2100

gcaagattca taaatcataa tgccaacaag ttgtcctggt attatgacta tgttagtaca 2160

ttttagatgg attcatactt cgctcttgtc ctttgcatag gttttgcatc ccattgctga 2220

ttctatcaat atcaacaaag agatttggaa aatgtatttt gatgaacttc ttccaagatt 2280

ggtgaaaaaa ggaagtgatg gtaatgctgg atccagtgct ctttgtgaca cgacctgctt 2340

gcaggtacag atgatattca tgttaaatcc tacactcagt tttatggtct gtttggttcc 2400

tttagtcctg ggactaaagt tgtccctacc aacaggttcc aaggactaaa agtattcaaa 2460

acgcattaaa tgactttata agaataccga aatgcccctt accgttctac cgtctttagt 2520

tcaactgaaa taaatgatgg acaaaaggta gaattaatat gttttagtcc tttttagtca 2580

ccccttgagg ggctagggac taaaataatt tagtctttgt tttagttcca ctgtttgtca 2640

atttagggac taaatgtgat taaaatagaa ggactaatct ttaatccctg gaaccaaaca 2700

gacccttagt tctttgggtg cacatccact tttgttccag acttccagtc aactaggtgt 2760

ccaggttata aaactccttt tgctctgtct gccacagttt tcattcagtt aaaagaatat 2820

atttgagcaa acaaagccag atcaaatttg catgaatcaa tccatgcaag attattttca 2880

tagtactggc ctgatcattg ctggtaactt gtgcaatttt gcatttttat aattcccttt 2940

ctctgttttt cttgttccat actccaggcg ctctccaaaa ggatccacta tgggaagttt 3000

gtggcagagg ctaagtttca ggagtccccg gaagcttaca tgccagccat aatagctcag 3060

gtctgtgctt ttgtttcaac tctagtattt ctagtacaac tatttttcat ttttttgttt 3120

tatttcagag tttcataaca aaaatcatat ttcgattcat ttgaataaaa aaactgtcga 3180

tttcttgtca cgttttgcaa aatccaggac cgtgatcaac tcatgcacct tctcacatat 3240

gaaacggtgg agcgtgctat cgaacatagg gtggaagcca aagccaagat cttcgggcaa 3300

gaggtgaaca tcggtgtgga ggacaacggc agcccaccgg tgtacaagat cgttccgagc 3360

ttggtcgccg agctgtacag ctacagaatc atgccgctaa ccaaagaggt tcaaatcgcg 3420

tatttgctta ggaggctgga ttgagtatgt ttatgtagat gtaaattgcc agatcagaac 3480

tcctggcgtt aagccagaac attggtaaat accggttttg gtgtcaggat gcaaaatgta 3540

ccatgtgtgg aattgtgcca acaaacaagc ataatatcat gtggagttgt cgagtagtcg 3600

cgcctcgtgc gtactgccaa tggacaatgt ttgtagactg tatggaatga tgggtcactt 3660

cctttggcgg gtgccctttt gaaagggagt aaaattctct cattgtcgtt gaaatttata 3720

cagatccgct caagtgccat cgaaa 3745

<210> 11
<211> 939
<212> DNA
<213> Artificial Sequence

<220>
<223> cDNA of Zm00001d043356 (genotype B73)

<400> 11
atggccttca agctggccac caaggccgcg gcggcgtcgc ccgctgctgc tcaccgcggg 60

ggtctcgccc gggggccgga gggtacgagc cgcgttgcct tcggaccagc gcctagaaac 120

aaggggctcc gcgcggccaa caactccgcg acgcccgtgg ctaaggaaga gagggttgat 180

cgaagtgaaa tattgacatt ggatagcatt agacaagttt tgattagact agaagacagc 240

atcatatttg gccttttgga gagagcacag ttttgttaca atgctgatac atatgatagc 300

aatgctttcc acatggatgg ttttggcggc tctttggttg aatatatggt tagagaaact 360

gaaaagctcc atgcacaggt tgggagatac aagagcccag atgagcaccc tttctttcct 420

gaggatctgc ctgagccccg gttgccacct atgcagtacc caagggtttt gcatcccatt 480

gctgattcta tcaatatcaa caaagagatt tggaaaatgt attttgatga acttcttcca 540

agattggtga aaaaaggaag tgatggtaat gctggatcca gtgctctttg tgacacgacc 600

tgcttgcagg cgctctccaa aaggatccac tatgggaagt ttgtggcaga ggctaagttt 660

caggagtccc cggaagctta catgccagcc ataatagctc aggaccgtga tcaactcatg 720

caccttctca catatgaaac ggtggagcgt gctatcgaac atagggtgga agccaaagcc 780

aagatcttcg ggcaagaggt gaacatcggt gtggaggaca acggcagccc accggtgtac 840

aagatcgttc cgagcttggt cgccgagctg tacagctaca gaatcatgcc gctaaccaaa 900

gaggttcaaa tcgcgtattt gcttaggagg ctggattga 939

<210> 12
<211> 312
<212> PRT
<213> Zea mays

<400> 12

Met Ala Phe Lys Leu Ala Thr Lys Ala Ala Ala Ala Ser Pro Ala Ala
1               5                   10                  15

Ala His Arg Gly Gly Leu Ala Arg Gly Pro Glu Gly Thr Ser Arg Val
            20                  25                  30

Ala Phe Gly Pro Ala Pro Arg Asn Lys Gly Leu Arg Ala Ala Asn Asn
        35                  40                  45

Ser Ala Thr Pro Val Ala Lys Glu Glu Arg Val Asp Arg Ser Glu Ile
    50                  55                  60

Leu Thr Leu Asp Ser Ile Arg Gln Val Leu Ile Arg Leu Glu Asp Ser
65                  70                  75                  80

Ile Ile Phe Gly Leu Leu Glu Arg Ala Gln Phe Cys Tyr Asn Ala Asp
                85                  90                  95

Thr Tyr Asp Ser Asn Ala Phe His Met Asp Gly Phe Gly Gly Ser Leu
            100                 105                 110

Val Glu Tyr Met Val Arg Glu Thr Glu Lys Leu His Ala Gln Val Gly
        115                 120                 125

Arg Tyr Lys Ser Pro Asp Glu His Pro Phe Phe Pro Glu Asp Leu Pro
    130                 135                 140

Glu Pro Arg Leu Pro Pro Met Gln Tyr Pro Arg Val Leu His Pro Ile
145                 150                 155                 160

Ala Asp Ser Ile Asn Ile Asn Lys Glu Ile Trp Lys Met Tyr Phe Asp
                165                 170                 175

Glu Leu Leu Pro Arg Leu Val Lys Lys Gly Ser Asp Gly Asn Ala Gly
            180                 185                 190

Ser Ser Ala Leu Cys Asp Thr Thr Cys Leu Gln Ala Leu Ser Lys Arg

195                 200                 205

Ile His Tyr Gly Lys Phe Val Ala Glu Ala Lys Phe Gln Glu Ser Pro
    210                 215                 220

Glu Ala Tyr Met Pro Ala Ile Ile Ala Gln Asp Arg Asp Gln Leu Met
225                 230                 235                 240

His Leu Leu Thr Tyr Glu Thr Val Glu Arg Ala Ile Glu His Arg Val
                245                 250                 255

Glu Ala Lys Ala Lys Ile Phe Gly Gln Glu Val Asn Ile Gly Val Glu
            260                 265                 270

Asp Asn Gly Ser Pro Pro Val Tyr Lys Ile Val Pro Ser Leu Val Ala
        275                 280                 285

Glu Leu Tyr Ser Tyr Arg Ile Met Pro Leu Thr Lys Glu Val Gln Ile
    290                 295                 300

Ala Tyr Leu Leu Arg Arg Leu Asp
305                 310

<210>  13
<211>  812
<212>  DNA
<213>  Zea mays

<400>  13
agcacaaaaa tcaattgaac gttttgagaa cgaaaataac acccgccgat actataccat      60

aaataaagtt caagaaatcc acccctgccc gccgccgcct cttccttccg gccggtcttc     120

gtttcgacat ccaggaaatg gctaccgcgg cgcgcctcct cctcgtcgtg gtgcttctct     180

ccgcggcgat accgatcccg tccttcgcga caagcttcgt cgtcggcgac aagaggcatc     240

gctgggctcc caacgtcaac tatacagact gggcggaccg tcaccagttc cacgtcggcg     300

actggctcgg tatcattctc ctcccctata tctatctacc tttacatact catcaacatc     360

aaacagcccg tgtgatggag atctagcttt cggcaaaaga attgatctaa gggtgggaag     420

acgcgttaat ttgcacgcat gcagaattca ggtacgagag ggacaggttc gatgtggtgc     480

aggtgaacga gacggcatac gcggcgtgcg acgccagcag ccccatcctc agctacagcc     540

gcggccacaa cttcgtcttc cggctcaacc acacgggccg gttctacttc atctgcagcc     600

gcggctactg ctggagtggc atgaaggtct ccgtgctcgt ccagccgccg ccgcccccgt     660

cgctgccgcc ggcgtcccac tcccacgcgt caagcgccag tgcgcgtgtg cggccggcgg     720

ccgggctttg gtgtgcggct ctctctacct tgctgggctg ggcggtgctc acgccgccgc     780

tgtcgtttcg ggtctggagt tctggatgtc ca                                    812

<210>  14
<211>  915
<212>  DNA
<213>  Zea mays

<400>  14
agcacaaaaa tcaattgaac gttttgagaa cgaaaataac acccgccgat actataccat      60

aaataaagtt caagaaatcc acccctgccc gccgccgcct cttccttccg gccggtcttc     120

gtttcgacat ccaggaaatg gctaccgcgg cgcgcctcct cctcgtcgtg gtgcttctct     180

ccgcggcgat accgatcccg tccttcgcga caagcttcgt cgtcggcgac aagaggcatc     240

gctgggctcc caacgtcaac tatacagact gggcggaccg tcaccagttc cacgtcggcg     300

actggctcgg tatcattctc ctcccctata tctatctacc tttacatact catcaacatc     360

aaacagcccg tgtgatggag atctagcttt cggcaaaaga attgatctaa gggtgggaag     420

acgcgttaat ttgcacgcat gcagaattca ggtacgagag ggacaggttc gatgtggtgc     480

aggtgaacga gacggcatac gcggcgtgcg acgccagcag ccccatcctc agctacagcc     540

gcggccacaa cttcgtcttc cggctcaacc acacgggccg gttctacttc atctgcagcc     600

gcggctactg ctggagtggc atgaaggtct ccgtgctcgt ccagccgccg ccgccgcccc     660

cgtcgctgcc gccggcgtcc cactcccacg cgtcaagcgc cagtgcgcgt gtgcggccgg     720

cggccgggct ttggtgtgcg gctctctcta ccttgctggg ctgggcggtg ctcacgccgc     780

cgctgtcgtt tcgggtctgg agttctggac gtccaggcgc gcacacgagt ctaaactcta     840

aagctgtttg ctaatgtact acccatttct tcttttttta gtatggaaat aagagttccg     900

gctttagcat aaaga                                                      915

<210>  15
<211>  582
<212>  DNA
<213>  Artificial Sequence

<220>
<223>  cDNA of Zm00001d043357 (genotype B73)

<400>  15
atggctaccg cggcgcgcct cctcctcgtc gtggtgcttc tctccgcggc gataccgatc      60

ccgtccttcg cgacaagctt cgtcgtcggc gacaagaggc atcgctgggc tcccaacgtc     120

aactatacag actgggcgga ccgtcaccag ttccacgtcg gcgactggct cgaattcagg     180

tacgagaggg acaggttcga tgtggtgcag gtgaacgaga cggcatacgc ggcgtgcgac     240

gccagcagcc ccatcctcag ctacagccgc ggccacaact tcgtcttccg gctcaaccac     300

acgggccggt tctacttcat ctgcagccgc ggctactgct ggagtggcat gaaggtctcc     360

gtgctcgtcc agccgccgcc gccgcccccg tcgctgccgc cggcgtccca ctcccacgcg 420

tcaagcgcca gtgcgcgtgt gcggccggcg gccgggcttt ggtgtgcggc tctctctacc 480

ttgctgggct gggcggtgct cacgccgccg ctgtcgtttc gggtctggag ttctggacgt 540

ccaggcgcgc acacgagtct aaactctaaa gctgtttgct aa 582

<210> 16
<211> 193
<212> PRT
<213> Zea mays

<400> 16

Met Ala Thr Ala Ala Arg Leu Leu Leu Val Val Val Leu Leu Ser Ala
1               5                   10                  15

Ala Ile Pro Ile Pro Ser Phe Ala Thr Ser Phe Val Val Gly Asp Lys
            20                  25                  30

Arg His Arg Trp Ala Pro Asn Val Asn Tyr Thr Asp Trp Ala Asp Arg
        35                  40                  45

His Gln Phe His Val Gly Asp Trp Leu Glu Phe Arg Tyr Glu Arg Asp
    50                  55                  60

Arg Phe Asp Val Val Gln Val Asn Glu Thr Ala Tyr Ala Ala Cys Asp
65                  70                  75                  80

Ala Ser Ser Pro Ile Leu Ser Tyr Ser Arg Gly His Asn Phe Val Phe
                85                  90                  95

Arg Leu Asn His Thr Gly Arg Phe Tyr Phe Ile Cys Ser Arg Gly Tyr
            100                 105                 110

Cys Trp Ser Gly Met Lys Val Ser Val Leu Val Gln Pro Pro Pro Pro
        115                 120                 125

Pro Pro Ser Leu Pro Pro Ala Ser His Ser His Ala Ser Ser Ala Ser
    130                 135                 140

Ala Arg Val Arg Pro Ala Ala Gly Leu Trp Cys Ala Ala Leu Ser Thr
145                 150                 155                 160

Leu Leu Gly Trp Ala Val Leu Thr Pro Pro Leu Ser Phe Arg Val Trp
                165                 170                 175

Ser Ser Gly Arg Pro Gly Ala His Thr Ser Leu Asn Ser Lys Ala Val
            180                 185                 190

Cys

<210> 17
<211> 71
<212> DNA
<213> Artificial Sequence

<220>
<223> marker ma0016fm86@36

<400> 17
acatcttctt gccagagccg ttggggtcga gggggrcgat cttcattgct tccgccggct 60

tgaagttgcc g 71

<210> 18
<211> 71
<212> DNA
<213> Artificial Sequence

<220>
<223> marker ma0016fn05@36

<400> 18
aagtactata agggatgtca agtgtgtcag aagttyagtg atctacaatt tttttggtct 60

ctgaacatca t 71

<210> 19
<211> 71
<212> DNA
<213> Artificial Sequence

<220>
<223> marker ma0016fn29@36

<400> 19
acctaggaag gtcagacggt ctgaccatgt gccctrgatg gtccataccc cggatagtcc 60

acgagatagc c 71

<210> 20
<211> 71
<212> DNA
<213> Artificial Sequence

<220>
<223> marker ma0016fn27@36

<400> 20
caccaccgca tagagcacca ccagaagata caatcsctag tgtttggccc gaatcgaaac 60

caacagtgct c 71

<210> 21
<211> 71

<212>  DNA
<213>  Artificial Sequence

<220>
<223>  marker ma0016fn16@36

<400>  21
cagttcgcaa gtagtcaaat tatagaaagg tgagarcgca tgataataac tttttcgagt      60

atatccattt g                                                            71


<210>  22
<211>  47
<212>  DNA
<213>  Artificial Sequence

<220>
<223>  marker ma0004tk22@12

<400>  22
cattacttgg arttcagtga taaagttcat cataaattaa aacatac                     47


<210>  23
<211>  71
<212>  DNA
<213>  Artificial Sequence

<220>
<223>  marker ma0004tk05@36

<400>  23
gaaacaagtc ccctctgtaa ggttctaccc ttgaamtata aaagagatag gtccccctcc      60

ttcgttccac a                                                            71


<210>  24
<211>  71
<212>  DNA
<213>  Artificial Sequence

<220>
<223>  marker ma0016fn55@36

<400>  24
agataaataa ttcacataac attagcggtt ataagygaga cgaagtaata gtgtgagacg      60

aaaatggcca c                                                            71


<210>  25
<211>  71
<212>  DNA
<213>  Artificial Sequence

<220>
<223>  marker ma0016fp29@36

<400>  25
actccctcta gtaagtgagt cttacataac tagctyatcg ttgtacagtg tacttgcatg      60

tgcacaatga t                                                          71


<210>  26
<211>  69
<212>  DNA
<213>  Artificial Sequence

<220>
<223>  marker ma0004tm29@36

<400>  26
ctagaattaa ggatgataat tttgaatagt taactraaga aacggtttaa tagtattttc      60

tctacaata                                                            69


<210>  27
<211>  71
<212>  DNA
<213>  Artificial Sequence

<220>
<223>  marker ma0004tm38@36

<400>  27
gaacttgtat caggcaggtc ctgtggaaga gtatgygaag aattttgagg aggcatgttg      60

tgcaacggca g                                                          71


<210>  28
<211>  48
<212>  DNA
<213>  Artificial Sequence

<220>
<223>  marker ma0016fq46@15

<400>  28
aatacaagtt tttgyatatt tcccttcgct cggcatgatg gtctgaac                  48


<210>  29
<211>  71
<212>  DNA
<213>  Artificial Sequence

<220>
<223>  marker ma0000ba98@36

<400>  29
aaattggaac ttttggggag tcacatactc tcttamtgtt agatgtcttt ctgatggctg      60

tgatatcaaa t                                                          71


<210>  30
<211>  71
<212>  DNA
<213>  Artificial Sequence

<220>

<223>  marker ma0000na46@36

<400>  30
gtgatgtgcc cacctctagc acctgaggga ttgaarcctg ttactggcat ggatctctga        60

gcaaaatcaa g                                                              71


<210>  31
<211>  71
<212>  DNA
<213>  Artificial Sequence

<220>
<223>  marker ma0001gr36@36

<400>  31
gaatgctgca ttcttaagat tgattcgatc tcttcrgatg acatgtactt ggatctaaac        60

tttatacctc c                                                              71


<210>  32
<211>  71
<212>  DNA
<213>  Artificial Sequence

<220>
<223>  marker ma0000kr37@36

<400>  32
taccagtatc agatggtagt tgagagaatc gaatgytatg ttttcccttc cccgaccttg        60

atctcttgtc t                                                              71


<210>  33
<211>  71
<212>  DNA
<213>  Artificial Sequence

<220>
<223>  marker ma0001gr37@36

<400>  33
tgatgaatgg gaactgccaa cagaaccaaa cctgcytgta atctctgcgg agtgatgggg        60

atataggacg t                                                              71


<210>  34
<211>  71
<212>  DNA
<213>  Artificial Sequence

<220>
<223>  marker ma0001gr41@36

<400>  34
ctctgctgat gctgagaaaa taggagtgac gacatygggc tattaggaag tgataattgt        60

ttgtgcaata a                                                              71

<210>  35
<211>  52
<212>  DNA
<213>  Artificial Sequence

<220>
<223>  marker ma0016fq61@17

<400>  35
aatatcatcc ttaaaawatg ttcaattgcc acagcctagg aatatagggg ca          52


<210>  36
<211>  64
<212>  DNA
<213>  Artificial Sequence

<220>
<223>  marker ma0022xj91@29

<400>  36
aattcaacaa aaggatgtgt gcaagcatrc ttgtcttcca tctctgtcgt gcataactca      60

atcc                                                                    64


<210>  37
<211>  71
<212>  DNA
<213>  Artificial Sequence

<220>
<223>  marker ma0016fq64@36

<400>  37
cataactcaa tccaataacg aagcgcgtgt gctacmaaac aaacgtataa ccatttattc      60

cacacatcta g                                                            71


<210>  38
<211>  71
<212>  DNA
<213>  Artificial Sequence

<220>
<223>  marker ma0001jh28@36

<400>  38
ccaccgccat tgccatcact gccggcttcc tccaarcccc cgagctcgaa cccctcgagt      60

ggctccttcc c                                                            71


<210>  39
<211>  71
<212>  DNA
<213>  Artificial Sequence

<220>
<223>  marker ma0004tn40@36

<400>  39
gacaaaatcg atttctgtta agattcttgg acccawaggc ctcacgaaca agtctccacc      60

cgagcccgaa a                                                          71


<210>  40
<211>  43
<212>  DNA
<213>  Artificial Sequence

<220>
<223>  marker ma0004tn32@36

<400>  40
agaaggattc agtgattcac agatcacaca caagakagct cag                       43


<210>  41
<211>  52
<212>  DNA
<213>  Artificial Sequence

<220>
<223>  marker ma0016fq88@36

<400>  41
tgccaaagac agtaagagcg aagtttgata gtcgayaccg ccaatataat ga             52


<210>  42
<211>  48
<212>  DNA
<213>  Artificial Sequence

<220>
<223>  marker ma0004tn60@34

<400>  42
atcatggcct cattccacaa atcaacacga tctycaaaaa ttgcaagc                  48


<210>  43
<211>  56
<212>  DNA
<213>  Artificial Sequence

<220>
<223>  marker ma0016fq93@36

<400>  43
taaagaaagt caagcccatc aacattgata agtacratgg caagaaggat ctaaat         56


<210>  44
<211>  52
<212>  DNA
<213>  Artificial Sequence

<220>
<223>  marker ma0016fq89@36

<400>  44

ccaacattag ggcgatttgt acgatgtcct ccatargcat gatatttgca ag 52

<210> 45
<211> 71
<212> DNA
<213> Artificial Sequence

<220>
<223> marker ma0016fq94@36

<400> 45
ttgggcaact ccatgacaca caaacttgag tctgayttct ataaagttgt aggacacaaa 60

gtctagaaca c 71

<210> 46
<211> 59
<212> DNA
<213> Artificial Sequence

<220>
<223> marker ma0004tn62@36

<400> 46
caagccaata atgtaagggt agataatagg gacaasgatg tatccaagac cgacttcat 59

<210> 47
<211> 62
<212> DNA
<213> Artificial Sequence

<220>
<223> marker ma0004tn31@27

<400> 47
tctagcaaac tccatagtcg caacatrgaa accacggtgg atcgagcatc tacagctctt 60

tg 62

<210> 48
<211> 71
<212> DNA
<213> Artificial Sequence

<220>
<223> marker ma0016fq50@36

<400> 48
gagcatcatg tttggtacca gctctttccc tgacarctcc cttctggatg ctgatgctgt 60

gttcagtttc g 71

<210> 49
<211> 41
<212> DNA
<213> Artificial Sequence

<220>

<223>  marker ma0004tn30@36

<400>  49
tttggagctg ttgcttcgac gatgattgag agagaycagt g                          41


<210>  50
<211>  71
<212>  DNA
<213>  Artificial Sequence

<220>
<223>  marker ma0000xk01@36

<400>  50
gtgatcaagc ctgagttaaa caagtgcaaa caaacmtact ctataggtag cattgtgtca      60

gttgacactt t                                                           71


<210>  51
<211>  71
<212>  DNA
<213>  Artificial Sequence

<220>
<223>  marker ma0011cv03@36

<400>  51
tctgcatacg ttggcagttt agttctacgg ggtcgsatga tgctcaggtt tggtgaatgg      60

ggtctctcct t                                                           71


<210>  52
<211>  71
<212>  DNA
<213>  Artificial Sequence

<220>
<223>  marker ma0016fq79@36

<400>  52
tgtatctctg gatgctgatt caactgtaaa tatgasacaa tgccagtaat tgcatcagtt      60

tatatagata g                                                           71


<210>  53
<211>  71
<212>  DNA
<213>  Artificial Sequence

<220>
<223>  marker ma0004tn51@36

<400>  53
cctccctccc ggcaaccaca acattgaaga cagccmctct gtttcacaaa ttctgactat      60

tatcttgaga a                                                           71


<210>  54

<211>  49
<212>  DNA
<213>  Artificial Sequence

<220>
<223>  marker ma0004tn53@14

<400>  54
gctatttcag atascgaatg attgtcccct atgatttcat taacaactc                49


<210>  55
<211>  71
<212>  DNA
<213>  Artificial Sequence

<220>
<223>  marker ma0000cm74@36

<400>  55
accgacccag tagctgtgtc cagtcttgct ttaaastttg aagctctctt cttattaaag      60

cagtttttgc t                                                           71


<210>  56
<211>  71
<212>  DNA
<213>  Artificial Sequence

<220>
<223>  marker ma0000jt04@36

<400>  56
   tcagaagtga tcttcttcct ttcttctcct aaaatytgta tgagctttct attttcttcc      60
   tctaattcag a                                                           71


<210>  57
<211>  58
<212>  DNA
<213>  Artificial Sequence

<220>
<223>  marker ma0016fq81@35

<400>  57
ccgcgaagaa caaacacgtc acacatgaag cgtcygcttc aaaggatctg agtggatg        58


<210>  58
<211>  71
<212>  DNA
<213>  Artificial Sequence

<220>
<223>  marker ma0016fq76@36

<400>  58
ggcaccgaat cgttgatatt cgcgtatact gttgcsatgc aggagaccct gaggaaggcg      60

gacgagatct t                                                           71

<210>  59
<211>  48
<212>  DNA
<213>  Artificial Sequence

<220>
<223>  marker ma0016fq97@18

<400>  59
aaaaaaacga cgtggacygt actaagacgt gtgccagtat aaggctgt                48


<210>  60
<211>  46
<212>  DNA
<213>  Artificial Sequence

<220>
<223>  marker ma0004tn72@11

<400>  60
ttataggccc yaaaacccct tatagtcgtt ggagatgcac tccaaa                  46


<210>  61
<211>  62
<212>  DNA
<213>  Artificial Sequence

<220>
<223>  marker ma0004tn96@36

<400>  61
tgtttacgaa ctatttctgg aggctgccac ataagmtggg tccaccggtt cctacaattt      60

gg                                                                  62


<210>  62
<211>  62
<212>  DNA
<213>  Artificial Sequence

<220>
<223>  marker ma0016fr46@27

<400>  62
tgggtccacc ggttcctaca atttggytat gtgcattacc ggatgagtcg tttaaaataa      60

tt                                                                  62


<210>  63
<211>  71
<212>  DNA
<213>  Artificial Sequence

<220>
<223>  marker ma0011rt36@36

<400>  63

gagaaacttc tcactgcaaa atcattagaa gcagaytgca ttcgaactcg caacacctct 60

ctgagaagac t 71

<210> 64
<211> 71
<212> DNA
<213> Artificial Sequence

<220>
<223> marker ma0022vg84@36

<400> 64
atatccagag aaccgagtta ctgaattctg caaagkctag gcagatggaa atagattcat 60

atttgaggga a 71

<210> 65
<211> 47
<212> DNA
<213> Artificial Sequence

<220>
<223> marker ma0001hr21@12

<400> 65
actatagacg crcttaatga gcaacgcgag aagttacaag agcaaag 47

<210> 66
<211> 66
<212> DNA
<213> Artificial Sequence

<220>
<223> marker ma0012wu17@36

<400> 66
tgagttagaa gagctgaaga ttgaatctga gaacargcaa ctgtctttga gacagtgtgg 60

gaaatc 66

<210> 67
<211> 66
<212> DNA
<213> Artificial Sequence

<220>
<223> marker ma0001hr20@31

<400> 67
gcaactgtct ttgagacagt gtgggaaatc raagcatgct ggtgtggaaa acctggaaga 60

caacgg 66

<210> 68
<211> 71
<212> DNA
<213> Artificial Sequence

<220>
<223>  marker ma0000sa77@36

<400>  68
gagcatgggc actttacact tgcacaaggt tgaccrtaga ttttgaatgg atagcccaca      60

acgaacccag c                                                          71


<210>  69

<400>  69
000

<210>  70
<211>  201
<212>  DNA
<213>  Artificial Sequence

<220>
<223>  marker ma61758s03@101

<400>  70
gatcagcatt tttaactgat tggttgcagc tctatagttg ttgctttagt ttatgtttaa      60

ttggaatgaa agatgtaatc accaatgcca actttgttta ytgggatagg tttcgatgta     120

ttccatagct gggttcgttg tgggctatcc attcaaaatc tayggtcaac cttgtgcaag     180

tgtaaagtgc ccatgctcat g                                               201


<210>  71
<211>  58
<212>  DNA
<213>  Artificial Sequence

<220>
<223>  marker ma0000qw65@23

<400>  71
tgtgacagca acagaggaaa ttrttacaaa actccagaga agtacacaag ggataaga       58


<210>  72
<211>  201
<212>  DNA
<213>  Artificial Sequence

<220>
<223>  marker ma61758s02@101

<400>  72
   tatgtgtagt agattagaaa tagagatgca ggtactcaga aaacactgct agacatttaa      60
   cagtacaaga taarcatggc tactgatcca tctgtgtatt ygttttttct catgtataac     120
   atagatttgg taaactgtgc gagtctgtta cacccgacct tcatccgttt yccctttgct     180
   caatcaggct gggtcaagca g                                               201

<210>  73
<211>  62
<212>  DNA
<213>  Artificial Sequence

<220>
<223>  marker ma0016ft38@27

<400>  73
aatacacaga tggatcagta gccatgytta tcttgtactg ttaaatgtct agcagtgttt        60

tc                                                                        62


<210>  74
<211>  71
<212>  DNA
<213>  Artificial Sequence

<220>
<223>  marker ma0016ft37@36

<400>  74
tatttctaat ctactacaca tagggatgag tacacktgtg gtccttgaat caaatatata        60

tgtaaagaaa t                                                              71


<210>  75
<211>  61
<212>  DNA
<213>  Artificial Sequence

<220>
<223>  marker ma0000wv70@36

<400>  75
tgtggtcctt gaatcaaata tatatgtaaa gaaatsaaat tccacagaat attggagtct        60

t                                                                         61


<210>  76
<211>  62
<212>  DNA
<213>  Artificial Sequence

<220>
<223>  marker ma0004tp85@27

<400>  76
catttaccca accaatatga taatttrtac ttttcatttt tattattcat gcagttttaa        60

ac                                                                        62


<210>  77
<211>  62
<212>  DNA
<213>  Artificial Sequence

<220>
<223>  marker ma0012sk12@36

<400>  77
gaagatcata ataggatagc accacatcta ccaccygaca attcacacca tttctgtcgc        60

tt                                                                        62


<210>  78
<211>  62
<212>  DNA
<213>  Artificial Sequence

<220>
<223>  marker ma0022mm17@27

<400>  78
gacaattcac accatttctg tcgcttyaat atgcagaata caaataaaac acctcgatcc        60

aa                                                                        62


<210>  79
<211>  68
<212>  DNA
<213>  Artificial Sequence

<220>
<223>  marker ma0004tp78@33

<400>  79
gactgtcttg tagtatttgt tctctagttg gcytttgtgg cgtgctagat tcatcagact        60

aatttttg                                                                  68


<210>  80
<211>  71
<212>  DNA
<213>  Artificial Sequence

<220>
<223>  marker ma0022mm15@36

<400>  80
gtgacccttt aaaccacatt taagtcaagt cattgrccct cttatcattt tcttcccctt        60

accaccgatg g                                                              71


<210>  81
<211>  71
<212>  DNA
<213>  Artificial Sequence

<220>
<223>  marker ma0004tp79@36

<400>  81
gtcatcattg gaacctttct tcttatggcc accctygaga catcccttga tccttaattt        60

tctttgcctt c                                                              71

<210>  82
<211>  71
<212>  DNA
<213>  Artificial Sequence

<220>
<223>  marker ma0004tp82@36

<400>  82
tgtctgttaa tggctcaatc tctctcccat aagctractt gaatttgtcc agtgagtaac      60

actcatgcac a                                                            71


<210>  83
<211>  71
<212>  DNA
<213>  Artificial Sequence

<220>
<223>  marker ma0016ft40@36

<400>  83
caagtatccc aaacctctag tatttgtcct cagctraacc atatgcaagt tagaatgata      60

ctcaatatta t                                                            71


<210>  84
<211>  68
<212>  DNA
<213>  Artificial Sequence

<220>
<223>  marker ma0016ft28@36

<400>  84
gaaaaaatat ttcataagta agcatgtcat gtcagytaga tcgggtggat attatgaatt      60

gtgaatat                                                                68


<210>  85
<211>  53
<212>  DNA
<213>  Artificial Sequence

<220>
<223>  marker ma0004tq11@36

<400>  85
ggttcagaga ttttttcaac aaaaaagacc tacagrcacc taatgggcca tac             53


<210>  86
<211>  69
<212>  DNA
<213>  Artificial Sequence

<220>
<223>  marker ma0004tq10@34

<400>  86

tcttgatagt cctgttatgt actgcacatc aaaygataaa actcgtctca tatgaattat 60

ttagtatta 69

<210> 87
<211> 68
<212> DNA
<213> Artificial Sequence

<220>
<223> marker ma0012aq25@36

<400> 87
attttatcat tgaattacta ggactgtacc atactragtg gagatgtcat cataacaaat 60

cttgcaca 68

<210> 88
<211> 201
<212> DNA
<213> Artificial Sequence

<220>
<223> marker ma61757s01@101

<400> 88
atgtttttga tacttaaggc atccaatttg tagctactgc tctagttcta ggatttggar 60

aactccttgt cgaattgtcc cacagagcta atgattgtgc kaacaaaatg acatgatgct 120

gattgttcta cgcctcttgc ttaataccct gtgaagttta attatttggt ggtacacttg 180

accttggaag tcatgacctc c 201

<210> 89
<211> 58
<212> DNA
<213> Artificial Sequence

<220>
<223> marker ma0016ft65@23

<400> 89
ctttgttttt acgaaacccg ctytagttat actgaaggaa ctgatcattt ctttatct 58

<210> 90
<211> 71
<212> DNA
<213> Artificial Sequence

<220>
<223> marker ma0004tq05@36

<400> 90
aacagttgat catggcatgc aaaagaataa catacrctat tgtttttaac ataggttggg 60

agatacaaga g 71

<210>  91
<211>  40
<212>  DNA
<213>  Artificial Sequence

<220>
<223>  marker ma0004tq06@5

<400>  91
gtaakagata taaaccatca tttgcaatct aatgtaccat                              40


<210>  92
<211>  201
<212>  DNA
<213>  Artificial Sequence

<220>
<223>  marker ma61757s03@101

<400>  92
gytctgtgac ttgcagaatg tactatcatt attgcaaatg ccacatccca cagaccttag        60

atacaagtga cggaaagttc tgtgtttact actatatatt ytactttctc caatatattt       120

tggaacaagc tgtacagctc tttattgtat tgcgttgagt craaataagt gatggggtta       180

tttttttgt tttttatta c                                                   201


<210>  93
<211>  201
<212>  DNA
<213>  Artificial Sequence

<220>
<223>  marker ma61757s04@101

<400>  93
cctgcatgaa tcttgtcata ttctacaagc gctaacatag ygccatccta atccatgtgc        60

aacatgaaat atttgtttat gctgcatttg gactatcatg ygttgcaaga ttcataaatc       120

ataatgccaa caagttgtcc tggtattatg actatgttag tacattttag atggattcat       180

acttcgctct tgtcctttgc a                                                 201


<210>  94
<211>  61
<212>  DNA
<213>  Artificial Sequence

<220>
<223>  marker ma0004tq13@26

<400>  94
cccttagttc tttgggtgca catccrcttt tgttccagac ttccagtcaa ctaggtgtcc        60

a                                                                        61


<210>  95

<211>  58
<212>  DNA
<213>  Artificial Sequence

<220>
<223>  marker ma0016ft76@23

<400>  95
tccttttgct ctgtctgcca carttttcat tcagttaaaa gaatatattt gagcaaac          58


<210>  96
<211>  40
<212>  DNA
<213>  Artificial Sequence

<220>
<223>  marker ma0016ft75@5

<400>  96
gggartaaaa ttctctcatt gtcgttgaaa tttatacaga                              40


<210>  97
<211>  60
<212>  DNA
<213>  Artificial Sequence

<220>
<223>  marker ma0022xj97@36

<400>  97
ttactttgat tgccactatt ctaagtttgc attttstttg ttgcaattca tgttaaaatc        60


<210>  98
<211>  41
<212>  DNA
<213>  Artificial Sequence

<220>
<223>  marker ma0004tq07@6

<400>  98
tgtcaygact gaaggtgact gtatctgctt aaagagtatt a                            41


<210>  99
<211>  56
<212>  DNA
<213>  Artificial Sequence

<220>
<223>  marker ma0016ft73@31

<400>  99
gtgtaataga caataagtgc acattagaac ractctttaa tattcctaac tacttc            56


<210>  100
<211>  71
<212>  DNA
<213>  Artificial Sequence

<220>
<223>  marker ma0004tq16@36

<400>  100
gtatggtccg acacatgtgc aaaaagtgct agagakcttc tataggccat cggtctgatg      60

agtatcgttg g                                                          71


<210>  101
<211>  61
<212>  DNA
<213>  Artificial Sequence

<220>
<223>  marker ma0016ft77@36

<400>  101
ggccatcggt ctgatgagta tcgttggatg gtacgrtgga actatgtagt tggcatggtg      60

c                                                                     61


<210>  102
<211>  43
<212>  DNA
<213>  Artificial Sequence

<220>
<223>  marker ma0016ft86@8

<400>  102
tccttggrag ccgttagttt cacagaatta cacatcatat att                       43


<210>  103
<211>  71
<212>  DNA
<213>  Artificial Sequence

<220>
<223>  marker ma0010yr95@36

<400>  103
gaaatcaata tcacacatct tttaaaactt atctargtgt taacacttct tatcgatgta      60

gcatttgcac c                                                          71


<210>  104
<211>  43
<212>  DNA
<213>  Artificial Sequence

<220>
<223>  marker ma0011dy20@8

<400>  104
gatgtctrgt ttattctcat tcatcaacat atatggggtc tta                       43


<210>  105

<211>  201
<212>  DNA
<213>  Artificial Sequence

<220>
<223>  marker ma61755s04@101

<400>  105
tttaacgaaa aaaaggaaaa gaatgacccc agcttccata ttttctgact atccatctyc      60

accctttgga tttctaacct tagatgaaag gaagttggga mtggcgttct ggcttgagtt     120

tatttacatc ctttaattct acaactccaa acgaaaagaa gtttggccaa ttttcctatg     180

tccaatgagt tcccgggcag c                                               201


<210>  106

<400>  106
000

<210>  107
<211>  201
<212>  DNA
<213>  Artificial Sequence

<220>
<223>  marker ma61755s03@101

<400>  107
   actgtcttgt tgcttttata gtgcaatttt aygtcttgat tcctgcctat ttttttcctt      60
   ttcttttttg cttctcgata cattcaccta ctgtcatttg kccatgtttt ttgctaggtc     120
   agaaattgct tggaaaaatg caaattatgc acaaattcta gaatatcttc gggaggaata     180
   tgaactgaat cagcgttttg g                                               201


<210>  108
<211>  201
<212>  DNA
<213>  Artificial Sequence

<220>
<223>  marker ma61755s02@101

<400>  108
aaattcatat caaaatgaac aaatctcagt cagctttgra ccagttgcac atactttgta      60

acattgacct atgctacgaa ttgatttcct tgtgattttg rttgatattt atgcagaaca     120

ttggagtgaa ggataacaga ttttgctatc gctatgtggt tgttttccag tatggttctg     180

ctgtgctttt caacattgct g                                               201


<210>  109
<211>  201
<212>  DNA
<213>  Artificial Sequence

<220>
<223>  marker ma61755s01@101

<400>  109
ccgccccagg aggaggagga cgagggccca gcacgctaca tccccgtcaa agcctacttc      60

ctctccacca ggtaagcacg cagcgccgct cccgcgcttg ytcgataggt gtttgatgat     120

ttatcatcga ggaaacattc gggactttcg ggtgctaccy tactattcta aaaatgtcca     180

tgccaattgg cactgcgaac t                                               201


<210>  110
<211>  67
<212>  DNA
<213>  Artificial Sequence

<220>
<223>  marker ma0016ft81@36

<400>  110
cactaaggta cgaggaacca tacctcatat ttccartgca tcaattatta ctgctttcca      60

tcaaggg                                                                67


<210>  111
<211>  50
<212>  DNA
<213>  Artificial Sequence

<220>
<223>  marker ma0016ft78@36

<400>  111
tttttgctct agctgacaaa tgtgtcagtt tggccraggg ttgagcatgg                 50


<210>  112
<211>  45
<212>  DNA
<213>  Artificial Sequence

<220>
<223>  marker ma0016ft83@36

<400>  112
gtctactttt gcgcaagcca tgaataagac cttcgrcaat ttgat                      45


<210>  113
<211>  45
<212>  DNA
<213>  Artificial Sequence

<220>
<223>  marker ma0004tq35@10

<400>  113
gcgtcacacr cacgaggctg aatcctgaaa aggtcatctt tggtg                      45

<210>  114
<211>  51
<212>  DNA
<213>  Artificial Sequence

<220>
<223>  marker ma0004tq36@16

<400>  114
accactattt tcagtyccat agagtaatgg tagtgacttc ttatccgctg a                51


<210>  115
<211>  62
<212>  DNA
<213>  Artificial Sequence

<220>
<223>  marker ma0004tq32@36

<400>  115
ccaacaacat ggtctagacg acaaagtcgc tcagcrgaag catccaacac gggcagagca       60

tc                                                                      62


<210>  116
<211>  40
<212>  DNA
<213>  Artificial Sequence

<220>
<223>  marker ma0022xj98@10

<400>  116
cgcttttgcy aaggtctcgc tcaatgggtc cattggcaaa                             40


<210>  117
<211>  68
<212>  DNA
<213>  Artificial Sequence

<220>
<223>  marker ma0004tq24@33

<400>  117
ccggtagaag caagagagga gtcagacgag ggygtgatcc cgacaaacat aaattgggtc       60

atccacac                                                                68


<210>  118
<211>  50
<212>  DNA
<213>  Artificial Sequence

<220>
<223>  marker ma0004tq26@36

<400>  118
tggagctgct gctagcaagc ctctttccct tgtccraagg gctagggcca                  50

<210>  119
<211>  71
<212>  DNA
<213>  Artificial Sequence

<220>
<223>  marker ma0004tq30@36

<400>  119
ctctataatc ctaatccatc acatatcaac tatgcytgta tctaatagga taatctaata      60

ttttgccatc t                                                          71


<210>  120
<211>  52
<212>  DNA
<213>  Artificial Sequence

<220>
<223>  marker ma0016ft97@17

<400>  120
gatctgccac tgcttcrctt agactataaa aggagagcac atacgatgca gg             52


<210>  121
<211>  41
<212>  DNA
<213>  Artificial Sequence

<220>
<223>  marker ma0004tq33@6

<400>  121
ctacaktaca agtcatcgat tatttatcat tcaacattta t                         41


<210>  122
<211>  67
<212>  DNA
<213>  Artificial Sequence

<220>
<223>  marker ma0016ft92@32

<400>  122
ctcagttcag tttattgcgc ggcagctttt cstgattgag ctgagctgga aagtgctgaa      60

cccagta                                                               67


<210>  123
<211>  43
<212>  DNA
<213>  Artificial Sequence

<220>
<223>  marker ma0004tq39@8

<400>  123
tatcctcygt ttatatacaa acatagtaca acgcctatgc gtt                       43

<210>  124
<211>  67
<212>  DNA
<213>  Artificial Sequence

<220>
<223>  marker ma0022mm26@36

<400>  124
tcgagcgatg caatgcatgt ggcttcctat cgtatyagga ttgggtgctt tctcaaggcc      60

catctgc                                                                67


<210>  125
<211>  46
<212>  DNA
<213>  Artificial Sequence

<220>
<223>  marker ma0016fu15@11

<400>  125
aaaggctaaa saaaagatct gatatactgc aaaacaaaac ttgact                     46


<210>  126
<211>  44
<212>  DNA
<213>  Artificial Sequence

<220>
<223>  marker ma0010yr98@9

<400>  126
actcataayc tcacgctctc aacattgatg gtgaacagac aata                       44


<210>  127
<211>  65
<212>  DNA
<213>  Artificial Sequence

<220>
<223>  marker ma0022mm25@36

<400>  127
aacattgatg gtgaacagac aatattatgg cgagayaaag gctaaagaaa atatctgata      60

tactg                                                                  65


<210>  128
<211>  40
<212>  DNA
<213>  Artificial Sequence

<220>
<223>  marker ma0016ft98@36

<400>  128

tttatttatt tatttacata gatcgcgcag catccrccct 40

<210> 129
<211> 40
<212> DNA
<213> Artificial Sequence

<220>
<223> marker ma0004tq38@5

<400> 129
ccctycaatg ttctacactg cctgctggcc acaaatctgc 40

<210> 130
<211> 46
<212> DNA
<213> Artificial Sequence

<220>
<223> marker ma0004tq54@36

<400> 130
ctatgcgcta cagtacaaga gttgtacgta agaatycgct agattc 46

<210> 131
<211> 71
<212> DNA
<213> Artificial Sequence

<220>
<223> marker ma0016fu20@36

<400> 131
aagattttgt tatggcaaaa cctgcccagg gcgatytcat gggagtaaac tcagcccatt 60

acaagtttca t 71

<210> 132
<211> 71
<212> DNA
<213> Artificial Sequence

<220>
<223> marker ma0016fu17@36

<400> 132
atccacggga aatatggcat gttatattcg catatrgtct gtagcatagg taaatatgga 60

aagaaaggat a 71

<210> 133
<211> 59
<212> DNA
<213> Artificial Sequence

<220>
<223> marker ma0004tq57@36

<400>  133
atttgttttt ccttgacaca acttccaaga ttgttycaca ggttgccttc aattccctc        59


<210>  134
<211>  71
<212>  DNA
<213>  Artificial Sequence

<220>
<223>  marker ma0016fu11@36

<400>  134
tcaacactaa cacgtatgag atgacttccc aacccwtaga atctagttat ctttcctcta        60

atcgtcttgt t                                                             71


<210>  135
<211>  60
<212>  DNA
<213>  Artificial Sequence

<220>
<223>  marker ma0016fu10@36

<400>  135
tattacttag ataatcatta gcatttttaa gactakaaat ggaacaatgc catgaagaca        60


<210>  136
<211>  40
<212>  DNA
<213>  Artificial Sequence

<220>
<223>  marker ma0016fu09@5

<400>  136
ttcarcacat aataaatcat ttttcatctt ttctaaagca                              40


<210>  137
<211>  42
<212>  DNA
<213>  Artificial Sequence

<220>
<223>  marker ma0004tq43@36

<400>  137
tggcctcaga cgaatcgaaa ctccatctcc catctraggt tg                           42


<210>  138
<211>  58
<212>  DNA
<213>  Artificial Sequence

<220>
<223>  marker ma0016fu01@36

<400>  138

taaggacttc accaatgact tgagcattag tcgcaygacg tcgtgatccg tccatctt 58

<210> 139
<211> 64
<212> DNA
<213> Artificial Sequence

<220>
<223> marker ma0004tq42@36

<400> 139
tcgatcacct tgctctttcc tttagcgtca ttgctrgcga tctcttcagg ttgttagaca 60

caac 64

<210> 140
<211> 61
<212> DNA
<213> Artificial Sequence

<220>
<223> marker ma0016fu05@29

<400> 140
gcgatctctt caggttgtta gacacaacrg aagagaccga atctaatact aattgagagc 60

a 61

<210> 141
<211> 55
<212> DNA
<213> Artificial Sequence

<220>
<223> marker ma0016ft99@36

<400> 141
gaagaaatcc gtagcatcgt caccctatgt gtaatyggcg gccgcacagg acagg 55

<210> 142
<211> 47
<212> DNA
<213> Artificial Sequence

<220>
<223> marker ma0016fu07@12

<400> 142
ggtatggcca awttttggct tggtacaaga tgccctcagc tgaaagc 47

<210> 143
<211> 45
<212> DNA
<213> Artificial Sequence

<220>
<223> marker ma0010yt02@36

<400>  143
gtgattggga gggcatagat attcagtcaa ccagakaatt tcttt                    45


<210>  144
<211>  71
<212>  DNA
<213>  Artificial Sequence

<220>
<223>  marker ma0000gu42@36

<400>  144
tcatccagtt gtttggcttc cccaccaggg tctaargtcc ttaacttttc agatagttgt    60

ttgattttgc g                                                         71


<210>  145
<211>  101
<212>  DNA
<213>  Artificial Sequence

<220>
<223>  marker ma52981s02@51

<400>  145
gttctttttc tcccttttgt actttggggt ttgggcctct ctttctcttc mcctgcgagc    60

ctctttcctc gctcttgtct cctgggcgag ttctgaatct g                        101


<210>  146
<211>  101
<212>  DNA
<213>  Artificial Sequence

<220>
<223>  marker ma52981s01@51

<400>  146
gttccacaca cctccagtga cctgacaacc acaccgcaat aatcgtattc mgttcttttt    60

ctcccttttg tactttgggg tttgggcctc tctttctctt c                         101


<210>  147
<211>  68
<212>  DNA
<213>  Artificial Sequence

<220>
<223>  marker ma0004tq83@33

<400>  147
taaaagaaag tcgcattggc agtgacggct acycgcaact tctcatgtcc ctcgcaacgt    60

aaatgcat                                                             68


<210>  148
<211>  67
<212>  DNA

<213>  Artificial Sequence

<220>
<223>  marker ma0004tq66@36

<400>  148
ctagtgatgc aaaagcatct acactaccat acgcargcag gactttccca ccatgtgatc      60

taaaagt                                                                67


<210>  149
<211>  44
<212>  DNA
<213>  Artificial Sequence

<220>
<223>  marker ma0016fu32@9

<400>  149
tcaaggctwt gtgtttgtcc aattcaaaag tggagagtcc gttt                       44


<210>  150
<211>  47
<212>  DNA
<213>  Artificial Sequence

<220>
<223>  marker ma0011zk10@36

<400>  150
   ggaatttttg agaaaacgag tcttcaatgg aacgcraggt caaacga                     47


<210>  151
<211>  71
<212>  DNA
<213>  Artificial Sequence

<220>
<223>  marker ma0004tq62@36

<400>  151
aaggcttccg gaacgggagc tgaagaaaag agaacmggtg tctgggcgcc ggagcgtttg      60

agcgctgaaa a                                                           71


<210>  152
<211>  40
<212>  DNA
<213>  Artificial Sequence

<220>
<223>  marker ma0004tq68@36

<400>  152
cttggcttct tgcgtctagc tactgagctt cttctrcctc                            40


<210>  153
<211>  71

<212>  DNA
<213>  Artificial Sequence

<220>
<223>  marker ma0004tq78@36

<400>  153
cacaatcaca aactctgagc atctagcagt caaacrcatg aagacagagc agtttacaac      60

tttacatcca t                                                           71


<210>  154
<211>  71
<212>  DNA
<213>  Artificial Sequence

<220>
<223>  marker ma0004tq59@36

<400>  154
tgcgcggatc cagttggtct ccttcgcaca caaatytccc acatttgcgc ccattttccg      60

tgcatgcagc t                                                           71


<210>  155
<211>  40
<212>  DNA
<213>  Artificial Sequence

<220>
<223>  marker ma0022mm29@9

<400>  155
cacttgtgsc ttaaacagca ccaaattgtg cctaggcaat                            40


<210>  156
<211>  101
<212>  DNA
<213>  Artificial Sequence

<220>
<223>  marker ma53009s01@51

<400>  156
gttttgggac ccaaccacta cctccacatt ttgctgcaaa gttcaaccac rcacgctctc      60

tatgccgatt ctttaacatg gagccgttaa tgtagttttt a                         101


<210>  157
<211>  40
<212>  DNA
<213>  Artificial Sequence

<220>
<223>  marker ma0016fu41@7

<400>  157
cacgctstct atgccgattc tttaacatgg agccgttaat                            40

<210>  158
<211>  54
<212>  DNA
<213>  Artificial Sequence

<220>
<223>  marker ma0016fu35@19

<400>  158
gtttgttttc cattgttcyt aggccattgt tcttatgcca tgttggccat attt          54


<210>  159
<211>  58
<212>  DNA
<213>  Artificial Sequence

<220>
<223>  marker ma0016fu36@36

<400>  159
ttcatgacca tgaagtctct accaaaggag tgtgaracat ttcatgttca gtacaaca      58


<210>  160
<211>  71
<212>  DNA
<213>  Artificial Sequence

<220>
<223>  marker ma0004tq69@36

<400>  160
actcatgtgc aaatattcat gttgtcaact ctctayaggg actccgttca agccaaaggc    60

tcctaaaggt c                                                          71


<210>  161
<211>  62
<212>  DNA
<213>  Artificial Sequence

<220>
<223>  marker ma0016fu28@36

<400>  161
tgtagagact agaagagttg tgatccatgg caacargccc tgtgattttc ccaataggca    60

cg                                                                    62


<210>  162
<211>  71
<212>  DNA
<213>  Artificial Sequence

<220>
<223>  marker ma0016fu26@36

<400>  162
gttcctcctc ctaccactaa agctcctact atcaaygcta cttgtccacc accattcact    60

tatccatatc c                                                          71


<210>  163
<211>  71
<212>  DNA
<213>  Artificial Sequence

<220>
<223>  marker ma0010yt03@36

<400>  163
ggccgatgtg tgaggtgccc tatggtgcga aagggrccta gggttgtatt gttgagagaa      60

atatcctcat c                                                          71


<210>  164
<211>  40
<212>  DNA
<213>  Artificial Sequence

<220>
<223>  marker ma0016fu45@36

<400>  164
aagtgggaca tttactacac tgttctgcat cacacyggac                           40


<210>  165
<211>  71
<212>  DNA
<213>  Artificial Sequence

<220>
<223>  marker ma0022mm28@36

<400>  165
gcactgaact gccgccacgt caccaaagcc gttggrgcga agtcaatcgt tgaacctatc      60

agacggtctg g                                                          71


<210>  166
<211>  53
<212>  DNA
<213>  Artificial Sequence

<220>
<223>  marker ma0022xj99@18

<400>  166
aatcatgatt gtccccgyaa aaaatggtgc agtgttttcc ttgtattata gta            53


<210>  167
<211>  65
<212>  DNA
<213>  Artificial Sequence

<220>
<223>  marker ma0016fu43@30

<400>  167
ggatagagta tttagcaaaa gaataatgts tccatatact taataaatga tacttatgtc      60

ataca                                                                  65


<210>  168
<211>  71
<212>  DNA
<213>  Artificial Sequence

<220>
<223>  marker ma0004tq81@36

<400>  168
agtcaggcac acagcacatc tggtctctgc aaagamcaaa gcaagcgcgc gcgtatacgc      60

atgctggaga g                                                           71


<210>  169
<211>  57
<212>  DNA
<213>  Artificial Sequence

<220>
<223>  marker ma0016fu29@22

<400>  169
tgcatcacca tcattccgtg crtgatcgtc cctttaatta cttttggtta agttaac         57


<210>  170
<211>  44
<212>  DNA
<213>  Artificial Sequence

<220>
<223>  marker ma0022mm31@36

<400>  170
atagtgacct ctagctaaga actgatacta aactartttt gaca                       44


<210>  171
<211>  40
<212>  DNA
<213>  Artificial Sequence

<220>
<223>  marker ma0010yt04@9

<400>  171
tttaggttrc agagagaatt tctcggagca ttgaacttgt                            40


<210>  172
<211>  67
<212>  DNA
<213>  Artificial Sequence

<220>

<223>  marker ma0004tr07@32

<400>  172
cagagagaat ttctcggagc attgaacttg tygtctacgg cccatttcac gtaattcctg      60

ataaagt                                                                67


<210>  173
<211>  59
<212>  DNA
<213>  Artificial Sequence

<220>
<223>  marker ma0012fz92@36

<400>  173
agtatataat atgccaatct gggatcggac gagtaygatg cagccacgtt ttccagtcg       59


<210>  174
<211>  71
<212>  DNA
<213>  Artificial Sequence

<220>
<223>  marker ma0001ac17@36

<400>  174
cgctacgcgt ggaagccgaa ccttggtcag aagatmaacg aaaccgtaag ctctcctccc      60

atcgccttcg c                                                           71


<210>  175
<211>  71
<212>  DNA
<213>  Artificial Sequence

<220>
<223>  marker ma0012pg35@36

<400>  175
aggttccgac ctctgtcaga aatcaccggc gtctgycagc gctgccggga tcaaatcgac      60

tggaaacgca g                                                           71


<210>  176
<211>  71
<212>  DNA
<213>  Artificial Sequence

<220>
<223>  marker ma0000en22@36

<400>  176
gcgctgccgg gatcaaatcg actggaaacg caggtsggga attgtcatcc ctcatcggtt      60

gtctaagctc a                                                           71


<210>  177

<211>  40
<212>  DNA
<213>  Artificial Sequence

<220>
<223>  marker ma0000en21@36

<400>  177
atttagctag tgtgtagctt cgattgtctc ctaggyccaa                              40


<210>  178
<211>  50
<212>  DNA
<213>  Artificial Sequence

<220>
<223>  marker ma0016fu62@36

<400>  178
aatctctttc cctgtttagc acgtatactg agcagygaga gaggtgacgc                   50


<210>  179
<211>  71
<212>  DNA
<213>  Artificial Sequence

<220>
<223>  marker ma0004tq90@36

<400>  179
atgcagaggt gaattttggc cactcaatct cttgtktcac taggcctggt tgttacttgt        60

taaacattga t                                                             71


<210>  180
<211>  101
<212>  DNA
<213>  Artificial Sequence

<220>
<223>  marker ma08364s01@51

<400>  180
tctcttgtgt cactaggcct ggttgttact tgttaaacat tgataggttg rattgttact        60

gatccgggtt tgctgctgct taatgcagat tgctccaagg a                           101


<210>  181
<211>  71
<212>  DNA
<213>  Artificial Sequence

<220>
<223>  marker ma0011zt60@36

<400>  181
ctcggcatat gtgctaagtg ctgcacatgt gttaaygagc ttgtcggaag gtgtgattcc        60

tgctgtgcat t                                                             71

<210>  182
<211>  42
<212>  DNA
<213>  Artificial Sequence

<220>
<223>  marker ma0004tq98@7

<400>  182
taattckatt tagggatgcg aatgaggagg aaagtgaacg ca                    42


<210>  183
<211>  71
<212>  DNA
<213>  Artificial Sequence

<220>
<223>  marker ma0016fu52@36

<400>  183
ggaacgacgt acacttcttc gtattgtgag tactgytttt caacttttca cactgaacaa      60

acttttgttc g                                                          71


<210>  184
<211>  46
<212>  DNA
<213>  Artificial Sequence

<220>
<223>  marker ma0016fu55@11

<400>  184
tttaaaactt magtattttc tcactgttgc caattgaata attttt                    46


<210>  185
<211>  59
<212>  DNA
<213>  Artificial Sequence

<220>
<223>  marker ma0011dy21@24

<400>  185
gagctagggt cctgttttgg caargttcca tccagtgaaa ccttacgata caatgaaaa      59


<210>  186
<211>  71
<212>  DNA
<213>  Artificial Sequence

<220>
<223>  marker ma0004tq88@36

<400>  186
cagatgtaga ggctggagat tttgtggaag attaartttg gaatgcttta tgctgtaaag      60

acgatacttt c                                                          71

<210>  187
<211>  40
<212>  DNA
<213>  Artificial Sequence

<220>
<223>  marker ma0022xk01@5

<400>  187
tggtmggatt gtcgcgcatt tccttatgtt tgttcgaaag                          40

<210>  188
<211>  71
<212>  DNA
<213>  Artificial Sequence

<220>
<223>  marker ma0016fu57@36

<400>  188
ggattgtcgc gcatttcctt atgtttgttc gaaagyttgg aattggaaga tgttaaactg    60

cgttgattta a                                                         71

<210>  189
<211>  71
<212>  DNA
<213>  Artificial Sequence

<220>
<223>  marker ma0010yt06@36

<400>  189
aacgttgcaa attaccgata tgcaaagcaa agcgtyacgc aggtaaagcc agatcttgtc    60

cgtaggctgc c                                                         71

<210>  190
<211>  47
<212>  DNA
<213>  Artificial Sequence

<220>
<223>  marker ma0001dg29@12

<400>  190
gactcagtac arctgcatgt ggtggaggaa ttatggcaaa cattgca                  47

<210>  191
<211>  40
<212>  DNA
<213>  Artificial Sequence

<220>
<223>  marker ma0016fu59@7

<400>  191
cagtggrccc gacaatgtaa ggaactcgta gcaaaccttt                              40


<210>  192
<211>  49
<212>  DNA
<213>  Artificial Sequence

<220>
<223>  marker ma0016fu58@34

<400>  192
cccgacaatg taaggaactc gtagcaaacc tttyctaaag tgcccttca                    49


<210>  193
<211>  42
<212>  DNA
<213>  Artificial Sequence

<220>
<223>  marker ma0004tr05@36

<400>  193
ggttgaacct agtgagaggt caactaataa gagatmaaaa gg                           42


<210>  194
<211>  57
<212>  DNA
<213>  Artificial Sequence

<220>
<223>  marker ma0004tr10@22

<400>  194
aacgcttcga ctactacaaa cstaatgttt ataggatagg aaattctccg atcacct           57


<210>  195
<211>  41
<212>  DNA
<213>  Artificial Sequence

<220>
<223>  marker ma0004tr03@36

<400>  195
gataggaaat tctccgatca cctacacaag aatatrcgaa t                            41


<210>  196
<211>  69
<212>  DNA
<213>  Artificial Sequence

<220>
<223>  marker ma0004tq84@36

<400>  196
ggagttgctg gatcatatac cagaaaccag tatttycaga gaagtgcacg agagagcagt        60

gacagtgaa                                                              69

<210>  197
<211>  52
<212>  DNA
<213>  Artificial Sequence

<220>
<223>  marker ma0016fu56@17

<400>  197
atgtgttttg tgttccraaa acttcaaatc taaccatgat gaattggatg ga              52

<210>  198
<211>  44
<212>  DNA
<213>  Artificial Sequence

<220>
<223>  marker ma0011jn34@9

<400>  198
agagaattkt tttgagtcac ccttgatccc attagcattg tttt                       44

<210>  199
<211>  60
<212>  DNA
<213>  Artificial Sequence

<220>
<223>  marker ma0016fu80@25

<400>  199
tactactacg gttcatacca tcatyagcac tatatataaa caccgtatcg atcggcccgt      60

<210>  200
<211>  42
<212>  DNA
<213>  Artificial Sequence

<220>
<223>  marker ma0004tr23@36

<400>  200
tgttgcatcg taaacgacct gctgatcggt gtcctsatcg gc                         42

<210>  201
<211>  1143
<212>  DNA
<213>  Artificial Sequence

<220>
<223>  cDNA of Zm00001d043358 (restorer)

<400>  201
atggggcggc ttcgcgcgtg ctgccgtctc caccgcttcc tcgcgccgcc gccggcacaa      60

gtgccatcac cgggacaatc tctggcccgc gggtccaaca ccgctaccgc tcacgtagcg     120

cctttctcga ggcttctctc gtcggccgcg gccgttgcgc cccacgacac acgcgactcg 180

ggcctcggcg gttcggcgta ctgggcctgg attcgcgcgg cgaccgagtc ggccccggct 240

ccttcgccgc cccaggagga ggaggacgag ggcccagcac gctacatccc cgtcaaagcc 300

tacttcctct ccaccagcat tgatctgaag agcatgcaag cggagcacgg gaatgatatt 360

gtacctccgt cgacccgttt gcttaactac attgcgcttc ggtactccga gttcccacct 420

gagattatga acattggagt gaaggataac agattttgct atcgctatgt ggttgttttc 480

cagtatggtt ctgctgtgct tttcaacatt gctgatcatg aagccgagta ctatcttgat 540

ataattagga agcatgcttc aggatggctt ccagagatga gaaaagatga ttatgcagtg 600

gttgagaaac catccttgac aacatggatg aaaggaggac ttgattatat agttcttaaa 660

agtttagata cagacgggat tcgcataatt tcaagcgttc ttggtcaaag tatcgccctt 720

gatcactata ttcggcaggt actgaaactc aaagttaatg aacttatcac tgaaattaat 780

cgtgtcatgg agaaaaccgg taactttacc atgcaaagaa aaaaactctt tcaacttgtg 840

ggtaaggcca attctaatct tgcggatgtt atcatcagac ttggtctttt tgacaggtca 900

gaaattgctt ggaaaaatgc aaattatgca caaattctag aatatcttcg ggaggaatat 960

gaactgaatc agcgttttgg aagccttgac ttcaaactga aatttgtgga gcataacatt 1020

cactttcttc aagaggtcct ccaaaacaga cggtctgatc tgctggaatg gggtgtcata 1080

atactgctga ttattgagat tgctatctca ttatatgaaa ttgccaagga ctccatgatc 1140

gat 1143

<210> 202
<211> 381
<212> PRT
<213> Zea mays

<400> 202

Met Gly Arg Leu Arg Ala Cys Cys Arg Leu His Arg Phe Leu Ala Pro
1               5                   10                  15

Pro Pro Ala Gln Val Pro Ser Pro Gly Gln Ser Leu Ala Arg Gly Ser
            20                  25                  30

Asn Thr Ala Thr Ala His Val Ala Pro Phe Ser Arg Leu Leu Ser Ser
        35                  40                  45

Ala Ala Ala Val Ala Pro His Asp Thr Arg Asp Ser Gly Leu Gly Gly
    50                  55                  60

Ser Ala Tyr Trp Ala Trp Ile Arg Ala Ala Thr Glu Ser Ala Pro Ala
65                  70                  75                  80

Pro Ser Pro Pro Gln Glu Glu Glu Asp Glu Gly Pro Ala Arg Tyr Ile
                85                  90                  95

Pro Val Lys Ala Tyr Phe Leu Ser Thr Ser Ile Asp Leu Lys Ser Met
            100                 105                 110

Gln Ala Glu His Gly Asn Asp Ile Val Pro Pro Ser Thr Arg Leu Leu
        115                 120                 125

Asn Tyr Ile Ala Leu Arg Tyr Ser Glu Phe Pro Pro Glu Ile Met Asn
    130                 135                 140

Ile Gly Val Lys Asp Asn Arg Phe Cys Tyr Arg Tyr Val Val Val Phe
145                 150                 155                 160

Gln Tyr Gly Ser Ala Val Leu Phe Asn Ile Ala Asp His Glu Ala Glu
                165                 170                 175

Tyr Tyr Leu Asp Ile Ile Arg Lys His Ala Ser Gly Trp Leu Pro Glu
            180                 185                 190

Met Arg Lys Asp Asp Tyr Ala Val Val Glu Lys Pro Ser Leu Thr Thr
        195                 200                 205

Trp Met Lys Gly Gly Leu Asp Tyr Ile Val Leu Lys Ser Leu Asp Thr
    210                 215                 220

Asp Gly Ile Arg Ile Ile Ser Ser Val Leu Gly Gln Ser Ile Ala Leu
225                 230                 235                 240

Asp His Tyr Ile Arg Gln Val Leu Lys Leu Lys Val Asn Glu Leu Ile
                245                 250                 255

Thr Glu Ile Asn Arg Val Met Glu Lys Thr Gly Asn Phe Thr Met Gln
            260                 265                 270

Arg Lys Lys Leu Phe Gln Leu Val Gly Lys Ala Asn Ser Asn Leu Ala
        275                 280                 285

Asp Val Ile Ile Arg Leu Gly Leu Phe Asp Arg Ser Glu Ile Ala Trp
    290                 295                 300

Lys Asn Ala Asn Tyr Ala Gln Ile Leu Glu Tyr Leu Arg Glu Glu Tyr
305                 310                 315                 320

Glu Leu Asn Gln Arg Phe Gly Ser Leu Asp Phe Lys Leu Lys Phe Val

325                 330                 335

Glu His Asn Ile His Phe Leu Gln Glu Val Leu Gln Asn Arg Arg Ser
            340                 345                 350

Asp Leu Leu Glu Trp Gly Val Ile Ile Leu Leu Ile Ile Glu Ile Ala
        355                 360                 365

Ile Ser Leu Tyr Glu Ile Ala Lys Asp Ser Met Ile Asp
    370                 375                 380

<210>  203
<211>  525
<212>  DNA
<213>  Artificial Sequence

<220>
<223>  cDNA of Zm00001d043352 (restorer)

<400>  203
atgaggctgc tgcaagcgga ggagctattt cggaaggttc tggaggtcgg gtcgaagaat      60

aaggcggctc ggctgctggg gctcgacgtc ggcagcaagt acgtcggggt ggccatctcc     120

gatgagaaga acagggtcgc tatgcctctg agtgttttgt gtcggacaaa aacaaacatc     180

aacttgatgg cagatgattt caaaacattg gtttcgatgt attccatagc tgggttcgtt     240

gtgggctatc cattcaaaat ctacggtcaa ccttgtgcaa gtgcaatcca agtaaggcat     300

cttgctgggg aactttgtaa aacagggaaa cttgatgatc tgccctacgc atattgggat     360

gaaaatttca cctcaaagtg tgtagaagcc ctcttgcatc ccctgaatct aaaagacttg     420

gacgatgcca aaacaatgac agacaaattt gctgcagtct gtatactcca gggttatctt     480

gacaacatga acagaaaact gagatccaca gataagtctg aagca                     525

<210>  204
<211>  175
<212>  PRT
<213>  Zea mays

<400>  204

Met Arg Leu Leu Gln Ala Glu Glu Leu Phe Arg Lys Val Leu Glu Val
1               5                   10                  15

Gly Ser Lys Asn Lys Ala Ala Arg Leu Leu Gly Leu Asp Val Gly Ser
            20                  25                  30

Lys Tyr Val Gly Val Ala Ile Ser Asp Glu Lys Asn Arg Val Ala Met
        35                  40                  45

Pro Leu Ser Val Leu Cys Arg Thr Lys Thr Asn Ile Asn Leu Met Ala

50                  55                  60

Asp Asp Phe Lys Thr Leu Val Ser Met Tyr Ser Ile Ala Gly Phe Val
65                  70                  75                  80

Val Gly Tyr Pro Phe Lys Ile Tyr Gly Gln Pro Cys Ala Ser Ala Ile
                85                  90                  95

Gln Val Arg His Leu Ala Gly Glu Leu Cys Lys Thr Gly Lys Leu Asp
            100                 105                 110

Asp Leu Pro Tyr Ala Tyr Trp Asp Glu Asn Phe Thr Ser Lys Cys Val
        115                 120                 125

Glu Ala Leu Leu His Pro Leu Asn Leu Lys Asp Leu Asp Asp Ala Lys
    130                 135                 140

Thr Met Thr Asp Lys Phe Ala Ala Val Cys Ile Leu Gln Gly Tyr Leu
145                 150                 155                 160

Asp Asn Met Asn Arg Lys Leu Arg Ser Thr Asp Lys Ser Glu Ala
                165                 170                 175

<210>  205
<211>  936
<212>  DNA
<213>  Artificial Sequence

<220>
<223>  cDNA of Zm00001d043356 (restorer)

<400>  205
atggccttca agctggccac caaggccgcg gcggcgtcgc ccgctgctgc tcaccgcggg      60

ggtctcgccc gggggccgga gggtacgagc cgcgttgcct tcggaccagc gcctagaaac     120

aaggggctcc gcgcggccaa caactccgcg acgcccgtgg ctaaggaaga gagggttgat     180

cgaagtgaaa tattgacatt ggatagcatt agacaagttt tgattagact agaagacagc     240

atcatattcg gccttttgga gagagcacag ttttgttaca acgctgatac atatgatagc     300

aatgctttcc acatggatgg ttttggcggc tctttggttg aatatatggt tagagaaact     360

gaaaagctcc atgcacaggt tgggagatac aagagcccag atgagcaccc tttctttcct     420

gaggatctgc ctgagccccg gttgccacct atgcagtacc caagggtttt gcatcccatt     480

gccgattcta tcaatatcaa caaagagatt tggaaaatgt attttgatga acttcttcca     540

agattggtga aaaaaggaag tgatggtaat gctggatcca gtgctctttg tgacacgacc     600

tgcttgcagg cgctctccaa aaggatccac tatgggaagt ttgtggcaga ggctaagttt     660

caggagtccc cggaagctta catgccagcc ataatagctc aggaccgtga tcaactcatg     720

caccttctca catatgaaac ggtggagcgt gctatcgaac acagggtgga agccaaagcc 780

aagatcttcg ggcaagaggt gaacatcggt gtggaggaca acggcagccc accggtgtac 840

aagatcgttc cgagcttggt cgccgagctg tacagctaca gaatcatgcc gctaaccaaa 900

gaggttcaaa tcgcgtattt gcttaggagg ctggat 936

<210> 206
<211> 312
<212> PRT
<213> Zea mays

<400> 206

Met Ala Phe Lys Leu Ala Thr Lys Ala Ala Ala Ala Ser Pro Ala Ala
1               5                   10                  15

Ala His Arg Gly Gly Leu Ala Arg Gly Pro Glu Gly Thr Ser Arg Val
            20                  25                  30

Ala Phe Gly Pro Ala Pro Arg Asn Lys Gly Leu Arg Ala Ala Asn Asn
        35                  40                  45

Ser Ala Thr Pro Val Ala Lys Glu Glu Arg Val Asp Arg Ser Glu Ile
    50                  55                  60

Leu Thr Leu Asp Ser Ile Arg Gln Val Leu Ile Arg Leu Glu Asp Ser
65                  70                  75                  80

Ile Ile Phe Gly Leu Leu Glu Arg Ala Gln Phe Cys Tyr Asn Ala Asp
                85                  90                  95

Thr Tyr Asp Ser Asn Ala Phe His Met Asp Gly Phe Gly Gly Ser Leu
            100                 105                 110

Val Glu Tyr Met Val Arg Glu Thr Glu Lys Leu His Ala Gln Val Gly
        115                 120                 125

Arg Tyr Lys Ser Pro Asp Glu His Pro Phe Phe Pro Glu Asp Leu Pro
    130                 135                 140

Glu Pro Arg Leu Pro Pro Met Gln Tyr Pro Arg Val Leu His Pro Ile
145                 150                 155                 160

Ala Asp Ser Ile Asn Ile Asn Lys Glu Ile Trp Lys Met Tyr Phe Asp
                165                 170                 175

Glu Leu Leu Pro Arg Leu Val Lys Lys Gly Ser Asp Gly Asn Ala Gly
            180                 185                 190

Ser Ser Ala Leu Cys Asp Thr Thr Cys Leu Gln Ala Leu Ser Lys Arg
        195                 200                 205

Ile His Tyr Gly Lys Phe Val Ala Glu Ala Lys Phe Gln Glu Ser Pro
    210                 215                 220

Glu Ala Tyr Met Pro Ala Ile Ile Ala Gln Asp Arg Asp Gln Leu Met
225                 230                 235                 240

His Leu Leu Thr Tyr Glu Thr Val Glu Arg Ala Ile Glu His Arg Val
                245                 250                 255

Glu Ala Lys Ala Lys Ile Phe Gly Gln Glu Val Asn Ile Gly Val Glu
            260                 265                 270

Asp Asn Gly Ser Pro Pro Val Tyr Lys Ile Val Pro Ser Leu Val Ala
        275                 280                 285

Glu Leu Tyr Ser Tyr Arg Ile Met Pro Leu Thr Lys Glu Val Gln Ile
    290                 295                 300

Ala Tyr Leu Leu Arg Arg Leu Asp
305                 310

<210>  207
<211>  540
<212>  DNA
<213>  Artificial Sequence

<220>
<223>  cDNA of Zm00001d043356 (restorer) - 5′ end

<400>  207
atggctaccg cggcgcgcct cctcctcgtc gtggtgcttc tctccgcggc gataccgatc      60

ccgtccttcg cgacaagctt cgtcgtcggc gacaagaggc atcgctgggc tcccaacgtc     120

aactatacag actgggcgga ccgtcaccag ttccacgtcg gcgactggct cgaattcagg     180

tacgagaggg acaggttcga tgtggtgcag gtgaacgaga cggcatacgc ggcgtgcgac     240

gccagcagcc ccatcctcag ctacagccgc ggccacaact tcgtcttccg gctcaaccac     300

acgggccggt tctacttcat ctgcagccgc ggctactgct ggagtggcat gaaggtctcc     360

gtgctcgtcc agccgccgcc gcccccgtcg ctgccgccgg cgtcccactc ccacgcgtca     420

agcgccagtg cgcgtgtgcg gccggcggcc gggctttggt gtgcggctct ctctaccttg     480

ctgggctggg cggtgctcac gccgccgctg tcgtttcggg tctggagttc tggatgtcca     540

<210>  208

<211>  180
<212>  PRT
<213>  Zea mays

<400>  208

Met Ala Thr Ala Ala Arg Leu Leu Leu Val Val Val Leu Leu Ser Ala
1               5                   10                  15

Ala Ile Pro Ile Pro Ser Phe Ala Thr Ser Phe Val Val Gly Asp Lys
            20                  25                  30

Arg His Arg Trp Ala Pro Asn Val Asn Tyr Thr Asp Trp Ala Asp Arg
        35                  40                  45

His Gln Phe His Val Gly Asp Trp Leu Glu Phe Arg Tyr Glu Arg Asp
    50                  55                  60

Arg Phe Asp Val Val Gln Val Asn Glu Thr Ala Tyr Ala Ala Cys Asp
65                  70                  75                  80

Ala Ser Ser Pro Ile Leu Ser Tyr Ser Arg Gly His Asn Phe Val Phe
                85                  90                  95

Arg Leu Asn His Thr Gly Arg Phe Tyr Phe Ile Cys Ser Arg Gly Tyr
            100                 105                 110

Cys Trp Ser Gly Met Lys Val Ser Val Leu Val Gln Pro Pro Pro Pro
        115                 120                 125

Pro Ser Leu Pro Pro Ala Ser His Ser His Ala Ser Ser Ala Ser Ala
    130                 135                 140

Arg Val Arg Pro Ala Ala Gly Leu Trp Cys Ala Ala Leu Ser Thr Leu
145                 150                 155                 160

Leu Gly Trp Ala Val Leu Thr Pro Pro Leu Ser Phe Arg Val Trp Ser
                165                 170                 175

Ser Gly Cys Pro
            180

## Description

### FIELD OF THE INVENTION

The invention relates to methods for identifying plants or plant parts, in particular maize plants or plant parts having a restorer of fertility genotype or phenotype, in particular a cytoplasmic fertility restorer of fertility genotype or phenotype. The present invention also relates to plants identified as such, as well as methods for generating such plants. The present invention further relates to polynucleic acids and polypeptides suitable for identifying or generating such plants.

### BACKGROUND OF THE INVENTION

With the aim to inhibit self-pollination of female lines in hybrid production, CMS (cytoplasmic male sterility) is applied in plant breeding programs worldwide. CMS is characterized by maternally inherited mutations, which result in plants that are unable to produce pollen. (Schnable, P. S., & Wise, R. P. (1998). The molecular basis of cytoplasmic male sterility and fertility restoration. Trends in plant science, 3(5), 175-180.)

In order to ensure that the hybrid progeny is entirely fertile and able to produce seeds, male parental lines in hybrid production need to possess so called restorer genes, which cover and cancel the effects of CMS.

For the maize cms plasma cmsC, the major restorer locus RF4 at the beginning of chromosome 8, is well known (WO 2012/047595). Before conversion of a female line to CMSC, it has to be guaranteed, that RF4 is not active in this line. Otherwise, the maintainer locus has to be introgressed before CMS conversion is possible. Defining restorer genotype is done both by marker application and by phenotypic observations.

It is an objective of the present invention to identify new restorer genotypes for use in plant breeding programs, in particular maize plant breeding programs including the use of CMS. The identification of new restorer genotypes, in particular different from the restorer genotypes already known on maize chromosome 8 expands their usability in plant breeding, in particular introgression.

### SUMMARY OF THE INVENTION

The present invention relates to plants or plant parts, in particular maize plants or plants parts having a restorer genotype or phenotype and their use. The restorer genotype or phenotype in particular refers to a cytoplasmic male sterility (CMS) restorer genotype or phenotype, i.e. a genotype or phenotype which restores male fertility.

Preferably, the restorer genotype is caused by one or more restorer genes which are located on maize chromosome 3, i.e. RF-03-01.

The present invention advantageously allows to identify maize lines having a CMS restorer phenotype, which comprise well known restorer genes or loci, such as RF4. The different chromosomal location of the restorer locus of the present invention compared to known restorer loci expands the tool kit for generating as well as maintaining restorer lines, or alternatively for ensuring that an unwanted restorer genotype/phenotype remains absent or can be selected against.

The present invention is in particular captured by any one or any combination of one or more of the below numbered statements 1 to 56, which can be combined with any other statements and/or embodiments.
1. A method for identifying a (maize) plant or plant part, comprising screening for the presence of, detecting, or identifying (a haplotype associated with) a cytoplasmic male sterility (CMS) (fertility) restorer locus on chromosome 3 (RF-03-01).
2. The method according to statement 1, wherein said locus comprises or is comprised in a region on chromosome 3 corresponding to positions 195629901 to 198023573 of B73 AGPv4, or a fragment thereof.
3. The method according to statement 1 or 2, wherein said locus comprises or is comprised in a region on chromosome 3 corresponding to positions 197453646 to 197698278 of B73 AGPv4, or a fragment thereof.
4. The method according to any of statements 1 to 3, wherein said locus comprises one or more of molecular marker(s) (alleles) of Table 4 or Table 5 .
5. The method according to any of statements 1 to 4, wherein said locus comprises one or more of a polynucleic acid comprising one or more of SEQ ID NOs: 17 to 200 .
6. The method according to any of statements 1 to 5, wherein said locus comprises one or more of a polynucleic acid comprising one or more of SEQ ID NOs: 68 to 140.
7. The method according to any of statements 1 to 6, wherein said locus comprises one or more of Zm00001d043358, Zm00001d043352, Zm00001d043356, and Zm00001d043357.
8. The method according to any of statements 1 to 7, wherein said locus comprises Zm00001d043358.
9. The method according to any of statements 1 to 8, wherein said locus comprises a polynucleic acid comprising one or more of SEQ ID NOs: 1, 5, 9, and 13 or one or more of SEQ ID NOs: 2, 6, 10, and 14.
10. The method according to any of statements 1 to 9, wherein said locus comprises a polynucleic acid comprising SEQ ID NOs: 1 or 2.
11. The method according to any of statements 1 to 10, comprising screening for the presence of any one or more of SEQ ID NOs: 17 to 200.
12. The method according to any of statements 1 to 11, comprising screening for the presence of any one or more of SEQ ID NOs: 68 to 140.
13. The method according to any of statements 1 to 12, comprising screening for the presence of any one or more of SEQ ID NOs: 70, 72, 76, 79, 86, 88, 92, 93, 99, 104, 105, 107, 108, 109, 115, and 134.
14. The method according to any of statements 1 to 13, comprising screening for the presence of any one or more of SEQ ID NOs: 1, 5, 9, and 13 or one or more of SEQ ID NOs: 2, 6, 10, and 14, or a (unique) fragment thereof.
15. The method according to any of statements 1 to 14, comprising screening for the presence of SEQ ID NOs: 1 or 2, or a (unique) fragment thereof.
16. The method according to any of statements 1 to 14, comprising screening for the presence of one or more of Zm00001d043358, Zm00001d043352, Zm00001d043356, and/or Zm00001d043357, or a fragment thereof, wherein
Zm00001 d043358
   has a genomic sequence of SEQ ID NO: 1 or 2;
   has a coding sequence of SEQ ID NO: 201 or 3; and/or
   encodes a protein having a sequence of SEQ ID NO: 202 or 4;
Zm00001 d043352
   has a genomic sequence of SEQ ID NO: 5 or 6;
   has a coding sequence of SEQ ID NO: 203 or 7; and/or
   encodes a protein having a sequence of SEQ ID NO: 204 or 8;
Zm00001 d043356
   has a genomic sequence of SEQ ID NO: 9 or 10;
   has a coding sequence of SEQ ID NO: 205 or 11; and/or
   encodes a protein having a sequence of SEQ ID NO: 206 or 12;
Zm00001d043357
   has a genomic sequence of SEQ ID NO: 13 or 14;
   has a coding sequence of SEQ ID NO: 207 or 15; and/or
   encodes a protein having a sequence of SEQ ID NO: 208 or 16.
17. The method according to any of statements 1 to 16, comprising screening for the presence of any one or more molecular markers (alleles) of Table 4 or Table 5.
18. A method for identifying a (maize) plant or plant part (having a restorer of fertility locus on chromosome 3), comprising screening for the presence of any one or more molecular markers (alleles)of Table 4 or Table 5.
19. A method for identifying a (maize) plant or plant part (having a restorer of fertility locus on chromosome 3), comprising screening for the presence of any one or more of SEQ ID NOs: 17 to 200.
20. A method for identifying a (maize) plant or plant part (having a restorer of fertility locus on chromosome 3), comprising screening for the presence of any one or more of SEQ ID NOs: 68 to 140.
21. A method for identifying a (maize) plant or plant part (having a restorer of fertility locus on chromosome 3), comprising screening for the presence of any one or more of SEQ ID NOs: 70, 72, 76, 79, 86, 88, 92, 93, 99, 104, 105, 107, 108, 109, 115, and 134.
22. A method for identifying a (maize) plant or plant part (having a restorer of fertility locus on chromosome 3), comprising screening for the presence of any one or more of SEQ ID NOs: 1, 5, 9, and 13 or one or more of SEQ ID NOs: 2, 6, 10, and 14, or a (unique) fragment thereof.
23. A method for identifying a (maize) plant or plant part (having a restorer of fertility locus on chromosome 3), comprising screening for the presence of SEQ ID NOs: 1 or 2, or a (unique) fragment thereof.
24. A method for identifying a (maize) plant or plant part (having a restorer of fertility locus on chromosome 3), comprising screening for the presence of Zm00001d043358, Zm00001d043352, Zm00001 d043356, and/or Zm00001 d043357, or a fragment thereof, wherein
Zm00001d043358
   has a genomic sequence of SEQ ID NO: 1 or 2;
   has a coding sequence of SEQ ID NO: 201 or 3; and/or
   encodes a protein having a sequence of SEQ ID NO: 202 or 4;
Zm00001d043352
   has a genomic sequence of SEQ ID NO: 5 or 6;
   has a coding sequence of SEQ ID NO: 203 or 7; and/or
   encodes a protein having a sequence of SEQ ID NO: 204 or 8;
Zm00001d043356
   has a genomic sequence of SEQ ID NO: 9 or 10;
   has a coding sequence of SEQ ID NO: 205 or 11; and/or
   encodes a protein having a sequence of SEQ ID NO: 206 or 12;
Zm00001 d043357
   has a genomic sequence of SEQ ID NO: 13 or 14;
   has a coding sequence of SEQ ID NO: 207 or 15; and/or
   encodes a protein having a sequence of SEQ ID NO: 208 or 16.
25. An (isolated) polynucleic acid comprising one or more molecular marker (allele) of Table 4 or Table 5, or the complement or reverse complement of said polynucleic acid.
26. An (isolated) polynucleic acid comprising one or more nucleotides corresponding to an SNP of Table 4, or the complement or reverse complement of said polynucleic acid.
27. An (isolated) polynucleic acid comprising at least 15 contiguous nucleotides comprised in a region corresponding to a region flanked by any of the indicated 5' and 3' positions of the table below and comprising the nucleotide corresponding to the position of the indicated SNP referenced to maize chromosome 3, B73 AGPv4

| 5' (-100 nt) | 5' (-50 nt) | SNP | 3' (+50 nt) | 3' (+100 nt) |
|---|---|---|---|---|
| 195629801 | 195629851 | 195629901 | 195629951 | 195630001 |
| 195639594 | 195639644 | 195639694 | 195639744 | 195639794 |
| 195677699 | 195677749 | 195677799 | 195677849 | 195677899 |
| 195678256 | 195678306 | 195678356 | 195678406 | 195678456 |
| 195680690 | 195680740 | 195680790 | 195680840 | 195680890 |
| 195732836 | 195732886 | 195732936 | 195732986 | 195733036 |
| 195733816 | 195733866 | 195733916 | 195733966 | 195734016 |
| 195783601 | 195783651 | 195783701 | 195783751 | 195783801 |
| 196069986 | 196070036 | 196070086 | 196070136 | 196070186 |
| 196198636 | 196198686 | 196198736 | 196198786 | 196198836 |
| 196244614 | 196244664 | 196244714 | 196244764 | 196244814 |
| 196653646 | 196653696 | 196653746 | 196653796 | 196653846 |
| 196693401 | 196693451 | 196693501 | 196693551 | 196693601 |
| 196702711 | 196702761 | 196702811 | 196702861 | 196702911 |
| 196703908 | 196703958 | 196704008 | 196704058 | 196704108 |
| 196703996 | 196704046 | 196704096 | 196704146 | 196704196 |
| 196704069 | 196704119 | 196704169 | 196704219 | 196704269 |
| 196704190 | 196704240 | 196704290 | 196704340 | 196704390 |
| 196705370 | 196705420 | 196705470 | 196705520 | 196705570 |
| 196706597 | 196706647 | 196706697 | 196706747 | 196706797 |
| 196706655 | 196706705 | 196706755 | 196706805 | 196706855 |
| 196707090 | 196707140 | 196707190 | 196707240 | 196707290 |
| 196707315 | 196707365 | 196707415 | 196707465 | 196707515 |
| 196707897 | 196707947 | 196707997 | 196708047 | 196708097 |
| 196773793 | 196773843 | 196773893 | 196773943 | 196773993 |
| 196774022 | 196774072 | 196774122 | 196774172 | 196774222 |
| 196774233 | 196774283 | 196774333 | 196774383 | 196774433 |
| 196774402 | 196774452 | 196774502 | 196774552 | 196774602 |
| 196774723 | 196774773 | 196774823 | 196774873 | 196774923 |
| 196774865 | 196774915 | 196774965 | 196775015 | 196775065 |
| 196775496 | 196775546 | 196775596 | 196775646 | 196775696 |
| 196776500 | 196776550 | 196776600 | 196776650 | 196776700 |
| 196776777 | 196776827 | 196776877 | 196776927 | 196776977 |
| 196839968 | 196840018 | 196840068 | 196840118 | 196840168 |
| 196840715 | 196840765 | 196840815 | 196840865 | 196840915 |
| 196841549 | 196841599 | 196841649 | 196841699 | 196841749 |
| 196841890 | 196841940 | 196841990 | 196842040 | 196842090 |
| 196842902 | 196842952 | 196843002 | 196843052 | 196843102 |
| 196843235 | 196843285 | 196843335 | 196843385 | 196843435 |
| 196843984 | 196844034 | 196844084 | 196844134 | 196844184 |
| 196851351 | 196851401 | 196851451 | 196851501 | 196851551 |
| 196853434 | 196853484 | 196853534 | 196853584 | 196853634 |
| 196853662 | 196853712 | 196853762 | 196853812 | 196853862 |
| 196880273 | 196880323 | 196880373 | 196880423 | 196880473 |
| 196985756 | 196985806 | 196985856 | 196985906 | 196985956 |
| 196985783 | 196985833 | 196985883 | 196985933 | 196985983 |
| 196987184 | 196987234 | 196987284 | 196987334 | 196987384 |
| 196988925 | 196988975 | 196989025 | 196989075 | 196989125 |
| 196989152 | 196989202 | 196989252 | 196989302 | 196989352 |
| 196989277 | 196989327 | 196989377 | 196989427 | 196989477 |
| 196989308 | 196989358 | 196989408 | 196989458 | 196989508 |
| 197453546 | 197453596 | 197453646 | 197453696 | 197453746 |
| 197453608 | 197453658 | 197453708 | 197453758 | 197453808 |
| 197454348 | 197454398 | 197454448 | 197454498 | 197454548 |
| 197454530 | 197454580 | 197454630 | 197454680 | 197454730 |
| 197454557 | 197454607 | 197454657 | 197454707 | 197454757 |
| 197454644 | 197454694 | 197454744 | 197454794 | 197454844 |
| 197454680 | 197454730 | 197454780 | 197454830 | 197454880 |
| 197454733 | 197454783 | 197454833 | 197454883 | 197454933 |
| 197454907 | 197454957 | 197455007 | 197455057 | 197455107 |
| 197454934 | 197454984 | 197455034 | 197455084 | 197455134 |
| 197456822 | 197456872 | 197456922 | 197456972 | 197457022 |
| 197457034 | 197457084 | 197457134 | 197457184 | 197457234 |
| 197457114 | 197457164 | 197457214 | 197457264 | 197457314 |
| 197457251 | 197457301 | 197457351 | 197457401 | 197457451 |
| 197457503 | 197457553 | 197457603 | 197457653 | 197457703 |
| 197459088 | 197459138 | 197459188 | 197459238 | 197459288 |
| 197487865 | 197487915 | 197487965 | 197488015 | 197488065 |
| 197488651 | 197488701 | 197488751 | 197488801 | 197488851 |
| 197488967 | 197489017 | 197489067 | 197489117 | 197489167 |
| 197524389 | 197524439 | 197524489 | 197524539 | 197524589 |
| 197524755 | 197524805 | 197524855 | 197524905 | 197524955 |
| 197525093 | 197525143 | 197525193 | 197525243 | 197525293 |
| 197525265 | 197525315 | 197525365 | 197525415 | 197525465 |
| 197525525 | 197525575 | 197525625 | 197525675 | 197525725 |
| 197525890 | 197525940 | 197525990 | 197526040 | 197526090 |
| 197526521 | 197526571 | 197526621 | 197526671 | 197526721 |
| 197526590 | 197526640 | 197526690 | 197526740 | 197526790 |
| 197527482 | 197527532 | 197527582 | 197527632 | 197527682 |
| 197527582 | 197527632 | 197527682 | 197527732 | 197527782 |
| 197528552 | 197528602 | 197528652 | 197528702 | 197528752 |
| 197556127 | 197556177 | 197556227 | 197556277 | 197556327 |
| 197609586 | 197609636 | 197609686 | 197609736 | 197609786 |
| 197609630 | 197609680 | 197609730 | 197609780 | 197609830 |
| 197611592 | 197611642 | 197611692 | 197611742 | 197611792 |
| 197611732 | 197611782 | 197611832 | 197611882 | 197611932 |
| 197611794 | 197611844 | 197611894 | 197611944 | 197611994 |
| 197613035 | 197613085 | 197613135 | 197613185 | 197613235 |
| 197613558 | 197613608 | 197613658 | 197613708 | 197613758 |
| 197614989 | 197615039 | 197615089 | 197615139 | 197615189 |
| 197615361 | 197615411 | 197615461 | 197615511 | 197615561 |
| 197631460 | 197631510 | 197631560 | 197631610 | 197631660 |
| 197631588 | 197631638 | 197631688 | 197631738 | 197631788 |
| 197632490 | 197632540 | 197632590 | 197632640 | 197632690 |
| 197632606 | 197632656 | 197632706 | 197632756 | 197632806 |
| 197633270 | 197633320 | 197633370 | 197633420 | 197633470 |
| 197633760 | 197633810 | 197633860 | 197633910 | 197633960 |
| 197638678 | 197638728 | 197638778 | 197638828 | 197638878 |
| 197638849 | 197638899 | 197638949 | 197638999 | 197639049 |
| 197639280 | 197639330 | 197639380 | 197639430 | 197639480 |
| 197651923 | 197651973 | 197652023 | 197652073 | 197652123 |
| 197652378 | 197652428 | 197652478 | 197652528 | 197652578 |
| 197653025 | 197653075 | 197653125 | 197653175 | 197653225 |
| 197654442 | 197654492 | 197654542 | 197654592 | 197654642 |
| 197687170 | 197687220 | 197687270 | 197687320 | 197687370 |
| 197687424 | 197687474 | 197687524 | 197687574 | 197687624 |
| 197688112 | 197688162 | 197688212 | 197688262 | 197688312 |
| 197688345 | 197688395 | 197688445 | 197688495 | 197688545 |
| 197688392 | 197688442 | 197688492 | 197688542 | 197688592 |
| 197692891 | 197692941 | 197692991 | 197693041 | 197693091 |
| 197692896 | 197692946 | 197692996 | 197693046 | 197693096 |
| 197694165 | 197694215 | 197694265 | 197694315 | 197694365 |
| 197695268 | 197695318 | 197695368 | 197695418 | 197695468 |
| 197695491 | 197695541 | 197695591 | 197695641 | 197695691 |
| 197695757 | 197695807 | 197695857 | 197695907 | 197695957 |
| 197696092 | 197696142 | 197696192 | 197696242 | 197696292 |
| 197696632 | 197696682 | 197696732 | 197696782 | 197696832 |
| 197696662 | 197696712 | 197696762 | 197696812 | 197696862 |
| 197697227 | 197697277 | 197697327 | 197697377 | 197697427 |
| 197697414 | 197697464 | 197697514 | 197697564 | 197697614 |
| 197698149 | 197698199 | 197698249 | 197698299 | 197698349 |
| 197698178 | 197698228 | 197698278 | 197698328 | 197698378 |
| 197708037 | 197708087 | 197708137 | 197708187 | 197708237 |
| 197708234 | 197708284 | 197708334 | 197708384 | 197708434 |
| 197757973 | 197758023 | 197758073 | 197758123 | 197758173 |
| 197760075 | 197760125 | 197760175 | 197760225 | 197760275 |
| 197761154 | 197761204 | 197761254 | 197761304 | 197761354 |
| 197761205 | 197761255 | 197761305 | 197761355 | 197761405 |
| 197776440 | 197776490 | 197776540 | 197776590 | 197776640 |
| 197777449 | 197777499 | 197777549 | 197777599 | 197777649 |
| 197777518 | 197777568 | 197777618 | 197777668 | 197777718 |
| 197778010 | 197778060 | 197778110 | 197778160 | 197778210 |
| 197781749 | 197781799 | 197781849 | 197781899 | 197781949 |
| 197781861 | 197781911 | 197781961 | 197782011 | 197782061 |
| 197784596 | 197784646 | 197784696 | 197784746 | 197784796 |
| 197785066 | 197785116 | 197785166 | 197785216 | 197785266 |
| 197785170 | 197785220 | 197785270 | 197785320 | 197785370 |
| 197786048 | 197786098 | 197786148 | 197786198 | 197786248 |
| 197786055 | 197786105 | 197786155 | 197786205 | 197786255 |
| 197787668 | 197787718 | 197787768 | 197787818 | 197787868 |
| 197805956 | 197806006 | 197806056 | 197806106 | 197806156 |
| 197806383 | 197806433 | 197806483 | 197806533 | 197806583 |
| 197812495 | 197812545 | 197812595 | 197812645 | 197812695 |
| 197813489 | 197813539 | 197813589 | 197813639 | 197813689 |
| 197813982 | 197814032 | 197814082 | 197814132 | 197814182 |
| 197840702 | 197840752 | 197840802 | 197840852 | 197840902 |
| 197840851 | 197840901 | 197840951 | 197841001 | 197841051 |
| 197855889 | 197855939 | 197855989 | 197856039 | 197856089 |
| 197859223 | 197859273 | 197859323 | 197859373 | 197859423 |
| 197860611 | 197860661 | 197860711 | 197860761 | 197860811 |
| 197861273 | 197861323 | 197861373 | 197861423 | 197861473 |
| 197895172 | 197895222 | 197895272 | 197895322 | 197895372 |
| 197902723 | 197902773 | 197902823 | 197902873 | 197902923 |
| 197902755 | 197902805 | 197902855 | 197902905 | 197902955 |
| 197902823 | 197902873 | 197902923 | 197902973 | 197903023 |
| 197903019 | 197903069 | 197903119 | 197903169 | 197903219 |
| 197903164 | 197903214 | 197903264 | 197903314 | 197903364 |
| 197903202 | 197903252 | 197903302 | 197903352 | 197903402 |
| 197903272 | 197903322 | 197903372 | 197903422 | 197903472 |
| 197903375 | 197903425 | 197903475 | 197903525 | 197903575 |
| 197903487 | 197903537 | 197903587 | 197903637 | 197903687 |
| 197903529 | 197903579 | 197903629 | 197903679 | 197903729 |
| 197903616 | 197903666 | 197903716 | 197903766 | 197903816 |
| 197903716 | 197903766 | 197903816 | 197903866 | 197903916 |
| 197903916 | 197903966 | 197904016 | 197904066 | 197904116 |
| 197904555 | 197904605 | 197904655 | 197904705 | 197904755 |
| 197906573 | 197906623 | 197906673 | 197906723 | 197906773 |
| 197907466 | 197907516 | 197907566 | 197907616 | 197907666 |
| 197907517 | 197907567 | 197907617 | 197907667 | 197907717 |
| 197907553 | 197907603 | 197907653 | 197907703 | 197907753 |
| 197907742 | 197907792 | 197907842 | 197907892 | 197907942 |
| 197909724 | 197909774 | 197909824 | 197909874 | 197909924 |
| 197948446 | 197948496 | 197948546 | 197948596 | 197948646 |
| 197948480 | 197948530 | 197948580 | 197948630 | 197948680 |
| 197948590 | 197948640 | 197948690 | 197948740 | 197948790 |
| 197948731 | 197948781 | 197948831 | 197948881 | 197948931 |
| 197948779 | 197948829 | 197948879 | 197948929 | 197948979 |
| 197973532 | 197973582 | 197973632 | 197973682 | 197973732 |
| 197974393 | 197974443 | 197974493 | 197974543 | 197974593 |
| 197994108 | 197994158 | 197994208 | 197994258 | 197994308 |
| 198023332 | 198023382 | 198023432 | 198023482 | 198023532 |
| 198023473 | 198023523 | 198023573 | 198023623 | 198023673 |

or the complement, or reverse complement of said polynucleic acid.
27. An (isolated) polynucleic acid comprising at least 15 contiguous nucleotides comprised in a region corresponding to a region flanked by any of the indicated 5' and 3' positions of the table below and comprising the nucleotide corresponding to the position of the indicated SNP referenced to maize chromosome 3, B73 AGPv4

| 5' (-100 nt) | 5' (-50 nt) | SNP | 3' (+50 nt) | 3' (+100 nt) |
|---|---|---|---|---|
| 197453608 | 197453658 | 197453708 | 197453758 | 197453858 |
| 197454530 | 197454580 | 197454630 | 197454680 | 197454780 |
| 197454733 | 197454783 | 197454833 | 197454883 | 197454983 |
| 197456822 | 197456872 | 197456922 | 197456972 | 197457072 |
| 197488651 | 197488701 | 197488751 | 197488801 | 197488901 |
| 197524389 | 197524439 | 197524489 | 197524539 | 197524639 |
| 197525525 | 197525575 | 197525625 | 197525675 | 197525775 |
| 197525890 | 197525940 | 197525990 | 197526040 | 197526140 |
| 197556127 | 197556177 | 197556227 | 197556277 | 197556377 |
| 197611794 | 197611844 | 197611894 | 197611944 | 197612044 |
| 197613035 | 197613085 | 197613135 | 197613185 | 197613285 |
| 197613558 | 197613608 | 197613658 | 197613708 | 197613808 |
| 197614989 | 197615039 | 197615089 | 197615139 | 197615239 |
| 197615361 | 197615411 | 197615461 | 197615511 | 197615611 |
| 197633760 | 197633810 | 197633860 | 197633910 | 197634010 |
| 197696092 | 197696142 | 197696192 | 197696242 | 197696342 |

or the complement, or reverse complement of said polynucleic acid.
29. An (isolated) polynucleic acid comprising at least 15 contiguous nucleotides comprised in a region corresponding to a region flanked by any of the indicated 5' and 3' positions of the table below and comprising the nucleotide corresponding to the position of the indicated SEQ ID NO

| SEQ ID NO | 5' (-100 nt) | 5' (-50 nt) | polymorphism | 3' (+50 nt) | 3' (+100 nt) |
|---|---|---|---|---|---|
| 1 | -65 | -15 | 35 | 85 | 135 |
| 1 | 304 | 354 | 404 | 454 | 504 |
| 1 | 344 | 394 | 444-452 | 502 | 552 |
| 1 | 363 | 413 | 463 | 513 | 563 |
| 1 | 437 | 487 | 537 | 587 | 637 |
| 1 | 635 | 685 | 735 | 785 | 835 |
| 1 | 648 | 698 | 748-759 | 809 | 859 |
| 1 | 661 | 711 | 761 | 811 | 861 |
| 1 | 697 | 747 | 797 | 847 | 897 |
| 1 | 948 | 998 | 1048 | 1098 | 1148 |
| 1 | 956 | 1006 | 1056 | 1106 | 1156 |
| 1 | 956 | 1015 | 1065-1066 | 1116 | 1166 |
| 1 | 972 | 1022 | 1072-1073 | 1123 | 1173 |
| 1 | 971 | 1021 | 1071 | 1121 | 1171 |
| 1 | 1088 | 1138 | 1188 | 1238 | 1288 |
| 1 | 1118 | 1168 | 1218 | 1268 | 1318 |
| 1 | 1665 | 1715 | 1765 | 1815 | 1865 |
| 1 | 1669 | 1719 | 1769 | 1819 | 1869 |
| 1 | 1744 | 1794 | 1844 | 1894 | 1944 |
| 1 | 1756 | 1806 | 1856 | 1906 | 1956 |
| 1 | 1976 | 2026 | 2076 | 2126 | 2176 |
| 1 | 1989 | 2039 | 2089 | 2139 | 2189 |
| 1 | 2046 | 2096 | 2146 | 2196 | 2246 |
| 1 | 2068 | 2118 | 2168-2169 | 2219 | 2269 |
| 1 | 2114 | 2164 | 2214 | 2264 | 2314 |
| 1 | 2270 | 2320 | 2370 | 2420 | 2470 |
| 1 | 2482 | 2532 | 2582 | 2632 | 2682 |
| 1 | 2532 | 2582 | 2632-2637 | 2687 | 2737 |
| 1 | 2541 | 2591 | 2641 | 2691 | 2741 |
| 1 | 2543 | 2593 | 2643-2644 | 2694 | 2744 |
| 1 | 2596 | 2646 | 2696 | 2746 | 2796 |
| 1 | 2638 | 2688 | 2738 | 2788 | 2838 |
| 1 | 2743 | 2793 | 2843 | 2893 | 2943 |
| 1 | 2749 | 2799 | 2849 | 2899 | 2949 |
| 1 | 2854 | 2904 | 2954-2955 | 3005 | 3055 |
| 1 | 2904 | 2954 | 3004 | 3054 | 3104 |
| 1 | 2947 | 2997 | 3047 | 3097 | 3147 |
| 1 | 2968 | 3018 | 3068 | 3118 | 3168 |
| 1 | 3118 | 3168 | 3218 | 3268 | 3318 |
| 5 | 386 | 436 | 486 | 536 | 586 |
| 5 | 511 | 561 | 611 | 661 | 711 |
| 5 | 538 | 588 | 638 | 688 | 738 |
| 5 | 589 | 639 | 689 | 739 | 789 |
| 5 | 712 | 762 | 812 | 862 | 912 |
| 5 | 765 | 815 | 865 | 915 | 965 |
| 5 | 801 | 851 | 901 | 951 | 1001 |
| 5 | 888 | 938 | 988 | 1038 | 1088 |
| 5 | 915 | 965 | 1015 | 1065 | 1115 |
| 5 | 985 | 1035 | 1085 | 1135 | 1185 |
| 5 | 1097 | 1147 | 1197 | 1247 | 1297 |
| 5 | 1245 | 1295 | 1345 | 1395 | 1445 |
| 5 | 1361 | 1411 | 1461 | 1511 | 1561 |
| 5 | 1837 | 1887 | 1937 | 1987 | 2037 |
| 5 | 1899 | 1949 | 1999 | 2049 | 2099 |
| 5 | 2013 | 2063 | 2113-2115 | 2165 | 2215 |
| 5 | 2186 | 2236 | 2286 | 2336 | 2386 |
| 5 | 2193 | 2243 | 2293-2297 | 2347 | 2397 |
| 5 | 2299 | 2349 | 2399 | 2449 | 2499 |
| 5 | 2348 | 2398 | 2448-2450 | 2500 | 2550 |
| 5 | 2722 | 2772 | 2822-2823 | 2873 | 2923 |
| 5 | 2756 | 2806 | 2856 | 2906 | 2956 |
| 5 | 2826 | 2876 | 2926 | 2976 | 3026 |
| 5 | 2898 | 2948 | 2998 | 3048 | 3098 |
| 5 | 2929 | 2979 | 3029 | 3079 | 3129 |
| 5 | 2985 | 3035 | 3085 | 3135 | 3185 |
| 5 | 3002 | 3052 | 3102 | 3152 | 3202 |
| 5 | 3012 | 3062 | 3112 | 3162 | 3212 |
| 5 | 3020 | 3070 | 3120 | 3170 | 3220 |
| 5 | 3068 | 3118 | 3168 | 3218 | 3268 |
| 9 | 292 | 342 | 392-393 | 443 | 493 |
| 9 | 450 | 500 | 550 | 600 | 650 |
| 9 | 491 | 541 | 591 | 641 | 691 |
| 9 | 786 | 836 | 886-887 | 937 | 987 |
| 9 | 834 | 884 | 934 | 984 | 1034 |
| 9 | 857 | 907 | 957 | 1007 | 1057 |
| 9 | 997 | 1047 | 1097 | 1147 | 1197 |
| 9 | 1030 | 1080 | 1130 | 1180 | 1230 |
| 9 | 1195 | 1245 | 1295 | 1345 | 1395 |
| 9 | 1362 | 1412 | 1462 | 1512 | 1562 |
| 9 | 1367 | 1417 | 1467 | 1517 | 1567 |
| 9 | 1441 | 1491 | 1541 | 1591 | 1641 |
| 9 | 1484 | 1534 | 1584 | 1634 | 1684 |
| 9 | 1514 | 1564 | 1614 | 1664 | 1714 |
| 9 | 1613 | 1663 | 1713 | 1763 | 1813 |
| 9 | 1674 | 1724 | 1774 | 1824 | 1874 |
| 9 | 1695 | 1745 | 1795-1796 | 1846 | 1896 |
| 9 | 1715 | 1765 | 1815 | 1865 | 1915 |
| 9 | 1794 | 1844 | 1894-1895 | 1945 | 1995 |
| 9 | 1810 | 1860 | 1910 | 1960 | 2010 |
| 9 | 1854 | 1904 | 1954-1955 | 2005 | 2055 |
| 9 | 1900 | 1950 | 2000 | 2050 | 2100 |
| 9 | 1960 | 2010 | 2060 | 2110 | 2160 |
| 9 | 2081 | 2131 | 2181 | 2231 | 2281 |
| 9 | 2254 | 2304 | 2354 | 2404 | 2454 |
| 9 | 2272 | 2322 | 2372 | 2422 | 2472 |
| 9 | 2294 | 2344 | 2394-2399 | 2449 | 2499 |
| 9 | 2328 | 2378 | 2428 | 2478 | 2528 |
| 9 | 2339 | 2389 | 2439-2440 | 2490 | 2540 |
| 13 | 551 | 601 | 651-652 | 702 | 752 |
| 13 | 707 | 757 | 807 | 857 | 907 |

or the complement, or reverse complement of said polynucleic acid.
30. An (isolated) polynucleic acid comprising at least 15 contiguous nucleotides comprised in a region corresponding to a region flanked by any of the indicated 5' and 3' positions of the table below and comprising the nucleotide corresponding to the position of the indicated SEQ ID NO

| SEQ ID NO | 5' (-100 nt) | 5' (-50 nt) | polymorphism | 3' (+50 nt) | 3' (+100 nt) |
|---|---|---|---|---|---|
| 2 | -65 | -15 | 35 | 85 | 135 |
| 2 | 304 | 354 | 404 | 454 | 504 |
| 2 | 343 | 393 | 443-444 | 494 | 544 |
| 2 | 354 | 404 | 454 | 504 | 554 |
| 2 | 428 | 478 | 528 | 578 | 628 |
| 2 | 626 | 676 | 726 | 776 | 826 |
| 2 | 638 | 688 | 738-739 | 789 | 839 |
| 2 | 640 | 690 | 740 | 790 | 840 |
| 2 | 676 | 726 | 776 | 826 | 876 |
| 2 | 927 | 977 | 1027 | 1077 | 1127 |
| 2 | 935 | 985 | 1035 | 1085 | 1135 |
| 2 | 945 | 995 | 1045 | 1095 | 1145 |
| 2 | 953 | 1003 | 1053-1059 | 1109 | 1159 |
| 2 | 958 | 1008 | 1058 | 1108 | 1158 |
| 2 | 1075 | 1125 | 1175 | 1225 | 1275 |
| 2 | 1108 | 1158 | 1208 | 1258 | 1308 |
| 2 | 1657 | 1707 | 1757 | 1807 | 1857 |
| 2 | 1661 | 1711 | 1761 | 1811 | 1861 |
| 2 | 1736 | 1786 | 1836 | 1886 | 1936 |
| 2 | 1748 | 1798 | 1848 | 1898 | 1948 |
| 2 | 1968 | 2018 | 2068 | 2118 | 2168 |
| 2 | 1981 | 2031 | 2081 | 2131 | 2181 |
| 2 | 2038 | 2088 | 2138 | 2188 | 2238 |
| 2 | 2061 | 2111 | 2161 | 2211 | 2261 |
| 2 | 2107 | 2157 | 2207 | 2257 | 2307 |
| 2 | 2262 | 2312 | 2362-2363 | 2413 | 2463 |
| 2 | 2474 | 2524 | 2574 | 2624 | 2674 |
| 2 | 2524 | 2574 | 2624-2629 | 2679 | 2729 |
| 2 | 2533 | 2583 | 2633 | 2683 | 2733 |
| 2 | 2535 | 2585 | 2635-2636 | 2686 | 2736 |
| 2 | 2588 | 2638 | 2688 | 2738 | 2788 |
| 2 | 2630 | 2680 | 2730 | 2780 | 2830 |
| 2 | 2734 | 2784 | 2834-2835 | 2885 | 2935 |
| 2 | 2740 | 2790 | 2840 | 2890 | 2940 |
| 2 | 2846 | 2896 | 2946-2947 | 2997 | 3047 |
| 2 | 2897 | 2947 | 2997 | 3047 | 3097 |
| 2 | 2940 | 2990 | 3040 | 3090 | 3140 |
| 2 | 2961 | 3011 | 3061 | 3111 | 3161 |
| 2 | 3111 | 3161 | 3211 | 3261 | 3311 |
| 6 | 386 | 436 | 486 | 536 | 586 |
| 6 | 511 | 561 | 611 | 661 | 711 |
| 6 | 538 | 588 | 638 | 688 | 738 |
| 6 | 589 | 639 | 689 | 739 | 789 |
| 6 | 712 | 762 | 812 | 862 | 912 |
| 6 | 765 | 815 | 865 | 915 | 965 |
| 6 | 801 | 851 | 901 | 951 | 1001 |
| 6 | 888 | 938 | 988 | 1038 | 1088 |
| 6 | 915 | 965 | 1015 | 1065 | 1115 |
| 6 | 985 | 1035 | 1085 | 1135 | 1185 |
| 6 | 1097 | 1147 | 1197 | 1247 | 1297 |
| 6 | 1245 | 1295 | 1345 | 1395 | 1445 |
| 6 | 1361 | 1411 | 1461 | 1511 | 1561 |
| 6 | 1837 | 1887 | 1937 | 1987 | 2037 |
| 6 | 1899 | 1949 | 1999 | 2049 | 2099 |
| 6 | 2012 | 2062 | 2112-2113 | 2163 | 2213 |
| 6 | 2183 | 2233 | 2283 | 2333 | 2383 |
| 6 | 2189 | 2239 | 2289-2290 | 2340 | 2390 |
| 6 | 2291 | 2341 | 2391 | 2441 | 2491 |
| 6 | 2339 | 2389 | 2439-2440 | 2490 | 2540 |
| 6 | 2710 | 2760 | 2810-2811 | 2861 | 2911 |
| 6 | 2743 | 2793 | 2843 | 2893 | 2943 |
| 6 | 2813 | 2863 | 2913 | 2963 | 3013 |
| 6 | 2885 | 2935 | 2985 | 3035 | 3085 |
| 6 | 2916 | 2966 | 3016 | 3066 | 3116 |
| 6 | 2972 | 3022 | 3072 | 3122 | 3172 |
| 6 | 2989 | 3039 | 3089 | 3139 | 3189 |
| 6 | 2999 | 3049 | 3099 | 3149 | 3199 |
| 6 | 3007 | 3057 | 3107 | 3157 | 3207 |
| 6 | 3055 | 3105 | 3155 | 3205 | 3255 |
| 10 | 293 | 343 | 393-397 | 447 | 497 |
| 10 | 455 | 505 | 555 | 605 | 655 |
| 10 | 496 | 546 | 596 | 646 | 696 |
| 10 | 791 | 841 | 891-892 | 942 | 992 |
| 10 | 839 | 889 | 939 | 989 | 1039 |
| 10 | 862 | 912 | 962 | 1012 | 1062 |
| 10 | 1002 | 1052 | 1102 | 1152 | 1202 |
| 10 | 1035 | 1085 | 1135 | 1185 | 1235 |
| 10 | 1200 | 1250 | 1300 | 1350 | 1400 |
| 10 | 1367 | 1417 | 1467 | 1517 | 1567 |
| 10 | 1372 | 1422 | 1472 | 1522 | 1572 |
| 10 | 1446 | 1496 | 1546 | 1596 | 1646 |
| 10 | 1503 | 1553 | 1603 | 1653 | 1703 |
| 10 | 1533 | 1583 | 1633 | 1683 | 1733 |
| 10 | 1632 | 1682 | 1732 | 1782 | 1832 |
| 10 | 1693 | 1743 | 1793 | 1843 | 1893 |
| 10 | 1715 | 1765 | 1815-1823 | 1873 | 1923 |
| 10 | 1743 | 1793 | 1843 | 1893 | 1943 |
| 10 | 1823 | 1873 | 1923 | 1973 | 2023 |
| 10 | 1839 | 1889 | 1939 | 1989 | 2039 |
| 10 | 1884 | 1934 | 1984-1991 | 2041 | 2091 |
| 10 | 1937 | 1987 | 2037 | 2087 | 2137 |
| 10 | 1997 | 2047 | 2097 | 2147 | 2197 |
| 10 | 2118 | 2168 | 2218 | 2268 | 2318 |
| 10 | 2291 | 2341 | 2391 | 2441 | 2491 |
| 10 | 2309 | 2359 | 2409 | 2459 | 2509 |
| 10 | 2331 | 2381 | 2431-2434 | 2484 | 2534 |
| 10 | 2363 | 2413 | 2463 | 2513 | 2563 |
| 10 | 2375 | 2425 | 2475-2476 | 2526 | 2576 |
| 14 | 552 | 602 | 652-654 | 704 | 754 |
| 14 | 710 | 760 | 810 | 860 | 910 |

or the complement, or reverse complement of said polynucleic acid.
31. The (isolated) polynucleic acid according to any of statements 25 to 30, comprising at most 500 nucleotides, preferably at most 200 nucleotides, more preferably at most 100 nucleotides, most preferably at most 50 nucleotides, such as at most 35 nucleotides.
32. An (isolated) polynucleic acid specifically hybridizing with the polynucleic acid according to any of statements 25 to 31, or the complement or reverse complement of said polynucleic acid.
33. The (isolated) polynucleic acid according to any of statements 25 to 32, which is a primer or a probe.
34. The (isolated) polynucleotide according to any of statements 25 to 33, which is an allele-specific primer or probe.
35. The (isolated) polynucleic acid according to any of statements 25 to 34 which is a KASP primer.
36. A primer or a probe comprising the (isolated) polynucleic acid according to any of statements 25 to 35.
37. The primer according to statement 36, which is an allele-specific primer.
38. The primer according to statement 36 or 37, which is a KASP primer.
39. A primer specifically hybridizing with a molecular marker (allele) of Table 4 or Table 5, or the complement or reverse complement thereof.
40. A primer capable of specifically detecting a molecular marker (allele) of Table 4 or Table 5.
41. A primer set capable of specifically detecting a molecular marker (allele) of Table 4 or Table 5.
42. A primer set capable of amplifying a polynucleic acid comprising a molecular marker (allele) of Table 4 or Table 5.
43. A (maize) plant or plant part comprising one or more molecular marker (allele) of Table 4 or Table 5, the locus as defined in any of statements 1 to 10, and/or a polynucleic acid as defined in any of statements 5, 6, 9, 10, or 25 to 30.
44. A method for generating a (maize) plant or plant part, comprising introducing in the genome of said plant or plant part a locus as defined in any of statements 1 to 10, or a (functional) fragment thereof.
45. The method according to statement 44, wherein introducing into the genome comprises transgenesis.
4462. The method according to statements 44 or 45, wherein introducing into the genome comprises introgression.
47. The method according to any of statements 44 to 46, comprising transforming a plant or plant part, preferably a plant cell, more preferably a protoplast, with a polynucleic acid encoding a locus as defined in any of statements 1 to 10, and optionally regenerating a plant from said plant cell, preferably protoplast.
48. The method according to statement 47, wherein said polynucleic acid which is introduced has a different sequence than a corresponding polynucleic acid of the plant.
49. A method for generating a (maize) plant or plant part, comprising (a) providing a first (maize) plant identified according to any of statements 1 to 24 or generated according to any of statements 40 to 44, (b) crossing said first (maize) plant with a second (maize) plant having cytoplasmic male sterility; and optionally (d) harvesting said (maize) plant part from the progeny.
50. The method according to any of statements 1 to 24 or 44 to 49, wherein said plant part is a cell, tissue or organ.
51. The method according to any of statements 1 to 24 or 44 to 50, wherein said plant part is a protoplast.
52. The method according to any of statements 1 to 24 or 44 to 50, wherein said plant part is a seed.
53. The method according to any of statements 1 to 24, wherein said locus, polynucleic acid, or molecular marker (allele) is homozygous.
54. The method according to any of statements 1 to 24, wherein said locus, polynucleic acid, or molecular marker (allele) is heterozygous.
55. Use of a polynucleic acid, primer or probe, or primer set according to any of statements 5, 6, 9, 10, or 25 to 42 for identifying a (maize) plant or plant part.
56. Use of a polynucleic acid according to any of statements 5, 6, 9, 10, or 25 to 30, or a locus as defined in any of statements 1 to 10, for generating a (maize) plant or plant part.

### BRIEF DESCRIPTION OF THE FIGURES

**Figure 1**: GWAS analysis using rf4- lines based on Illumina Chip data (35K)
**Figure 2**: Sequence alignment of the genomic sequence of Zm00001d043358 of the reference B73 genome (SEQ ID NO: 2) and Zm00001d043358 of an embodiment of the present invention (SEQ ID NO: 1).
**Figure 3**: Sequence alignment of the genomic sequence of Zm00001d043352of the reference B73 genome (SEQ ID NO: 6) and Zm00001d043352of an embodiment of the present invention (SEQ ID NO: 5).
**Figure 4**: Sequence alignment of the genomic sequence of Zm00001d043356of the reference B73 genome (SEQ ID NO: 10) and Zm00001d043356of an embodiment of the present invention (SEQ ID NO: 9).
**Figure 5**: Sequence alignment of the genomic sequence of Zm00001d043357of the reference B73 genome (SEQ ID NO: 14) and Zm00001d043357of an embodiment of the present invention (SEQ ID NO: 13).

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel et al. 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous.

The term "plant" includes whole plants, including descendants or progeny thereof. As used herein unless clearly indicated otherwise, the term "plant" intends to mean a plant at any developmental stage. The term "plant part" includes any part or derivative of the plant, including particular plant tissues or structures, plant cells, plant protoplast, plant cell or tissue culture from which plants can be regenerated, plant calli, plant clumps and plant cells that are intact in plants or parts of plants, such as seeds, kernels, cobs, flowers, cotyledons, leaves, stems, buds, roots, root tips, stover, and the like. Plant parts may include processed plant parts or derivatives, including flower, oils, extracts etc. "Parts of a plant" are e.g. shoot vegetative organs/structures, e.g., leaves, stems and tubers; roots, flowers and floral organs/structures, e.g. bracts, sepals, petals, stamens, carpels, anthers and ovules; seed, including embryo, endosperm, and seed coat; fruit and the mature ovary; plant tissue, e.g. vascular tissue, ground tissue, and the like; and cells, e.g. guard cells, egg cells, pollen, trichomes and the like; and progeny of the same. Parts of plants may be attached to or separate from a whole intact plant. Such parts of a plant include, but are not limited to, organs, tissues, and cells of a plant, and preferably seeds. A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant. "Plant cell culture" means cultures of plant units such as, for example, protoplasts, cell culture cells, cells in plant tissues, pollen, pollen tubes, ovules, embryo sacs, zygotes and embryos at various stages of development. "Plant material" refers to leaves, stems, roots, flowers or flower parts, fruits, pollen, egg cells, zygotes, seeds, cuttings, cell or tissue cultures, or any other part or product of a plant, including meal. This also includes callus or callus tissue as well as extracts (such as extracts from taproots) or samples. A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

In certain embodiments, the plant part or derivative is or comprises (functional) propagation material, such as germplasm, a seed, or plant embryo or other material from which a plant can be regenerated. In certain embodiments, the plant part or derivative is not (functional) propagation material, such as germplasm, a seed, or plant embryo or other material from which a plant can be regenerated. In certain embodiments, the plant part or derivative does not comprise (functional) male and female reproductive organs. In certain embodiments, the plant part or derivative is or comprises propagation material, but propagation material which does not or cannot be used (anymore) to produce or generate new plants, such as propagation material which have been chemically, mechanically or otherwise rendered non-functional, for instance by heat treatment, acid treatment, compaction, crushing, chopping, etc.

As used herein, the terms "progeny" and "progeny plant" refer to a plant generated from sexual reproduction from one or more parent plants. A progeny plant can be obtained by selfing a single parent plant, or by crossing two parental plants. For instance, a progeny plant can be obtained by selfing of a parent plant or by crossing two parental plants and include selfings as well as the F1 or F2 or still further generations. An F1 is a first-generation progeny produced from parents at least one of which is used for the first time as donor of a trait, while progeny of second generation (F2) or subsequent generations (F3 , F4 , and the like) are specimens produced from selfings, intercrosses, backcrosses, and/or other crosses of F1 s, F2 s, and the like. An F1 can thus be (and in some embodiments is) a hybrid resulting from a cross between two true breeding parents (i.e., parents that are true-breeding are each homozygous for a trait of interest or an allele thereof), while an F2 can be (and in some embodiments is) a progeny resulting from self-pollination of the F1 hybrids. The term "progeny" can in certain embodiments be used interchangeably with "offspring", in particular when the plant or plant material is derived from sexual crossing of parent plants. According to the present invention, progeny preferably refers to the F1 progeny.

As used herein, the terms "crossed" or "cross" or "crossing" means the fusion of gametes via pollination to produce progeny (i.e., cells, seeds, or plants). The term encompasses both sexual crosses (the pollination of one plant by another) and self-fertilization (selfing, self-pollination, i.e., when the pollen and ovule (or microspores and megaspores) are from the same plant or genetically identical plants). Preferably, crossing as referred to herein fertilization of one plant by another plant, i.e. not self-pollination.

As used herein, the term plant population may be used interchangeably with population of plants. A plant population preferably comprises a multitude of individual plants, such as preferably at least 10, such as 20, 30, 40, 50, 60, 70, 80, or 90, more preferably at least 100, such as 200, 300, 400, 500, 600, 700, 800, or 900, even more preferably at least 1000, such as at least 10000 or at least 100000.

As used herein, the terms "phenotype," "phenotypic trait" or "trait" refer to one or more traits of a plant or plant cell. The phenotype can be observable to the naked eye, or by any other means of evaluation known in the art, e.g., microscopy, biochemical analysis, or an electromechanical assay. In some cases, a phenotype is directly controlled by a single gene or genetic locus (i.e., corresponds to a "single gene trait"). In the case of haploid induction use of color markers, such as R Navajo, and other markers including transgenes visualized by the presences or absences of color within the seed evidence if the seed is an induced haploid seed. The use of R Navajo as a color marker and the use of transgenes is well known in the art as means to detect induction of haploid seed on the female plant. In other cases, a phenotype is the result of interactions among several genes, which in some embodiments also results from an interaction of the plant and/or plant cell with its environment.

As used herein, "maize" refers to a plant of the species Zea *mays,* preferably Zea *mays ssp mays.*

As used herein, the term "male sterile" plant (line, cultivar, or variety) has its ordinary meaning in the art. By means of further guidance, and without limitation, the term refers to a plant which is unable to produce offspring as a pollen donor, and may result from the failure to produce (functional) anthers, pollen, or gametes. Cytoplasmic male sterile plants have cytoplasmic genes, usually in the mitochondria, that encode factors that disrupt or prevent pollen development, making them male-sterile, with male sterility inherited maternally. The utilization of cytoplasmic male sterility for hybrid seed production typically requires three separate plant lines: the male-sterile line, an isogeneic male-fertile line for propagation ("maintainer line") and a line for restoring fertility to the hybrid so that it can produce seed ("restorer line"). The male-sterile line is used as the receptive parent in a hybrid cross, the maintainer line is genetically identical to the male-sterile line, excepting that it lacks the cytoplasmic sterility factors, and the restorer line is any line that masks the cytoplasmic sterility factor. The restorer line is very important for those plants, such as grain sorghum or cotton, the useful crop of which is the seed itself or seed-associated structures. Genetic male sterility is similar to cytoplasmic male sterility, but differs in that the sterility factors are encoded in nuclear DNA. Typically, genetic male sterility refers to a change in a plant's genetic structure which results in its ability to produce and/or spread viable pollen. Genetic male sterile plant lines may occur naturally. It is also possible to create a male-sterile plant line using recombinant techniques. Whether naturally occurring or transgenic, male-sterile lines still require the use of a sister maintainer line for their propagation, which of necessity leads to a minimum of 50% male-fertile plants in propagated seed. This is a result of the genetics of male-sterility and maintainer lines. If the male-sterility factor is recessive, as most are, a male-sterile plant would have to be homozygous recessive in order to display the trait. Preferably, according to the invention male sterility refers to genetical male sterility. Preferably, according to the invention male sterility is not or does not encompass cytoplasmic male sterility.

Preferably, according to the invention CMS as referred to herein is CMS-C (or C-type CMS), although other types of CMS are also envisaged, including CMS-T and CMS-S.

As used herein, the term "restorer" or "restorer of fertility" means the gene(s) that restore(s) fertility to a CMS plant. The term "restorer" may also mean the plant or line carrying the restorer gene. By means of extension, the term restorer can be applied to a restorer locus (allele), haplotype, or genotype, meaning a locus (allele), haplotype, or genotype carrying the restores gene or being responsible for the restorer phenotype. According to the invention, the restorer gene, locus (allele), haplotype, genotype, or phenotype is associated/linked with the polymorphisms (alleles), polynucleic acids, or markers of the invention as described herein elsewhere. Accordingly, a restorer locus (allele) or fertility restorer locus (allele) refers to a genomic interval carrying the restorer gene(s), and is characterized by the presence of one or more of the polymorphisms (alleles), polynucleic acids, or molecular markers as described herein. According to the invention, the restorer is not (solely) or does not (solely) comprise Rf4.

The combination of any one or more of the marker(s) (allele(s)) of the invention may be referred to as a marker haplotype of the invention.

As used herein, the term "maintainer" may equally be used for the male fertile as well as the (isogenic) male sterile lines, and hence refers to a plant (or line) which does not have the restorer phenotype and/or comprise the restorer genotype, haplotype, or locus (allele) (all either heterozygous or homozygous), as opposed to the term "restorer", which does have the restorer phenotype and/or comprises the restorer genotype, haplotype, or (allele) (all either heterozygous or homozygous), preferably the restorer phenotype, genotype, haplotype or locus (allele) of the present invention. Accordingly, the term "maintainer" may be used equally for the maintainer line sensu strictu, i.e. the isogenic fertile counterpart of the CMS line for use in "maintaining" the CMS line, as well as for the CMS line itself. In certain embodiments, the maintainer does not have the restorer phenotype and/or comprise the restorer genotype, haplotype, or locus (allele) of the invention, such as any one or more molecular markers (alleles) of the invention, in particular the molecular markers (alleles) associated /linked with the restorer phenotype, genotype, haplotype, or locus (allele) of the invention, which may be homozygous or heterozygous. In certain embodiments, the maintainer has a different restorer phenotype and/or comprise the restorer genotype, haplotype, or locus (allele) than the restorer phenotype and/or comprise the restorer genotype, haplotype, or locus (allele) of the invention, e.g. Rf4, which may be homozygous or heterozygous.

The term "locus" (loci plural) means a specific place or places or a site on a chromosome where for example a QTL/haplotype, a gene or genetic marker is found. As used herein, the term "quantitative trait locus" or "QTL" has its ordinary meaning known in the art. By means of further guidance, and without limitation, a QTL may refer to a region of DNA that is associated with the differential expression of a quantitative phenotypic trait in at least one genetic background, e.g., in at least one breeding population. The region of the QTL encompasses or is closely linked to the gene or genes that affect the trait in question.

As used herein, the term "allele" or "alleles" refers to one or more alternative forms, i.e. different nucleotide sequences, of a locus.

An "allele of a locus" can comprise multiple genes or other genetic factors within a contiguous genomic region or linkage group, such as a haplotype. An allele of a locus can denote a haplotype within a specified window wherein said window is a contiguous genomic region that can be defined, and tracked, with a set of one or more polymorphic markers. A haplotype can be defined by the unique fingerprint of alleles at each marker within the specified window. A locus may encode for one or more alleles that affect the expressivity of a continuously distributed (quantitative) phenotype. In certain embodiments, the locus, allele, polynucleic acid, or molecular marker (allele) as described herein may be homozygous. In certain embodiments, the locus, allele, polynucleic acid, or molecular marker (allele) as described herein may be heterozygous.

As used herein, the term "mutant alleles" or "mutation" of alleles include alleles having one or more mutations, such as insertions, deletions, stop codons, base changes (e.g. , transitions or transversions), or alterations in splice junctions, which may or may not give rise to altered gene products. Modifications in alleles may arise in coding or non-coding regions (e.g. promoter regions, exons, introns or splice junctions).

A "marker" is a (means of finding a position on a) genetic or physical map, or else linkages among markers and trait loci (loci affecting traits). The position that the marker detects may be known via detection of polymorphic alleles and their genetic mapping, or else by hybridization, sequence match or amplification of a sequence that has been physically mapped. A marker can be a DNA marker (detects DNA polymorphisms), a protein (detects variation at an encoded polypeptide), or a simply inherited phenotype (such as the 'waxy' phenotype). A DNA marker can be developed from genomic nucleotide sequence or from expressed nucleotide sequences (e.g., from a spliced RNA or a cDNA). Depending on the DNA marker technology, the marker may consist of complementary primers flanking the locus and/or complementary probes that hybridize to polymorphic alleles at the locus. The term marker locus is the locus (gene, sequence or nucleotide) that the marker detects. "Marker" or "molecular marker" or "marker locus" may also be used to denote a nucleic acid or amino acid sequence that is sufficiently unique to characterize a specific locus on the genome. Any detectable polymorphic trait can be used as a marker so long as it is inherited differentially and exhibits linkage disequilibrium with a phenotypic trait of interest.

Markers that detect genetic polymorphisms between members of a population are well-established in the art. Markers can be defined by the type of polymorphism that they detect and also the marker technology used to detect the polymorphism. Marker types include but are not limited to, e.g., detection of restriction fragment length polymorphisms (RFLP), detection of isozyme markers, randomly amplified polymorphic DNA (RAPD), amplified fragment length polymorphisms (AFLPs), detection of simple sequence repeats (SSRs), detection of amplified variable sequences of the plant genome, detection of self-sustained sequence replication, or detection of single nucleotide polymorphisms (SNPs). SNPs can be detected e.g. via DNA sequencing, PCR-based sequence specific amplification methods, detection of polynucleotide polymorphisms by allele specific hybridization (ASH), dynamic allele-specific hybridization (DASH), molecular beacons, microarray hybridization, oligonucleotide ligase assays, Flap endonucleases, 5' endonucleases, primer extension, single strand conformation polymorphism (SSCP) or temperature gradient gel electrophoresis (TGGE). DNA sequencing, such as the pyrosequencing technology has the advantage of being able to detect a series of linked SNP alleles that constitute a haplotype. Haplotypes tend to be more informative (detect a higher level of polymorphism) than SNPs.

A "marker allele", alternatively an "allele of a marker locus", can refer to one of a plurality of polymorphic nucleotide sequences found at a marker locus in a population. With regard to a SNP marker, allele refers to the specific nucleotide base present at that SNP locus in that individual plant.

"Fine-mapping" refers to methods by which the position of a genomic region (e.g. QTL) can be determined more accurately (narrowed down) and by which the size of the introgression fragment comprising the QTL is reduced. For example Near Isogenic Lines for the QTL or haplotype (QTL/haplotype-NILs) can be made, which contain different, overlapping fragments of the introgression fragment within an otherwise uniform genetic background of the recurrent parent. Such lines can then be used to map on which fragment the QTL/haplotype is located and to identify a line having a shorter introgression fragment comprising the QTL/haplotype.

"Marker assisted selection" (of MAS) is a process by which individual plants are selected based on marker genotypes. "Marker assisted counter-selection" is a process by which marker genotypes are used to identify plants that will not be selected, allowing them to be removed from a breeding program or planting. Marker assisted selection uses the presence of molecular markers, which are genetically linked to a particular locus or to a particular chromosome region (e.g. introgression fragment, transgene, polymorphism, mutation, etc), to select plants for the presence of the specific locus or region (introgression fragment, transgene, polymorphism, mutation, etc). For example, a molecular marker genetically linked to a genomic region (e.g. haplotype) or gene (e.g. the RLK1 allele conferring pathogen resistance) as defined herein, can be used to detect and/or select plants comprising the HT2/HT3 on chromosome 8. The closer the genetic linkage of the molecular marker to the locus (e.g. about 7 cM, 6 cM, 5 cM, 4 cM, 3 cM, 2 cM, 1 cM, 0.5 cM or less), the less likely it is that the marker is dissociated from the locus through meiotic recombination. Likewise, the closer two markers are linked to each other (e.g. within 7 or 5 cM, 4 cM , 3 cM, 2 cM, 1 cM or less) the less likely it is that the two markers will be separated from one another (and the more likely they will co-segregate as a unit). A marker "within 7 cM or within 5 cM, 3 cM, 2 cM, or 1 cM" of another marker refers to a marker which genetically maps to within the 7 cM or 5 cM, 3 cM, 2 cM, or 1 cM region flanking the marker (i.e. either side of the marker). Similarly, a marker within 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10kb, 5kb, 2kb, 1 kb or less of another marker refers to a marker which is physically located within the 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10 kb, 5 kb, 2 kb, 1 kb or less, of the genomic DNA region flanking the marker (i.e. either side of the marker). "LOD-score" (logarithm (base 10) of odds) refers to a statistical test often used for linkage analysis in animal and plant populations. The LOD score compares the likelihood of obtaining the test data if the two loci (molecular marker loci and/or a phenotypic trait locus) are indeed linked, to the likelihood of observing the same data purely by chance. Positive LOD scores favour the presence of linkage and a LOD score greater than 3.0 is considered evidence for linkage. A LOD score of +3 indicates 1000 to 1 odds that the linkage being observed did not occur by chance.

A "marker haplotype" refers to a combination of (marker) alleles at a (marker) locus.

A "marker locus" is a specific chromosome location in the genome of a species where a specific marker can be found. A marker locus can be used to track the presence of a second linked locus, e.g., one that affects the expression of a phenotypic trait. For example, a marker locus can be used to monitor segregation of alleles at a genetically or physically linked locus.

A "marker probe" is a nucleic acid sequence or molecule that can be used to identify the presence of a marker locus, e.g., a nucleic acid probe that is complementary to a marker locus sequence, through nucleic acid hybridization. Marker probes comprising 30 or more contiguous nucleotides of the marker locus ("all or a portion" of the marker locus sequence) may be used for nucleic acid hybridization. Alternatively, in some aspects, a marker probe refers to a probe of any type that is able to distinguish (i.e., genotype) the particular allele that is present at a marker locus.

The term "molecular marker" may be used to refer to a genetic marker or an encoded product thereof (e.g., a protein) used as a point of reference when identifying a linked locus. A marker can be derived from genomic nucleotide sequences or from expressed nucleotide sequences (e.g., from a spliced RNA, a cDNA, etc.), or from an encoded polypeptide. The term also refers to nucleic acid sequences complementary to or flanking the marker sequences, such as nucleic acids used as probes or primer pairs capable of amplifying the marker sequence. A "molecular marker probe" is a nucleic acid sequence or molecule that can be used to identify the presence of a marker locus, e.g., a nucleic acid probe that is complementary to a marker locus sequence. Alternatively, in some aspects, a marker probe refers to a probe of any type that is able to distinguish (i.e., genotype) the particular allele that is present at a marker locus. Nucleic acids are "complementary" when they specifically hybridize in solution, e.g., according to Watson-Crick base pairing rules. Some of the markers described herein are also referred to as hybridization markers when located on an indel region, such as the non- collinear region described herein. This is because the insertion region is, by definition, a polymorphism vis a vis a plant without the insertion. Thus, the marker need only indicate whether the indel region is present or absent. Any suitable marker detection technology may be used to identify such a hybridization marker, e.g. SNP technology is used in the examples provided herein.

"Genetic markers" are nucleic acids that are polymorphic in a population and where the alleles of which can be detected and distinguished by one or more analytic methods, e.g., RFLP, AFLP, isozyme, SNP, SSR, and the like. The terms "molecular marker" and "genetic marker" are used interchangeably herein. The term also refers to nucleic acid sequences complementary to the genomic sequences, such as nucleic acids used as probes. Markers corresponding to genetic polymorphisms between members of a population can be detected by methods well- established in the art. These include, e.g., PCR-based sequence specific amplification methods, detection of restriction fragment length polymorphisms (RFLP), detection of isozyme markers, detection of polynucleotide polymorphisms by allele specific hybridization (ASH), detection of amplified variable sequences of the plant genome, detection of self-sustained sequence replication, detection of simple sequence repeats (SSRs), detection of single nucleotide polymorphisms (SNPs), or detection of amplified fragment length polymorphisms (AFLPs). Well established methods are also know for the detection of expressed sequence tags (ESTs) and SSR markers derived from EST sequences and randomly amplified polymorphic DNA (RAPD).

As referred to herein, a polynucleic acid of the invention as described herein, is said to be flanked by certain molecular markers or molecular marker alleles if the polynucleic acid is comprised within a polynucleic acid wherein respectively a first marker (allele) is located upstream (i.e. 5') of said polynucleic acid and a second marker (allele) is located downstream (i.e. 3') of said polynucleic acid. Such first and second marker (allele) may border the polynucleic acid. The nucleic acid may equally comprise such first and second marker (allele), such as respectively at or near the 5' and 3' end, for instance respectively within 50 kb of the 5' and 3' end, preferably within 10 kb of the 5' and 3' end, such as within 5 kb of the 5' and 3' end, within 1 kb of the 5' and 3' end, or less.

A "polymorphism" is a variation in the DNA between two or more individuals within a population. A polymorphism preferably has a frequency of at least 1 % in a population. A useful polymorphism can include a single nucleotide polymorphism (SNP), a simple sequence repeat (SSR), or an insertion/deletion polymorphism, also referred to herein as an "indel". The term "indel" refers to an insertion or deletion, wherein one line may be referred to as having an inserted nucleotide or piece of DNA relative to a second line, or the second line may be referred to as having a deleted nucleotide or piece of DNA relative to the first line.

"Physical distance" between loci (e.g. between molecular markers and/or between phenotypic markers) on the same chromosome is the actually physical distance expressed in bases or base pairs (bp), kilo bases or kilo base pairs (kb) or megabases or mega base pairs (Mb).

"Genetic distance" between loci (e.g. between molecular markers and/or between phenotypic markers) on the same chromosome is measured by frequency of crossing-over, or recombination frequency (RF) and is indicated in centimorgans (cM). One cM corresponds to a recombination frequency of 1%. If no recombinants can be found, the RF is zero and the loci are either extremely close together physically or they are identical. The further apart two loci are, the higher the RF.

A "physical map" of the genome is a map showing the linear order of identifiable landmarks (including genes, markers, etc.) on chromosome DNA. However, in contrast to genetic maps, the distances between landmarks are absolute (for example, measured in base pairs or isolated and overlapping contiguous genetic fragments) and not based on genetic recombination (that can vary in different populations).

An allele "negatively" correlates with a trait when it is linked to it and when presence of the allele is an indicator that a desired trait or trait form will not occur in a plant comprising the allele. An allele "positively" correlates with a trait when it is linked to it and when presence of the allele is an indicator that the desired trait or trait form will occur in a plant comprising the allele.

A centimorgan ("cM") is a unit of measure of recombination frequency. One cM is equal to a 1 % chance that a marker at one genetic locus will be separated from a marker at a second locus due to crossing over in a single generation.

As used herein, the term "chromosomal interval" designates a contiguous linear span of genomic DNA that resides in planta on a single chromosome. The genetic elements or genes located on a single chromosomal interval are physically linked. The size of a chromosomal interval is not particularly limited. In some aspects, the genetic elements located within a single chromosomal interval are genetically linked, typically with a genetic recombination distance of, for example, less than or equal to 20 cM, or alternatively, less than or equal to 10 cM. That is, two genetic elements within a single chromosomal interval undergo recombination at a frequency of less than or equal to 20% or 10%.

The term "closely linked", in the present application, means that recombination between two linked loci occurs with a frequency of equal to or less than about 10% (i.e., are separated on a genetic map by not more than 10 cM). Put another way, the closely linked loci co-segregate at least 90% of the time. Marker loci are especially useful with respect to the subject matter of the current disclosure when they demonstrate a significant probability of co-segregation (linkage) with a desired trait (e.g., resistance to gray leaf spot). Closely linked loci such as a marker locus and a second locus can display an inter-locus recombination frequency of 10% or less, preferably about 9% or less, still more preferably about 8% or less, yet more preferably about 7% or less, still more preferably about 6% or less, yet more preferably about 5% or less, still more preferably about 4% or less, yet more preferably about 3% or less, and still more preferably about 2% or less. In highly preferred embodiments, the relevant loci display a recombination a frequency of about 1 % or less, e.g., about 0.75% or less, more preferably about 0.5% or less, or yet more preferably about 0.25% or less. Two loci that are localized to the same chromosome, and at such a distance that recombination between the two loci occurs at a frequency of less than 10% (e.g., about 9 %, 8%, 7%, 6%, 5%, 4%, 3%, 2%, 1 %, 0.75%, 0.5%, 0.25%, or less) are also said to be "proximal to" each other. In some cases, two different markers can have the same genetic map coordinates. In that case, the two markers are in such close proximity to each other that recombination occurs between them with such low frequency that it is undetectable.

"Linkage" refers to the tendency for alleles to segregate together more often than expected by chance if their transmission was independent. Typically, linkage refers to alleles on the same chromosome. Genetic recombination occurs with an assumed random frequency over the entire genome. Genetic maps are constructed by measuring the frequency of recombination between pairs of traits or markers. The closer the traits or markers are to each other on the chromosome, the lower the frequency of recombination, and the greater the degree of linkage. Traits or markers are considered herein to be linked if they generally co- segregate. A 1/100 probability of recombination per generation is defined as a genetic map distance of 1.0 centiMorgan (1.0 cM). The term "linkage disequilibrium" refers to a non-random segregation of genetic loci or traits (or both). In either case, linkage disequilibrium implies that the relevant loci are within sufficient physical proximity along a length of a chromosome so that they segregate together with greater than random (i.e., non-random) frequency. Markers that show linkage disequilibrium are considered linked. Linked loci co-segregate more than 50% of the time, e.g., from about 51 % to about 100% of the time. In other words, two markers that co-segregate have a recombination frequency of less than 50% (and by definition, are separated by less than 50 cM on the same linkage group.) As used herein, linkage can be between two markers, or alternatively between a marker and a locus affecting a phenotype. A marker locus can be "associated with" (linked to) a trait. The degree of linkage of a marker locus and a locus affecting a phenotypic trait is measured, e.g., as a statistical probability of co-segregation of that molecular marker with the phenotype (e.g., an F statistic or LOD score).

The genetic elements or genes located on a single chromosome segment are physically linked. In some embodiments, the two loci are located in close proximity such that recombination between homologous chromosome pairs does not occur between the two loci during meiosis with high frequency, e.g., such that linked loci co-segregate at least about 90% of the time, e.g., 91%, 92%, 93%, 94%, 95%, 96%, 97%, 98%, 99%, 99.5%, 99.75%, or more of the time. The genetic elements located within a chromosomal segment are also "genetically linked", typically within a genetic recombination distance of less than or equal to 50cM, e.g., about 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0.75, 0.5, 0.25 cM or less. That is, two genetic elements within a single chromosomal segment undergo recombination during meiosis with each other at a frequency of less than or equal to about 50%, e.g., about 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.75%, 0.5%, 0.25% or less. "Closely linked" markers display a cross over frequency with a given marker of about 10% or less, e.g., 9%, 8%, 7%, 6%, 5%, 4%, 3%, 2%, 1%, 0.75%, 0.5%, 0.25% or less (the given marker locus is within about 10 cM of a closely linked marker locus, e.g., 9, 8, 7, 6, 5, 4, 3, 2, 1, 0.75, 0.5, 0.25 cM or less of a closely linked marker locus). Put another way, closely linked marker loci co-segregate at least about 90% the time, e.g., 91%, 92%, 93%, 94%, 95%, 96%, 97%, 98%, 99%, 99.5%, 99.75%, or more of the time.

As used herein, the terms "introgression", "introgressed" and "introgressing" refer to both a natural and artificial process whereby chromosomal fragments or genes of one species, variety or cultivar are moved into the genome of another species, variety or cultivar, by crossing those species. The process may optionally be completed by backcrossing to the recurrent parent. For example, introgression of a desired allele at a specified locus can be transmitted to at least one progeny via a sexual cross between two parents of the same species, where at least one of the parents has the desired allele in its genome. Alternatively, for example, transmission of an allele can occur by recombination between two donor genomes, e.g., in a fused protoplast, where at least one of the donor protoplasts has the desired allele in its genome. The desired allele can be, e.g., detected by a marker that is associated with a phenotype, a haplotype, a QTL, a transgene, or the like. In any case, offspring comprising the desired allele can be repeatedly backcrossed to a line having a desired genetic background and selected for the desired allele, to result in the allele becoming fixed in a selected genetic background. The process of "introgressing" is often referred to as "backcrossing" when the process is repeated two or more times. "Introgression fragment" or "introgression segment" or "introgression region" refers to a chromosome fragment (or chromosome part or region) which has been introduced into another plant of the same or related species either artificially or naturally such as by crossing or traditional breeding techniques, such as backcrossing, i.e. the introgressed fragment is the result of breeding methods referred to by the verb "to introgress" (such as backcrossing). It is understood that the term "introgression fragment" never includes a whole chromosome, but only a part of a chromosome. The introgression fragment can be large, e.g. even three quarter or half of a chromosome, but is preferably smaller, such as about 15 Mb or less, such as about 10 Mb or less, about 9 Mb or less, about 8 Mb or less, about 7 Mb or less, about 6 Mb or less, about 5 Mb or less, about 4 Mb or less, about 3 Mb or less, about 2.5 Mb or 2 Mb or less, about 1 Mb (equals 1,000,000 base pairs) or less, or about 0.5 Mb (equals 500,000 base pairs) or less, such as about 200,000 bp (equals 200 kilo base pairs) or less, about 100,000 bp (100 kb) or less, about 50,000 bp (50 kb) or less, about 25,000 bp (25 kb) or less.

A genetic element, an introgression fragment, or a gene or allele conferring a trait (such as increased pathogen resistance or tolerance) is said to be "obtainable from" or can be "obtained from" or "derivable from" or can be "derived from" or "as present in" or "as found in" a plant or plant part as described herein elsewhere if it can be transferred from the plant in which it is present into another plant in which it is not present (such as a line or variety) using traditional breeding techniques without resulting in a phenotypic change of the recipient plant apart from the addition of the trait conferred by the genetic element, locus, introgression fragment, gene or allele. The terms are used interchangeably and the genetic element, locus, introgression fragment, gene or allele can thus be transferred into any other genetic background lacking the trait. Not only pants comprising the genetic element, locus, introgression fragment, gene or allele can be used, but also progeny/descendants from such plants which have been selected to retain the genetic element, locus, introgression fragment, gene or allele, can be used and are encompassed herein. Whether a plant (or genomic DNA, cell or tissue of a plant) comprises the same genetic element, locus, introgression fragment, gene or allele as obtainable from such plant can be determined by the skilled person using one or more techniques known in the art, such as phenotypic assays, whole genome sequencing, molecular marker analysis, trait mapping, chromosome painting, allelism tests and the like, or combinations of techniques. It will be understood that transgenic plants may also be encompassed.

In certain embodiments, the polynucleic acid is introduced (and genomically integrated) recombinantly or transgenically. The polynucleic acid may be introduced (and genomically integrated) at the native locus, to replace an endogenous polynucleic acid (such as the polynucleic acid not conferring pathogen resistance), or may be introduced (and genomically integrated) at a locus different than the endogenous locus (e.g. by random integration in the genome). In certain embodiments, the method for generating a maize plant or plant part comprises transforming a plant or plant part, preferably a plant cell, more preferably a protoplast, with the polynucleic acid, which may be provided on a vector, as described herein elsewhere. In certain embodiments, the polynucleic acid has a different sequence than an endogenous polynucleic acid (such as an endogenous polynucleic acid not conferring pathogen resistance).

As used herein the terms "genetic engineering", "transformation" and "genetic modification" are all used herein as synonyms for the transfer of isolated and cloned genes into the DNA, usually the chromosomal DNA or genome, of another organism.

"Transgenic" or "genetically modified organisms" (GMOs) as used herein are organisms whose genetic material has been altered using techniques generally known as "recombinant DNA technology". Recombinant DNA technology encompasses the ability to combine DNA molecules from different sources into one molecule ex vivo (e.g. in a test tube). The term "transgenic" here means genetically modified by the introduction of a non-endogenous nucleic acid sequence. Typically a species-specific nucleic acid sequence is introduced in a form, arrangement or quantity into the cell in a location where the nucleic acid sequence does not occur naturally in the cell. This terminology generally does not cover organisms whose genetic composition has been altered by conventional cross-breeding or by "mutagenesis" breeding, as these methods predate the discovery of recombinant DNA techniques. "Non-transgenic" as used herein refers to plants and food products derived from plants that are not "transgenic" or "genetically modified organisms" as defined above.

"Transgene" or "chimeric gene" refers to a genetic locus comprising a DNA sequence, such as a recombinant gene, which has been introduced into the genome of a plant by transformation, such as Agrobacterium mediated transformation. A plant comprising a transgene stably integrated into its genome is referred to as "transgenic plant".

"Gene editing" or "genome editing" refers to genetic engineering in which in which DNA or RNA is inserted, deleted, modified or replaced in the genome of a living organism. Gene editing may comprise targeted or non-targeted (random) mutagenesis. Targeted mutagenesis may be accomplished for instance with designer nucleases, such as for instance with meganucleases, zinc finger nucleases (ZFNs), transcription activator-like effector-based nucleases (TALEN), and the clustered regularly interspaced short palindromic repeats (CRISPR/Cas9) system. These nucleases create site-specific double-strand breaks (DSBs) at desired locations in the genome. The induced double-strand breaks are repaired through nonhomologous end-joining (NHEJ) or homologous recombination (HR), resulting in targeted mutations or nucleic acid modifications. The use of designer nucleases is particularly suitable for generating gene knockouts or knockdowns. In certain embodiments, designer nucleases are developed which specifically introduce one or more of the molecular marker (allele) according to the invention as described herein. Delivery and expression systems of designer nuclease systems are well known in the art.

In certain embodiments, the nuclease or targeted/site-specific/homing nuclease is, comprises, consists essentially of, or consists of a (modified) CRISPR/Cas system or complex, a (modified) Cas protein, a (modified) zinc finger, a (modified) zinc finger nuclease (ZFN), a (modified) transcription factor-like effector (TALE), a (modified) transcription factor-like effector nuclease (TALEN), or a (modified) meganuclease. In certain embodiments, said (modified) nuclease or targeted/site-specific/homing nuclease is, comprises, consists essentially of, or consists of a (modified) RNA-guided nuclease. It will be understood that in certain embodiments, the nucleases may be codon optimized for expression in plants. As used herein, the term "targeting" of a selected nucleic acid sequence means that a nuclease or nuclease complex is acting in a nucleotide sequence specific manner. For instance, in the context of the CRISPR/Cas system, the guide RNA is capable of hybridizing with a selected nucleic acid sequence. As uses herein, "hybridization" or "hybridizing" refers to a reaction in which one or more polynucleotides react to form a complex that is stabilized via hydrogen bonding between the bases of the nucleotide residues, i.e. a process in which a single-stranded nucleic acid molecule attaches itself to a complementary nucleic acid strand, i.e. agrees with this base pairing. Standard procedures for hybridization are described, for example, in Sambrook et al. (Molecular Cloning. A Laboratory Manual, Cold Spring Harbor Laboratory Press, 3rd edition 2001). The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PGR, or the cleavage of a polynucleotide by an enzyme. A sequence capable of hybridizing with a given sequence is referred to as the "complement" of the given sequence. Preferably this will be understood to mean an at least 50%, more preferably at least 55%, 60%, 65%, 70%, 75%, 80% or 85%, more preferably 90%, 91%, 92%, 93%, 94%, 95%, 96%, 97%, 98% or 99% of the bases of the nucleic acid strand form base pairs with the complementary nucleic acid strand. The possibility of such binding depends on the stringency of the hybridization conditions.

Gene editing may involve transient, inducible, or constitutive expression of the gene editing components or systems. Gene editing may involve genomic integration or episomal presence of the gene editing components or systems. Gene editing components or systems may be provided on vectors, such as plasmids, which may be delivered by appropriate delivery vehicles, as is known in the art. Preferred vectors are expression vectors.

Gene editing may comprise the provision of recombination templates, to effect homology directed repair (HDR). For instance a genetic element may be replaced by gene editing in which a recombination template is provided. The DNA may be cut upstream and downstream of a sequence which needs to be replaced. As such, the sequence to be replaced is excised from the DNA. Through HDR, the excised sequence is then replaced by the template. In certain embodiments, the marker (allele) of the invention as described herein may be provided on/as a template. By designing the system such that double strand breaks are introduced upstream and downstream of the corresponding region in the genome of a plant not comprising the marker (allele), this region is excised and can be replaced with the template comprising the marker (allele) of the invention. In this way, introduction of the marker (allele) of the invention in a plant need not involve multiple backcrossing, in particular in a plant of specific genetic background. Similarly, the polynucleic acid of the invention may be provided on/as a template. More advantageously however, the polynucleic acid of the invention may be generated without the use of a recombination template, but solely through the endonuclease action leading to a double strand DNA break which is repaired by NHEJ, resulting in the generation of indels.

In certain embodiments, the nucleic acid modification is effected by random mutagenesis. Cells or organisms may be exposed to mutagens such as UV radiation or mutagenic chemicals (such as for instance such as ethyl methanesulfonate (EMS)), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nucleases. The products are then separated by size, such as by HPLC. See also McCallum et al. "Targeted screening for induced mutations"; Nat Biotechnol. 2000 Apr;18(4):455-7 and McCallum et al. "Targeting induced local lesions IN genomes (TILLING) for plant functional genomics"; Plant Physiol. 2000 Jun; 123(2):439-42.

As used herein, the term "homozygote" refers to an individual cell or plant having the same alleles at one or more or all loci. When the term is used with reference to a specific locus or gene, it means at least that locus or gene has the same alleles. As used herein, the term "homozygous" means a genetic condition existing when identical alleles reside at corresponding loci on homologous chromosomes. As used herein, the term "heterozygote" refers to an individual cell or plant having different alleles at one or more or all loci. When the term is used with reference to a specific locus or gene, it means at least that locus or gene has different alleles. As used herein, the term "heterozygous" means a genetic condition existing when different alleles reside at corresponding loci on homologous chromosomes. In certain embodiments, the haplotype and/or one or more marker(s) as described herein is/are homozygous. In certain embodiments, the haplotype and/or one or more marker(s) as described herein are heterozygous. In certain embodiments, the haplotype allele and/or one or more marker(s) allele(s) as described herein is/are homozygous. In certain embodiments, the haplotype allele and/or one or more marker(s) allele(s) as described herein are heterozygous.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (BI2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the BI2seq program can be found in the readme file accompanying BLASTZ. BI2seq performs a comparison between two sequences using either the BLASTN or BLASTP algorithm.

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g. , C:\seq I .txt); -j is set to a file containing the second nucleic acid sequence to be compared (e.g. , C:\seq2.txt); -p is set to blastn; -o is set to any desired file name (e.g. , C :\output.txt); -q is set to - 1 ; -r is set to 2; and all other options are left at their default setting. The following command will generate an output file containing a comparison between two sequences: C:\B12seq -i c:\seql .txt -j c:\seq2.txt -p blastn -o c:\output.txt -q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the BI2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1 , while 78.15, 78.16, 78.17, 78.18, and 78.19 are rounded up to 78.2. It also is noted that the length value will always be an integer.

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used. The terms "nucleotide sequence(s)", "polynucleotide(s)", "nucleic acid sequence(s)", "nucleic acid(s)", "nucleic acid molecule", "polynucleic acid(s)" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

An "isolated nucleic acid sequence" or "isolated DNA" refers to a nucleic acid sequence which is no longer in the natural environment from which it was isolated, e.g. the nucleic acid sequence in a bacterial host cell or in the plant nuclear or plastid genome. When referring to a "sequence" herein, it is understood that the molecule having such a sequence is referred to, e.g. the nucleic acid molecule. A "host cell" or a "recombinant host cell" or "transformed cell" are terms referring to a new individual cell (or organism) arising as a result of at least one nucleic acid molecule, having been introduced into said cell. The host cell is preferably a plant cell or a bacterial cell. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

When reference is made to a nucleic acid sequence (e.g. DNA or genomic DNA) having "substantial sequence identity to" a reference sequence or having a sequence identity of at least 80%>, e.g. at least 85%, 90%, 95%, 98%> or 99%> nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

Amino acid substitutions encompass amino acid alterations in which an amino acid is replaced with a different naturally-occurring amino acid residue. Such substitutions may be classified as "conservative<1>, in which an amino acid residue contained in the wild-type protein is replaced with another naturally-occurring amino acid of similar character, for example Gly<->Ala, Val<->Ile<->Leu, Asp<->Glu, Lys<->Arg, Asn<->Gln or Phe<->Trp<->Tyr. Substitutions encompassed by the present invention may also be "non-conservative", in which an amino acid residue which is present in the wild-type protein is substituted with an amino acid with different properties, such as a naturally-occurring amino acid from a different group (e.g. substituting a charged or hydrophobic amino acid with alanine. "Similar amino acids", as used herein, refers to amino acids that have similar amino acid side chains, i.e. amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

A used herein, the term "endogenous" refers to a gene or allele which is present in its natural genomic location. The term "endogenous" can be used interchangeably with "native". This does not however exclude the presence of one or more nucleic acid differences with the wild-type allele. In particular embodiments, the difference with a wild-type allele can be limited to less than 9 preferably less than 6, more particularly less than 3 nucleotide differences, such as 0 nucleotides difference. More particularly, the difference with the wildtype sequence can be in only one nucleotide. Preferably, the endogenous allele encodes a modified protein having less than 9, preferably less than 6, more particularly less than 3 and even more preferably only one or no amino acid difference with the wild-type protein.

A used herein, the term "exogenous polynucleotide" refers to a polynucleotide, such as a gene (or cDNA) or allele which is or has been recombinantly introduced in a cell (or plant). The exogenous polynucleotide may be episomal or genomically integrated. Integration may be random or site-directed. Integration may include replacement of a corresponding endogenous polynucleotide. It will be understood that an exogenous polynucleotide is not naturally present in the cell or plant.

As used herein, the B73 reference genome AGPv4 (or AGPv04) refers to the assembly B73 RefGen_v4 (also known as AGPv4, B73 RefGen_v4) as provided on the Maize Genetics and Genomics Database (https://www.maizegdb.org/genome/genome_assembly/Zm-B73-REFERENCE-GRAMEN E-4.0).

Methods for screening for the presence of the polynucleic acid of the invention, or the (molecular) marker(s) (alleles) as described herein are known in the art. Without limitation, screening may encompass or comprise sequencing, hybridization based methods (such as (dynamic) allele-specific hybridization, molecular beacons, SNP microarrays), enzyme based methods (such as PCR, KASP (Kompetitive Allele Specific PCR), RFLP, ALFP, RAPD, Flap endonuclease, primer extension, 5'-nuclease, oligonucleotide ligation assay), post-amplification methods based on physical properties of DNA (such as single strand conformation polymorphism, temperature gradient gel electrophoresis, denaturing high performance liquid chromatography, high-resolution melting of the entire amplicon, use of DNA mismatch-binding proteins, SNPlex, surveyor nuclease assay), etc.

In an aspect, the invention relates to a method for identifying a plant, plant part or plant material, in particular a maize plant or plant part, comprising screening for the presence of or detecting or identifying a cytoplasmic male sterility restorer locus on chromosome 3, in particular RF-03-01, as described herein elsewhere.

In an aspect, the invention relates to a method for identifying a plant, plant part or plant material, in particular a maize plant or plant part, comprising screening for the absence of a cytoplasmic male sterility restorer locus on chromosome 3, in particular RF-03-01, as described herein elsewhere.

In an aspect, the invention relates to a method for identifying a plant, plant part or plant material, in particular a maize plant or plant part, comprising screening for the presence of or detecting or identifying a cytoplasmic male sterility maintainer locus on chromosome 3, in particular RF-03-01, as described herein elsewhere.

In an aspect, the invention relates to a method for identifying a plant, plant part or plant material, in particular a maize plant or plant part, comprising screening for the absence of a cytoplasmic male sterility maintainer locus on chromosome 3, in particular RF-03-01, as described herein elsewhere.

In an aspect, the invention relates to a method for identifying a plant, plant part or plant material, in particular a maize plant or plant part, comprising screening for the presence of or detecting or identifying (a haplotype associated with/linked) with a cytoplasmic male sterility restorer locus on chromosome 3, in particular RF-03-01, as described herein elsewhere.

In an aspect, the invention relates to a method for identifying a plant or plant part, in particular a maize plant or plant part, comprising screening for the absence of (a haplotype associated with/linked with) a cytoplasmic male sterility restorer locus on chromosome 3, in particular RF-03-01, as described herein elsewhere.

In an aspect, the invention relates to a method for identifying a plant, plant part or plant material, in particular a maize plant or plant part, comprising screening for the presence of or detecting or identifying (a haplotype associated with/linked with) a cytoplasmic male sterility maintainer locus on chromosome 3, in particular RF-03-01, as described herein elsewhere.

In an aspect, the invention relates to a method for identifying a plant, plant part or plant material, in particular a maize plant or plant part, comprising screening for the absence of (a haplotype associated with/linked with) a cytoplasmic male sterility maintainer locus on chromosome 3, in particular RF-03-01, as described herein elsewhere.

In an aspect, the invention relates to a method for identifying a plant, plant part or plant material, in particular a maize plant or plant part, comprising screening for the presence of or detecting or identifying one or more molecular markers of Table 4 or Table 5. In an aspect, the invention relates to a method for identifying a plant or plant part, in particular a maize plant or plant part, comprising screening for the presence of or detecting or identifying two or more molecular markers of Table 4 or Table 5. In an aspect, the invention relates to a method for identifying a plant or plant part, in particular a maize plant or plant part, comprising screening for the presence of or detecting or identifying three or more molecular markers of Table 4 or Table 5. In certain embodiments, the method is a method for identifying a plant or plant part comprising a cytoplasmic male sterility restorer locus. In certain embodiments, the method is a method for identifying a plant or plant part comprising a cytoplasmic male sterility maintainer locus.

In an aspect, the invention relates to a method for identifying a plant, plant part or plant material, in particular a maize plant or plant part, comprising screening for the presence of or detecting or identifying one or more molecular marker alleles of Table 4 or Table 5 (and having a polymorphism corresponding to or comprised in a cytoplasmic male sterility restorer locus). In an aspect, the invention relates to a method for identifying a plant or plant part, in particular a maize plant or plant part, comprising screening for the presence of or detecting or identifying two or more molecular marker alleles of Table 4 or Table 5 (and having a polymorphism corresponding to or comprised in a cytoplasmic male sterility restorer locus). In an aspect, the invention relates to a method for identifying a plant or plant part, in particular a maize plant or plant part, comprising screening for the presence of or detecting or identifying three or more molecular marker alleles of Table 4 or Table 5 (and having a polymorphism corresponding to or comprised in a cytoplasmic male sterility restorer locus).

In an aspect, the invention relates to a method for identifying a plant, plant part or plant material, in particular a maize plant or plant part, comprising screening for the presence of or detecting or identifying one or more molecular marker alleles of Table 4 or Table 5 (and having a polymorphism corresponding to or comprised in a cytoplasmic male sterility maintainer locus). In an aspect, the invention relates to a method for identifying a plant or plant part, in particular a maize plant or plant part, comprising screening for the presence of or detecting or identifying two or more molecular marker alleles of Table 4 or Table 5 (and having a polymorphism corresponding to or comprised in a cytoplasmic male sterility maintainer locus). In an aspect, the invention relates to a method for identifying a plant or plant part, in particular a maize plant or plant part, comprising screening for the presence of or detecting or identifying three or more molecular marker alleles of Table 4 or Table 5 (and having a polymorphism corresponding to or comprised in a cytoplasmic male sterility maintainer locus).

In an aspect, the invention relates to a method for identifying a plant, plant part or plant material, in particular a maize plant or plant part, comprising screening for the presence of or detecting or identifying one or more polynucleic acid sequences having a sequence as set forth in any of SEQ ID NOs: 1, 5, 9, 13, 2, 6, 10, or 14. In an aspect, the invention relates to a method for identifying a plant or plant part, in particular a maize plant or plant part, comprising screening for the presence of or detecting or identifying two or more polynucleic acid sequences having a sequence as set forth in any of SEQ ID NOs: 1, 5, 9, 13, 2, 6, 10, or 14. In an aspect, the invention relates to a method for identifying a plant or plant part, in particular a maize plant or plant part, comprising screening for the presence of or detecting or identifying three or more polynucleic acid sequences having a sequence as set forth in any of SEQ ID NOs: 1, 5, 9, 13, 2, 6, 10, or 14. In certain embodiments, the method is a method for identifying a plant or plant part comprising a cytoplasmic male sterility restorer locus. In certain embodiments, the method is a method for identifying a plant or plant part comprising a cytoplasmic male sterility maintainer locus.

In an aspect, the invention relates to a method for identifying a plant, plant part or plant material, in particular a maize plant or plant part, comprising screening for the presence of or detecting or identifying one or more polynucleic acid sequences having a sequence as set forth in any of SEQ ID NOs: 1, 5, 9, or 13. In an aspect, the invention relates to a method for identifying a plant or plant part, in particular a maize plant or plant part, comprising screening for the presence of or detecting or identifying two or more polynucleic acid sequences having a sequence as set forth in any of SEQ ID NOs: 1, 5, 9, or 13. In an aspect, the invention relates to a method for identifying a plant or plant part, in particular a maize plant or plant part, comprising screening for the presence of or detecting or identifying three or more polynucleic acid sequences having a sequence as set forth in any of SEQ ID NOs: 1, 5, 9, or 13. In certain embodiments, the method is a method for identifying a plant or plant part comprising a cytoplasmic male sterility restorer locus.

In an aspect, the invention relates to a method for identifying a plant, plant part or plant material, in particular a maize plant or plant part, comprising screening for the presence of or detecting or identifying one or more polynucleic acid sequences having a sequence as set forth in any of SEQ ID NOs: 2, 6, 10, or 14. In an aspect, the invention relates to a method for identifying a plant or plant part, in particular a maize plant or plant part, comprising screening for the presence of or detecting or identifying two or more polynucleic acid sequences having a sequence as set forth in any ofSEQ ID NOs: 2, 6, 10, or 14. In an aspect, the invention relates to a method for identifying a plant or plant part, in particular a maize plant or plant part, comprising screening for the presence of or detecting or identifying three or more polynucleic acid sequences having a sequence as set forth in any of SEQ ID NOs: 2, 6, 10, or 14. In certain embodiments, the method is a method for identifying a plant or plant part comprising a cytoplasmic male sterility maintainer locus.

In an aspect, the invention relates to a method for identifying a plant, plant part or plant material, in particular a maize plant or plant part, comprising screening for the presence of or detecting or identifying one or more genes selected from Zm00001d043358, Zm00001d043352, Zm00001d043356, and Zm00001d043357 having respectively a coding sequence as set forth in any of SEQ ID NOs: 201, 203, 205, 207, 3, 7, 11, or 15. In an aspect, the invention relates to a method for identifying a plant or plant part, in particular a maize plant or plant part, comprising screening for the presence of or detecting or identifying two or more genes selected from Zm00001d043358, Zm00001d043352, Zm00001d043356, and Zm00001d043357 having respectively a coding sequence as set forth in any of SEQ ID NOs: 201, 203, 205, 207, 3, 7, 11, or 15. In an aspect, the invention relates to a method for identifying a plant or plant part, in particular a maize plant or plant part, comprising screening for the presence of or detecting or identifying three or more genes selected from Zm00001d043358, Zm00001d043352, Zm00001d043356, and Zm00001d043357 having respectively a coding sequence as set forth in any of SEQ ID NOs: 201, 203, 205, 207, 3, 7, 11, or 15. In certain embodiments, the method is a method for identifying a plant or plant part comprising a cytoplasmic male sterility restorer locus. In certain embodiments, the method is a method for identifying a plant or plant part comprising a cytoplasmic male sterility maintainer locus.

In an aspect, the invention relates to a method for identifying a plant, plant part or plant material, in particular a maize plant or plant part, comprising screening for the presence of or detecting or identifying one or more genes selected from Zm00001d043358, Zm00001d043352, Zm00001d043356, and Zm00001d043357 having respectively a coding sequence as set forth in any of SEQ ID NOs: 201, 203, 205, or 207. In an aspect, the invention relates to a method for identifying a plant or plant part, in particular a maize plant or plant part, comprising screening for the presence of or detecting or identifying two or more genes selected from Zm00001d043358, Zm00001d043352, Zm00001d043356, and Zm00001d043357 having respectively a coding sequence as set forth in any of SEQ ID NOs: 201, 203, 205, or 207. In an aspect, the invention relates to a method for identifying a plant or plant part, in particular a maize plant or plant part, comprising screening for the presence of or detecting or identifying three or more genes selected from Zm00001d043358, Zm00001d043352, Zm00001d043356, and Zm00001d043357 having respectively a coding sequence as set forth in any of SEQ ID NOs: 201, 203, 205, or 207. In certain embodiments, the method is a method for identifying a plant or plant part comprising a cytoplasmic male sterility restorer locus.

In an aspect, the invention relates to a method for identifying a plant, plant part or plant material, in particular a maize plant or plant part, comprising screening for the presence of or detecting or identifying one or more genes selected from Zm00001d043358, Zm00001d043352, Zm00001d043356, and Zm00001d043357 having respectively a coding sequence as set forth in any of SEQ ID NOs: 3, 7, 11, or 15. In an aspect, the invention relates to a method for identifying a plant or plant part, in particular a maize plant or plant part, comprising screening for the presence of or detecting or identifying two or more genes selected from Zm00001d043358, Zm00001d043352, Zm00001d043356, and Zm00001d043357 having respectively a coding sequence as set forth in any of SEQ ID NOs: 3, 7, 11, or 15. In an aspect, the invention relates to a method for identifying a plant or plant part, in particular a maize plant or plant part, comprising screening for the presence of or detecting or identifying three or more genes selected from Zm00001d043358, Zm00001d043352, Zm00001d043356, and Zm00001d043357 having respectively a coding sequence as set forth in any of SEQ ID NOs: 3, 7, 11, or 15. In certain embodiments, the method is a method for identifying a plant or plant part comprising a cytoplasmic male sterility maintainer locus.

In an aspect, the invention relates to a method for identifying a plant, plant part or plant material, in particular a maize plant or plant part, comprising screening for the presence of or detecting or identifying one or more genes selected from Zm00001d043358, Zm00001d043352, Zm00001d043356, and Zm00001d043357 encoding respectively a polypeptide having a sequence as set forth in any of SEQ ID NOs: 202, 204, 206, 208, 4, 8, 12, or 16. In an aspect, the invention relates to a method for identifying a plant or plant part, in particular a maize plant or plant part, comprising screening for the presence of or detecting or identifying two or more genes selected from Zm00001d043358, Zm00001d043352, Zm00001d043356, and Zm00001d043357 encoding respectively a polypeptide having a sequence as set forth in any of SEQ ID NOs: 202, 204, 206, 208, 4, 8, 12, or 16. In an aspect, the invention relates to a method for identifying a plant or plant part, in particular a maize plant or plant part, comprising screening for the presence of or detecting or identifying three or more genes selected from Zm00001d043358, Zm00001d043352, Zm00001d043356, and Zm00001d043357 encoding respectively a polypeptide having a sequence as set forth in any of SEQ ID NOs: 202, 204, 206, 208, 4, 8, 12, or 16. In certain embodiments, the method is a method for identifying a plant or plant part comprising a cytoplasmic male sterility restorer locus. In certain embodiments, the method is a method for identifying a plant or plant part comprising a cytoplasmic male sterility maintainer locus. In an aspect, the invention relates to a method for identifying a plant, plant part or plant material, in particular a maize plant or plant part, comprising screening for the presence of or detecting or identifying one or more genes selected from Zm00001d043358, Zm00001d043352, Zm00001d043356, and Zm00001d043357 encoding respectively a polypeptide having a sequence as set forth in any of SEQ ID NOs: 202, 204, 206, or 208, preferably selected from Zm00001d043358, Zm00001d043352, and Zm00001d043357 encoding respectively a polypeptide having a sequence as set forth in any of SEQ ID NOs: 202, 204, or 208. In an aspect, the invention relates to a method for identifying a plant or plant part, in particular a maize plant or plant part, comprising screening for the presence of or detecting or identifying two or more genes selected from Zm00001d043358, Zm00001d043352, Zm00001d043356, and Zm00001d043357 encoding respectively a polypeptide having a sequence as set forth in any of SEQ ID NOs: 202, 204, 206, or 208, preferably selected from Zm00001d043358, Zm00001d043352, and Zm00001d043357 encoding respectively a polypeptide having a sequence as set forth in any of SEQ ID NOs: 202, 204, or 208. In an aspect, the invention relates to a method for identifying a plant or plant part, in particular a maize plant or plant part, comprising screening for the presence of or detecting or identifying three or more genes selected from Zm00001d043358, Zm00001d043352, Zm00001d043356, and Zm00001d043357 encoding respectively a polypeptide having a sequence as set forth in any of SEQ ID NOs: 202, 204, 206, or 208, preferably selected from Zm00001d043358, Zm00001d043352, and Zm00001d043357 encoding respectively a polypeptide having a sequence as set forth in any of SEQ ID NOs: 202, 204, or 208. In certain embodiments, the method is a method for identifying a plant or plant part comprising a cytoplasmic male sterility restorer locus.

In an aspect, the invention relates to a method for identifying a plant, plant part or plant material, in particular a maize plant or plant part, comprising screening for the presence of or detecting or identifying one or more genes selected from Zm00001d043358, Zm00001d043352, and Zm00001d043357 encoding respectively a polypeptide having a sequence as set forth in any of SEQ ID NOs: 4, 8, or 16. In an aspect, the invention relates to a method for identifying a plant or plant part, in particular a maize plant or plant part, comprising screening for the presence of or detecting or identifying two or more genes selected from Zm00001d043358, Zm00001d043352, and Zm00001d043357 encoding respectively a polypeptide having a sequence as set forth in any of SEQ ID NOs: 4, 8, or 16. In an aspect, the invention relates to a method for identifying a plant or plant part, in particular a maize plant or plant part, comprising screening for the presence of or detecting or identifying three or more genes selected from Zm00001d043358, Zm00001d043352, and Zm00001d043357 encoding respectively a polypeptide having a sequence as set forth in any of SEQ ID NOs: 4, 8, or 16. In certain embodiments, the method is a method for identifying a plant or plant part comprising a cytoplasmic male sterility maintainer locus.

In an aspect, the invention relates to a method for identifying a plant, plant part or plant material, in particular a maize plant or plant part, comprising screening for the presence of or detecting or identifying one or more polynucleic acid sequences having a sequence as set forth in any of SEQ ID NOs: 17 to 200. In an aspect, the invention relates to a method for identifying a plant or plant part, in particular a maize plant or plant part, comprising screening for the presence of or detecting or identifying two or more polynucleic acid sequences having a sequence as set forth in any of SEQ ID NOs: 17 to 200. In an aspect, the invention relates to a method for identifying a plant or plant part, in particular a maize plant or plant part, comprising screening for the presence of or detecting or identifying three or more polynucleic acid sequences having a sequence as set forth in any of SEQ ID NOs: 17 to 200.

In an aspect, the invention relates to a method for identifying a plant, plant part or plant material, in particular a maize plant or plant part, comprising screening for the presence of or detecting or identifying one or more polynucleic acid sequences having a sequence as set forth in any of SEQ ID NOs: 17 to 200 and having a polymorphism corresponding to a cytoplasmic male sterility restorer locus. In an aspect, the invention relates to a method for identifying a plant or plant part, in particular a maize plant or plant part, comprising screening for the presence of or detecting or identifying two or more polynucleic acid sequences having a sequence as set forth in any of SEQ ID NOs: 17 to 200 and having a polymorphism corresponding to a cytoplasmic male sterility restorer locus. In an aspect, the invention relates to a method for identifying a plant or plant part, in particular a maize plant or plant part, comprising screening for the presence of or detecting or identifying three or more polynucleic acid sequences having a sequence as set forth in any of SEQ ID NOs: 17 to 200 and having a polymorphism corresponding to a cytoplasmic male sterility restorer locus.

In an aspect, the invention relates to a method for identifying a plant, plant part or plant material, in particular a maize plant or plant part, comprising screening for the presence of or detecting or identifying one or more polynucleic acid sequences having a sequence as set forth in any of SEQ ID NOs: 17 to 200 and having a polymorphism not corresponding to a cytoplasmic male sterility maintainer locus. In an aspect, the invention relates to a method for identifying a plant or plant part, in particular a maize plant or plant part, comprising screening for the presence of or detecting or identifying two or more polynucleic acid sequences having a sequence as set forth in any of SEQ ID NOs: 17 to 200 and having a polymorphism not corresponding to a cytoplasmic male sterility maintainer locus. In an aspect, the invention relates to a method for identifying a plant or plant part, in particular a maize plant or plant part, comprising screening for the presence of or detecting or identifying three or more polynucleic acid sequences having a sequence as set forth in any of SEQ ID NOs: 17 to 200 and having a polymorphism not corresponding to a cytoplasmic male sterility maintainer locus.

In an aspect, the invention relates to a method for identifying a plant, plant part or plant material, in particular a maize plant or plant part, comprising screening for the presence of or detecting or identifying one or more, two or more, or three or more of the following polymorphisms (positions correspond to maize reference B73 AGPv4 chromosome 3):

| Position AGPv4 | polymorphism | |
|---|---|---|
| | maintainer | restorer |
| 195629901 | ade | gua |
| 195639694 | thy | cyt |
| 195677799 | gua | ade |
| 195678356 | cyt | gua |
| 195680790 | gua | ade |
| 195732936 | gua | ade |
| 195733916 | cyt | ade |
| 195783701 | cyt | thy |
| 196070086 | cyt | thy |
| 196198736 | ade | gua |
| 196244714 | thy | cyt |
| 196653746 | cyt | thy |
| 196693501 | cyt | ade |
| 196702811 | ade | gua |
| 196704008 | ade | gua |
| 196704096 | thy | cyt |
| 196704169 | thy | cyt |
| 196704290 | thy | cyt |
| 196705470 | thy | ade |
| 196706697 | gua | ade |
| 196706755 | cyt | ade |
| 196707190 | ade | gua |
| 196707415 | thy | ade |
| 196707997 | gua | gua |
| 196773893 | cyt | thy |
| 196774122 | cyt | thy |
| 196774333 | gua | ade |
| 196774502 | gua | ade |
| 196774823 | cyt | thy |
| 196774965 | cyt | gua |
| 196775596 | gua | ade |
| 196776600 | ade | gua |
| 196776877 | cyt | thy |
| 196840068 | cyt | ade |
| 196840815 | gua | cyt |
| 196841649 | gua | cyt |
| 196841990 | cyt | ade |
| 196843002 | cyt | gua |
| 196843335 | cyt | gua |
| 196844084 | cyt | thy |
| 196851451 | thy | cyt |
| 196853534 | gua | cyt |
| 196853762 | cyt | thy |
| 196880373 | cyt | thy |
| 196985856 | cyt | ade |
| 196985883 | cyt | thy |
| 196987284 | cyt | thy |
| 196989025 | gua | thy |
| 196989252 | gua | ade |
| 196989377 | gua | ade |
| 196989408 | ade | gua |
| 197453646 | ade | gua |
| 197453708 | ade | gua |
| 197453708 | thy | cyt |
| 197454448 | gua | ade |
| 197454630 | thy | cyt |
| 197454657 | thy | cyt |
| 197454744 | thy | gua |
| 197454780 | cyt | gua |
| 197454833 | ade | gua |
| 197455007 | cyt | thy |
| 197455034 | thy | cyt |
| 197456922 | thy | cyt |
| 197457134 | ade | gua |
| 197457214 | cyt | thy |
| 197457351 | gua | ade |
| 197457603 | gua | ade |
| 197459188 | thy | cyt |
| 197487965 | gua | ade |
| 197488751 | cyt | thy |
| 197489067 | gua | ade |
| 197524489 | gua | thy |
| 197524855 | cyt | thy |
| 197525193 | gua | ade |
| 197525365 | gua | thy |
| 197525625 | thy | cyt |
| 197525990 | cyt | thy |
| 197526621 | ade | gua |
| 197526690 | gua | ade |
| 197527582 | gua | ade |
| 197527682 | cyt | gua |
| 197528652 | cyt | thy |
| 197556227 | gua | ade |
| 197609686 | gua | thy |
| 197609730 | ade | gua |
| 197611692 | ade | gua |
| 197611832 | gua | ade |
| 197611894 | gua | ade |
| 197613135 | ade | cyt |
| 197613658 | ade | cyt |
| 197613658 | thy | gua |
| 197615089 | gua | ade |
| 197615461 | cyt | thy |
| 197631560 | ade | gua |
| 197631688 | gua | ade |
| 197632590 | gua | ade |
| 197632706 | ade | gua |
| 197633370 | thy | cyt |
| 197633860 | gua | ade |
| 197638778 | thy | cyt |
| 197638949 | thy | cyt |
| 197639380 | gua | ade |
| 197652023 | cyt | thy |
| 197652478 | ade | gua |
| 197653125 | gua | thy |
| 197654542 | cyt | gua |
| 197687270 | cyt | thy |
| 197687524 | cyt | thy |
| 197688212 | gua | cyt |
| 197688445 | thy | cyt |
| 197688492 | thy | cyt |
| 197692991 | ade | gua |
| 197692996 | thy | cyt |
| 197694265 | thy | cyt |
| 197695368 | cyt | thy |
| 197695591 | ade | gua |
| 197695857 | thy | cyt |
| 197696192 | thy | ade |
| 197696732 | gua | thy |
| 197696762 | gua | ade |
| 197697327 | gua | ade |
| 197697514 | cyt | thy |
| 197698249 | gua | ade |
| 197698278 | gua | ade |
| 197708137 | thy | cyt |
| 197708334 | thy | ade |
| 197758073 | thy | gua |
| 197760175 | gua | ade |
| 197761254 | cyt | ade |
| 197761305 | ade | cyt |
| 197776540 | thy | cyt |
| 197777549 | ade | gua |
| 197777618 | thy | ade |
| 197778110 | ade | gua |
| 197781849 | cyt | ade |
| 197781961 | gua | ade |
| 197784696 | ade | gua |
| 197785166 | cyt | thy |
| 197785270 | cyt | gua |
| 197786148 | gua | ade |
| 197786155 | cyt | gua |
| 197787768 | cyt | thy |
| 197806056 | gua | ade |
| 197806483 | cyt | thy |
| 197812595 | gua | ade |
| 197813589 | thy | cyt |
| 197814082 | gua | ade |
| 197840802 | thy | cyt |
| 197840951 | gua | ade |
| 197855989 | cyt | thy |
| 197859323 | gua | cyt |
| 197860711 | ade | cyt |
| 197861373 | gua | ade |
| 197895272 | ade | gua |
| 197902823 | gua | ade |
| 197902855 | cyt | thy |
| 197902923 | cyt | thy |
| 197903119 | cyt | ade |
| 197903264 | thy | cyt |
| 197903302 | gua | cyt |
| 197903372 | cyt | thy |
| 197903475 | cyt | thy |
| 197903587 | gua | thy |
| 197903629 | gua | ade |
| 197903716 | thy | cyt |
| 197903816 | thy | gua |
| 197904016 | thy | thy |
| 197904655 | cyt | ade |
| 197906673 | ade | gua |
| 197907566 | gua | ade |
| 197907617 | ade | cyt |
| 197907653 | thy | cyt |
| 197907842 | thy | cyt |
| 197909824 | ade | ade |
| 197948546 | ade | gua |
| 197948580 | cyt | thy |
| 197948690 | cyt | ade |
| 197948831 | gua | gua |
| 197948879 | ade | gua |
| 197973632 | cyt | cyt |
| 197974493 | ade | gua |
| 197994208 | thy | gua |
| 198023432 | thy | cyt |
| 198023573 | cyt | gua |

By means of example, the invention relates to a method for identifying a plant or plant part, in particular a maize plant or plant part, comprising screening for the presence of or detecting or identifying a polymorphism at a position corresponding to maize reference B73 AGPv4 chromosome 3 position 195629901. By means of example, the invention relates to a method for identifying a plant or plant part, in particular a maize plant or plant part, comprising screening for the presence of or detecting or identifying a SNP at a position corresponding to maize reference B73 AGPv4 chromosome 3 position 195629901. By means of example, the invention relates to a method for identifying a plant or plant part, in particular a maize plant or plant part, comprising screening for the presence of or detecting or identifying a SNP at a position corresponding to maize reference B73 AGPv4 chromosome 3 position 195629901, wherein if a G is detected, said maize plant or plant part is a restorer or comprises a restorer (gene(s), locus, haplotype, genome, or phenotype), in particular the restorer of the invention. By means of example, the invention relates to a method for identifying a plant or plant part, in particular a maize plant or plant part, comprising screening for the presence of or detecting or identifying a SNP at a position corresponding to maize reference B73 AGPv4 chromosome 3 position 195629901,wherein if a nucleotide other than G is detected, said maize plant or plant part is a not a restorer or does not comprise a restorer (gene(s), locus, haplotype, genome, or phenotype), in particular the restorer of the invention. By means of example, the invention relates to a method for identifying a plant or plant part, in particular a maize plant or plant part, comprising screening for the presence of or detecting or identifying a SNP at a position corresponding to maize reference B73 AGPv4 chromosome 3 position 195629901,wherein if a nucleotide other than G is detected, said maize plant or plant part is a maintainer or comprise a maintainer (gene(s), locus, haplotype, genome, or phenotype), in particular the maintainer of the invention. By means of example, the invention relates to a method for identifying a plant or plant part, in particular a maize plant or plant part, comprising screening for the presence of or detecting or identifying a SNP at a position corresponding to maize reference B73 AGPv4 chromosome 3 position 195629901,wherein if a nucleotide A is detected, said maize plant or plant part is a not a restorer or does not comprise a restorer (gene(s), locus, haplotype, genome, or phenotype), in particular the restorer of the invention. By means of example, the invention relates to a method for identifying a plant or plant part, in particular a maize plant or plant part, comprising screening for the presence of or detecting or identifying a SNP at a position corresponding to maize reference B73 AGPv4 chromosome 3 position 195629901,wherein if a nucleotide A is detected, said maize plant or plant part is a maintainer or comprise a maintainer (gene(s), locus, haplotype, genome, or phenotype), in particular the maintainer of the invention.

Corresponding embodiments apply to each of the other polymorphisms/SNPs.

It is to be understood that the indicated nucleotide positions are the nucleotide positions of the indicated AGPv04 B73 chromosome 3 positions and that the marker positions in the maize plants according to the invention correspond to the indicated marker positions, but are or comprise not necessarily identical positions in a different genome (e.g. from a different race or line). The skilled person will understand that corresponding nucleotide positions can be determined by suitable alignment, as is known in the art.

The nucleotides (SNPs) at the positions indicated for the restorer allele allow screening for or the identification of the restorer phenotype according to the invention. The nucleotides (SNPs) at the positions indicated for the maintainer allele allow screening for or the identification of the non-restorer phenotype (i.e. the restorer locus at maize chromosome is not present). It will be understood that for identification of the non-restorer allele the indicated SNP nucleotides may be different than those indicated in the Table (as long as these are different than the SNP nucleotides indicated for the restorer allele).

In an aspect, the invention relates to a method for identifying a plant or plant part, in particular a maize plant or plant part, comprising screening for the presence of or detecting or identifying one or more, two or more, or three or more of the following polymorphisms (positions correspond to the indicated positions of the respective SEQ ID NOs):

| SEQ ID NO: | Position B73 (AGPv4) | SEQ ID NO: | Position restorer | polymorphism | |
|---|---|---|---|---|---|
| | | | | maintainer | restorer |
| 1 | 35 | 2 | 35 | g | t |
| 1 | 404 | 2 | 404 | t | c |
| 1 | 444-452 | 2 | 443-444 | gggactttc | Deletion |
| 1 | 463 | 2 | 454 | c | t |
| 1 | 537 | 2 | 528 | g | c |
| 1 | 735 | 2 | 726 | g | a |
| 1 | 748-759 | 2 | 738-739 | tactttgtaaca | Deletion |
| 1 | 761 | 2 | 740 | t | a |
| 1 | 797 | 2 | 776 | a | g |
| 1 | 1048 | 2 | 1027 | g | t |
| 1 | 1056 | 2 | 1035 | a | c |
| 1 | 1065-1066 | 2 | 1045 | tc | Insertion a |
| 1 | 1072-1073 | 2 | 1053-1059 | cc | Insertion cttctcc |
| 1 | 1071 | 2 | 1058 | g | c |
| 1 | 1188 | 2 | 1175 | c | t |
| 1 | 1218 | 2 | 1208 | t | c |
| 1 | 1765 | 2 | 1757 | a | g |
| 1 | 1769 | 2 | 1761 | g | a |
| 1 | 1844 | 2 | 1836 | t | g |
| 1 | 1856 | 2 | 1848 | t | a |
| 1 | 2076 | 2 | 2068 | c | t |
| 1 | 2089 | 2 | 2081 | g | a |
| 1 | 2146 | 2 | 2138 | t | c |
| 1 | 2168-2169 | 2 | 2161 | tt | Insertion t |
| 1 | 2214 | 2 | 2207 | g | t |
| 1 | 2370 | 2 | 2362-2363 | c | deletion |
| 1 | 2582 | 2 | 2574 | c | g |
| 1 | 2632-2637 | 2 | 2624-2629 | tactgt | Substitution ccactg |
| 1 | 2641 | 2 | 2633 | a | t |
| 1 | 2643-2644 | 2 | 2635-2636 | cg | Substitution ac |
| 1 | 2696 | 2 | 2688 | t | c |
| 1 | 2738 | 2 | 2730 | c | a |
| 1 | 2843 | 2 | 2834-2835 | g | deletion |
| 1 | 2849 | 2 | 2840 | t | c |
| 1 | 2954-2955 | 2 | 2946-2947 | tt | Insertion tt |
| 1 | 3004 | 2 | 2997 | g | a |
| 1 | 3047 | 2 | 3040 | a | t |
| 1 | 3068 | 2 | 3061 | c | a |
| 1 | 3218 | 2 | 3211 | a | g |
| 5 | 486 | 6 | 486 | a | t |
| 5 | 611 | 6 | 611 | g | a |
| 5 | 638 | 6 | 638 | a | g |
| 5 | 689 | 6 | 689 | g | a |
| 5 | 812 | 6 | 812 | c | t |
| 5 | 865 | 6 | 865 | c | g |
| 5 | 901 | 6 | 901 | c | a |
| 5 | 988 | 6 | 988 | g | a |
| 5 | 1015 | 6 | 1015 | c | t |
| 5 | 1085 | 6 | 1085 | c | t |
| 5 | 1197 | 6 | 1197 | t | c |
| 5 | 1345 | 6 | 1345 | c | t |
| 5 | 1461 | 6 | 1461 | c | t |
| 5 | 1937 | 6 | 1937 | c | t |
| 5 | 1999 | 6 | 1999 | c | t |
| 5 | 2113-2115 | 6 | 2112-2113 | gca | Deletion |
| 5 | 2286 | 6 | 2283 | c | t |
| 5 | 2293-2297 | 6 | 2289-2290 | ctacg | Deletion |
| 5 | 2399 | 6 | 2391 | c | g |
| 5 | 2448-2450 | 6 | 2439-2440 | ctc | Deletion |
| 5 | 2822-2823 | 6 | 2810-2811 | tt | Deletion |
| 5 | 2856 | 6 | 2843 | g | a |
| 5 | 2926 | 6 | 2913 | a | g |
| 5 | 2998 | 6 | 2985 | t | c |
| 5 | 3029 | 6 | 3016 | t | c |
| 5 | 3085 | 6 | 3072 | t | c |
| 5 | 3102 | 6 | 3089 | t | c |
| 5 | 3112 | 6 | 3099 | c | a |
| 5 | 3120 | 6 | 3107 | t | g |
| 5 | 3168 | 6 | 3155 | t | a |
| 9 | 392-393 | 10 | 393-397 | tg | Insertion gtggt |
| 9 | 550 | 10 | 555 | g | a |
| 9 | 591 | 10 | 596 | t | g |
| 9 | 886-887 | 10 | 891-892 | ct | tc |
| 9 | 934 | 10 | 939 | a | g |
| 9 | 957 | 10 | 962 | t | c |
| 9 | 1097 | 10 | 1102 | c | t |
| 9 | 1130 | 10 | 1135 | c | t |
| 9 | 1295 | 10 | 1300 | a | g |
| 9 | 1462 | 10 | 1467 | t | c |
| 9 | 1467 | 10 | 1472 | t | g |
| 9 | 1541 | 10 | 1546 | g | a |
| 9 | 1584 | 10 | 1603 | g | t |
| 9 | 1614 | 10 | 1633 | c | t |
| 9 | 1713 | 10 | 1732 | c | t |
| 9 | 1774 | 10 | 1793 | a | g |
| 9 | 1795-1796 | 10 | 1815-1823 | tt | Insertion tttttgttt |
| 9 | 1815 | 10 | 1843 | a | c |
| 9 | 1894-1895 | 10 | 1923 | tt | Insertion t |
| 9 | 1910 | 10 | 1939 | a | t |
| 9 | 1954-1955 | 10 | 1984-1991 | gt | Insertion tttgacac |
| 9 | 2000 | 10 | 2037 | t | c |
| 9 | 2060 | 10 | 2097 | t | c |
| 9 | 2181 | 10 | 2218 | c | t |
| 9 | 2354 | 10 | 2391 | a | g |
| 9 | 2372 | 10 | 2409 | a | t |
| 9 | 2394-2399 | 10 | 2431-2434 | ctgttt | Substitution/d eletion aaca |
| 9 | 2428 | 10 | 2463 | a | g |
| 9 | 2439-2440 | 10 | 2475-2476 | ct | Insertion tt |
| 13 | 651-652 | 14 | 652-654 | cc | Insertion cgc |
| 13 | 807 | 14 | 810 | t | c |

By means of example, the invention relates to a method for identifying a plant or plant part, in particular a maize plant or plant part, comprising screening for the presence of or detecting or identifying a polymorphism at a position corresponding to position 35 of SEQ ID NO: 1. By means of example, the invention relates to a method for identifying a plant or plant part, in particular a maize plant or plant part, comprising screening for the presence of or detecting or identifying a polymorphism at a position corresponding to position 35 of SEQ ID NO: 2.

By means of example, the invention relates to a method for identifying a plant or plant part, in particular a maize plant or plant part, comprising screening for the presence of or detecting or identifying a polymorphism at a position corresponding to position 35 of SEQ ID NO: 1, wherein if a G is detected said maize plant or plant part is a maintainer or is not a restorer or comprises a maintainer (gene(s), locus, haplotype, genome, or phenotype) or does not comprise a restorer (gene(s), locus, haplotype, genome, or phenotype), in particular the maintainer/restorer of the invention. By means of example, the invention relates to a method for identifying a plant or plant part, in particular a maize plant or plant part, comprising screening for the presence of or detecting or identifying a polymorphism at a position corresponding to position 35 of SEQ ID NO: 2, wherein if a T is detected said maize plant or plant part is restorer or comprises a restorer (gene(s), locus, haplotype, genome, or phenotype), in particular the restorer of the invention.

Corresponding embodiments apply to each of the other polymorphisms, SNPs, insertions, deletions, and substitutions.

Screening for the respective polymorphisms can be achieved by means knows in the art, such as for instance KASP, as described herein elsewhere. KASP primers may for instance be developed to discriminate between the restorer and non-restorer/maintainer polymorphisms.

The methods for identifying a (maize) plant or plant part as described herein may comprise screening of a sample obtained from a (maize) plant or plant part, in particular a sample comprising genomic DNA of the (maize) plant or plant part. Accordingly, the method may comprise the step of obtaining a sample (comprising genomic DNA) from a (maize) plant or plant part, or providing a sample (comprising genomic DNA) obtained from a (maize) plant or plant part. Methods for screening or identifying markers are well known in the art, as also described herein elsewhere.

It will be understood that the methods for identifying a (maize) plant or plant part as described herein allow for discriminating between plants or plant parts having a cytoplasmic male sterility restorer or a cytoplasmic male sterility maintainer genotype, haplotype, and/or phenotype based on the identity of the polymorphisms or polymorphic alleles described herein. Accordingly, the molecular marker(s) (allele(s)) of the present invention can be advantageously used to identify maize plants as being a restorer or having a restorer gene, locus (allele), haplotype, genotype or phenotype or as not being a restorer or not having a restorer gene, locus (allele), haplotype, genotype or phenotype, in particular the restorer of the invention. As also described herein elsewhere, such plants or plant parts may nevertheless comprise other restorer genes or loci.

Underlying the present invention is the identification of a CMS (cytoplasmic male sterility) restorer locus, in particular located on maize chromosome 3. Accordingly, the methods for identifying plants or plant parts as described herein can be methods for identifying plants or plant parts comprising said CMS restorer locus or alternatively methods for identifying plants or plant parts not comprising said CMS restorer locus. Such identification can be based on the polymorphisms described herein, in particular the polymorphic alleles associated with/linked with the restorer locus or alternatively the polymorphic alleles associated with/linked with the maintainer locus. Accordingly, the methods for identifying plants or plant parts as described herein can be methods for identifying plants or plant parts comprising the CMS maintainer locus or alternatively methods for identifying plants or plant parts not comprising the CMS maintainer locus.

In certain embodiments, the restorer locus of the invention is comprised on maize chromosome 3 in a genomic interval corresponding to (nucleic acid) position 195629901 to 198023573 of the B73 reference maize genome AGPv4, or a fragment thereof. In certain embodiments, the restorer locus comprises on maize chromosome 3 a genomic interval corresponding to (nucleic acid) position 195629901 to 198023573 of the B73 reference maize genome AGPv4, or a fragment thereof. In certain embodiments, the restorer locus is flanked on maize chromosome 3 by (nucleic acid) positions corresponding to positions 195629901 and 198023573 of the B73 reference maize genome AGPv4, or a fragment thereof.

In certain embodiments, the restorer locus of the invention is comprised on maize chromosome 3 in a genomic interval corresponding to (nucleic acid) position 197453646 to 197698278 of the B73 reference maize genome AGPv4, or a fragment thereof. In certain embodiments, the restorer locus comprises on maize chromosome 3 a genomic interval corresponding to (nucleic acid) position 197453646 to 197698278 of the B73 reference maize genome AGPv4, or a fragment thereof. In certain embodiments, the restorer locus is flanked on maize chromosome 3 by (nucleic acid) positions corresponding to positions 197453646 and 197698278 of the B73 reference maize genome AGPv4, or a fragment thereof.

In certain embodiments, the restorer locus of the invention is comprised on maize chromosome 3 in a genomic interval corresponding to (nucleic acid) position 195629901 to 197698278 of the B73 reference maize genome AGPv4, or a fragment thereof. In certain embodiments, the restorer locus comprises on maize chromosome 3 a genomic interval corresponding to (nucleic acid) position 195629901 to 197698278 of the B73 reference maize genome AGPv4, or a fragment thereof. In certain embodiments, the restorer locus is flanked on maize chromosome 3 by (nucleic acid) positions corresponding to positions 195629901 and 197698278 of the B73 reference maize genome AGPv4, or a fragment thereof.

In certain embodiments, the restorer locus of the invention is comprised on maize chromosome 3 in a genomic interval corresponding to (nucleic acid) position 197453646 to 198023573 of the B73 reference maize genome AGPv4, or a fragment thereof. In certain embodiments, the restorer locus comprises on maize chromosome 3 a genomic interval corresponding to (nucleic acid) position 197453646 to 198023573 of the B73 reference maize genome AGPv4, or a fragment thereof. In certain embodiments, the restorer locus is flanked on maize chromosome 3 by (nucleic acid) positions corresponding to positions 197453646 and 198023573 of the B73 reference maize genome AGPv4, or a fragment thereof.

In certain embodiments, the molecular marker(s) (allele(s)) are selected from Table 4 or 5. As indicated in Tables 4 and 5, all markers are polymorphic and are capable of discriminating between the restorer and non-restorer (or maintainer). Accordingly, identification of a restorer entails identification of one or more restorer-associated/linked polymorphisms as indicated in Tables 4 and 5, whereas identification of a non-restorer/maintainer entails identification of one or more non-restorer/maintainer-associated/linked polymorphisms as indicated in Tables 4 and 5.

As referred to herein, a polynucleic acid or locus of the invention as described herein, is said to be flanked by certain molecular markers or molecular marker alleles if the polynucleic acid/locus is comprised within a polynucleic acid wherein respectively a first marker (allele) is located upstream (i.e. 5') of said polynucleic acid and a second marker (allele) is located downstream (i.e. 3') of said polynucleic acid. Such first and second marker (allele) may border the polynucleic acid. The nucleic acid may equally comprise such first and second marker (allele), such as respectively at or near the 5' and 3' end, for instance respectively within 50 kb of the 5' and 3' end, preferably within 10 kb of the 5' and 3' end, such as within 5 kb of the 5' and 3' end, within 1 kb of the 5' and 3' end, or less.

In certain embodiments, the methods of the invention for identifying a maize plant or plant part comprise screening for the presence of or detecting or identifying one or more molecular marker (allele) selected from SEQ ID Nos: 17 to 200, wherein n is the corresponding nucleotide for the restorer polymorphism (SNP) (as indicated in Table 4).

In certain embodiments, the methods of the invention for identifying a maize plant or plant part comprise screening for the presence of or detecting or identifying one or more molecular marker (allele) selected from SEQ ID Nos: 17 to 200, wherein n is the corresponding nucleotide for the non-restorer/maintainer polymorphism (SNP) (as indicated in Table 4).

In certain embodiments, the methods of the invention for identifying a maize plant or plant part comprise screening for the absence of one or more molecular marker (allele) selected from SEQ ID Nos: 17 to 200, wherein n is the corresponding nucleotide for the restorer polymorphism (SNP) (as indicated in Table 4).

In certain embodiments, the methods of the invention for identifying a maize plant or plant part comprise screening for the absence of one or more molecular marker (allele) selected from SEQ ID Nos: 17 to 200, wherein n is the corresponding nucleotide for the non-restorer/maintainer polymorphism (SNP) (as indicated in Table 4).

In certain embodiments, the methods of the invention for identifying a maize plant or plant part comprise screening for the presence of or detecting or identifying one or more molecular marker (allele) selected from SEQ ID Nos: 68 to 140, wherein n is the corresponding nucleotide for the restorer polymorphism (SNP) (as indicated in Table 4).

In certain embodiments, the methods of the invention for identifying a maize plant or plant part comprise screening for the presence of or detecting or identifying one or more molecular marker (allele) selected from SEQ ID Nos: 68 to 140, wherein n is the corresponding nucleotide for the non-restorer/maintainer polymorphism (SNP) (as indicated in Table 4).

In certain embodiments, the methods of the invention for identifying a maize plant or plant part comprise screening for the absence of one or more molecular marker (allele) selected from SEQ ID Nos: 68 to 140, wherein n is the corresponding nucleotide for the restorer polymorphism (SNP) (as indicated in Table 4).

In certain embodiments, the methods of the invention for identifying a maize plant or plant part comprise screening for the absence of one or more molecular marker (allele) selected from SEQ ID Nos: 68 to 140, wherein n is the corresponding nucleotide for the non-restorer/maintainer polymorphism (SNP) (as indicated in Table 4).

In certain embodiments, the methods of the invention for identifying a maize plant or plant part comprise screening for the presence of or detecting or identifying one or more molecular marker (allele) selected from SEQ ID Nos: 70, 72, 76, 79, 86, 88, 92, 93, 99, 104, 105, 107, 108, 109, 115, and 134, wherein n is the corresponding nucleotide for the restorer polymorphism (SNP) (as indicated in Table 4).

In certain embodiments, the methods of the invention for identifying a maize plant or plant part comprise screening for the presence of or detecting or identifying one or more molecular marker (allele) selected from SEQ ID Nos: 70, 72, 76, 79, 86, 88, 92, 93, 99, 104, 105, 107, 108, 109, 115, and 134, wherein n is the corresponding nucleotide for the non-restorer/maintainer polymorphism (SNP) (as indicated in Table 4).

In certain embodiments, the methods of the invention for identifying a maize plant or plant part comprise screening for the absence of one or more molecular marker (allele) selected from SEQ ID Nos: 70, 72, 76, 79, 86, 88, 92, 93, 99, 104, 105, 107, 108, 109, 115, and 134, wherein n is the corresponding nucleotide for the restorer polymorphism (SNP) (as indicated in Table 4).

In certain embodiments, the methods of the invention for identifying a maize plant or plant part comprise screening for the absence of one or more molecular marker (allele) selected from SEQ ID Nos: 70, 72, 76, 79, 86, 88, 92, 93, 99, 104, 105, 107, 108, 109, 115, and 134, wherein n is the corresponding nucleotide for the non-restorer/maintainer polymorphism (SNP) (as indicated in Table 4).

In certain embodiments, the methods of the invention for identifying a maize plant or plant part comprise screening for the presence of or detecting or identifying any one or more of SEQ ID NOs: 1, 5, 9, and 13, or a (unique) fragment thereof, or one or more of SEQ ID NOs: 2, 6, 10, and 14, or a (unique) fragment thereof. In certain embodiments, the methods of the invention for identifying a maize plant or plant part comprise screening for the presence of or detecting or identifying any one or more of SEQ ID NOs: 1, 5, 9, and 13, or a (unique) fragment thereof, wherein the presence indicates that the plant or plant part is a restorer or comprises a restorer (gene(s), locus (allele), haplotype, genome, or phenotype). In certain embodiments, the methods of the invention for identifying a maize plant or plant part comprise screening for the presence of or detecting or identifying any one or more of SEQ ID NOs: 1, 5, 9, and 13, or a (unique) fragment thereof, wherein the absence indicates that the plant or plant part is a not a restorer or does not comprise a restorer (gene(s), locus (allele), haplotype, genome, or phenotype). In certain embodiments, the methods of the invention for identifying a maize plant or plant part comprise screening for the presence of or detecting or identifying any one or more of SEQ ID NOs: 1, 5, 9, and 13, or a (unique) fragment thereof, wherein the absence indicates that the plant or plant part is a maintainer or comprises a maintainer (gene(s), locus (allele), haplotype, genome, or phenotype).

In certain embodiments, the methods of the invention for identifying a maize plant or plant part comprise screening for the presence of or detecting or identifying any one or more of SEQ ID NOs: 2, 6, 10, and 14, or a (unique) fragment thereof, wherein the presence indicates that the plant or plant part is not a restorer or does not comprise a restorer (gene(s), locus (allele), haplotype, genome, or phenotype). In certain embodiments, the methods of the invention for identifying a maize plant or plant part comprise screening for the presence of or detecting or identifying any one or more of SEQ ID NOs: 2, 6, 10, and 14, or a (unique) fragment thereof, wherein the presence indicates that the plant or plant part is a maintainer or comprises a maintainer (gene(s), locus (allele), haplotype, genome, or phenotype). In certain embodiments, the methods of the invention for identifying a maize plant or plant part comprise screening for the presence of or detecting or identifying any one or more of SEQ ID NOs: 2, 6, 10, and 14, or a (unique) fragment thereof, wherein the absence indicates that the plant or plant part is a restorer or comprises a restorer (gene(s), locus (allele), haplotype, genome, or phenotype).

In an aspect, the invention relates to an (isolated) polynucleic acid comprising any one or more of the sequences, molecular markers or molecular marker alleles as described herein elsewhere, or a fragment thereof, and/or the complement thereof or the reverse complement thereof.

In certain embodiments, the polynucleotide or polynucleic acid according to the invention as described herein is an isolated polynucleotide or polynucleic acid.

In an aspect, the invention relates to an (isolated) polynucleic acid comprising a (unique) fragment of any of the sequences, molecular markers or molecular marker alleles as described herein elsewhere, or the complement thereof or the reverse complement thereof. Preferably said polynucleic acid is at least 15 nucleotides, more preferably at least 20 nucleotides, such as at least 25, 30, 35, 40, 45, 50, 75, 100, 125, 150, 175, 200 or more nucleotides. In certain embodiments, the polynucleic acid is at most 500 nucleotides, preferably at most 250 nucleotides, such as at most 200, 150, 100, or 50 nucleotides. In certain embodiments, the polynucleic acid has from 15 to 500 nucleotides, such as from 20 to 250 nucleotides or from 20 to 100 nucleotides, such as from 20 to 50 nucleotides.

In an aspect, the invention relates to an (isolated) polynucleic acid (specifically) hybridizing with any of the sequences, molecular markers or molecular marker alleles as described herein elsewhere, or the complement thereof or the reverse complement thereof. Preferably said polynucleic acid is at least 15 nucleotides, more preferably at least 20 nucleotides, such as at least 25, 30, 35, 40, 45, 50, 75, 100, 125, 150, 175, 200 or more nucleotides. In certain embodiments, the polynucleic acid is at most 500 nucleotides, preferably at most 250 nucleotides, such as at most 200, 150, 100, or 50 nucleotides. In certain embodiments, the polynucleic acid has from 15 to 500 nucleotides, such as from 20 to 250 nucleotides or from 20 to 100 nucleotides, such as from 20 to 50 nucleotides.

In an aspect, the invention relates to a polynucleic acid comprising a molecular marker (allele) of Table 4 or Table 5, or a (unique) fragment thereof, and/or the complement or reverse complement thereof. In an aspect, the invention relates to a polynucleic acid comprising a restorer molecular marker (allele) of Table 4 or Table 5, or a (unique) fragment thereof, and/or the complement or reverse complement thereof. In an aspect, the invention relates to a polynucleic acid comprising a non-restorer/maintainer molecular marker (allele) of Table 4 or Table 5, or a (unique) fragment thereof, and/or the complement or reverse complement thereof.

According to certain embodiments, when reference is made to a fragment of a polynucleic acid or protein, such fragment comprises respectively at least 15 nucleotides or amino acids, preferably at least 20 nucleotides or amino acids.

It will be understood that the polynucleic acids according to the invention comprises or specifically hybridizes with one or more of the molecular marker (allele) and additional 5' and/or 3' contiguous nucleotides (naturally) flanking the respective marker (allele) (or the complement or reverse complement thereof). In this context, the amount of flanking may in certain embodiments be at least 14 or 15 nucleotides (which may or may not be entirely 5' or entirely 3' flanking nucleotides, such as for instance 5 3' flanking nucleotides plus 10 5' flanking nucleotides. In certain embodiments, the molecular marker (allele) of the present invention (or the complement thereof) is the most 5' nucleotide of the polynucleic acid. In certain embodiments, the molecular marker (allele) of the present invention (or the complement thereof) is the second most 5' nucleotide of the polynucleic acid. In certain embodiments, the molecular marker (allele) of the present invention (or the complement thereof) is the third most 5' nucleotide of the polynucleic acid. In certain embodiments, the molecular marker (allele) of the present invention (or the complement thereof) is the most 3' nucleotide of the polynucleic acid. In certain embodiments, the molecular marker (allele) of the present invention (or the complement thereof) is the second most 3' nucleotide of the polynucleic acid. In certain embodiments, the molecular marker (allele) of the present invention (or the complement thereof) is the third most 3' nucleotide of the polynucleic acid. Such terminally located markers (such as SNPs) advantageously allow for the development of allele specific primers, such as for use in KASP.

In an aspect, the invention relates to a polynucleic acid comprising or comprised in any of SEQ ID NOs: 1 to 208, or a (unique) fragment thereof, and/or the complement thereof or the reverse complement thereof. In an aspect, the invention relates to a polynucleic acid specifically hybridizing with a polynucleic acid comprising or comprised in any of SEQ ID NOs: 1 to 208, or a (unique) fragment thereof, and/or the complement thereof or the reverse complement thereof. It will be understood that such polynucleic acids comprise at least one or more of the polymorphic nucleotides, insertions, deletions, or substitutions of the invention as referred to herein elsewhere and contiguous 5' and/or 3" flanking sequences, as described herein elsewhere.

In certain embodiments, the polynucleic acid comprises or is comprised in any of SEQ ID NOs: 17 to 200, wherein n is the corresponding nucleotide for the restorer polymorphism (SNP) (as indicated in Table 4).

In certain embodiments, the polynucleic acid comprises or is comprised in any of SEQ ID Nos: 17 to 200, wherein n is the corresponding nucleotide for the non-restorer/maintainer polymorphism (SNP) (as indicated in Table 4).

In certain embodiments, the polynucleic acid comprises or is comprised in any of SEQ ID Nos: 68 to 140, wherein n is the corresponding nucleotide for the restorer polymorphism (SNP) (as indicated in Table 4).

In certain embodiments, the polynucleic acid comprises or is comprised in any of SEQ ID Nos: 68 to 140, wherein n is the corresponding nucleotide for the non-restorer/maintainer polymorphism (SNP) (as indicated in Table 4).

In certain embodiments, the polynucleic acid comprises or is comprised in any of SEQ ID Nos: 70, 72, 76, 79, 86, 88, 92, 93, 99, 104, 105, 107, 108, 109, 115, and 134, wherein n is the corresponding nucleotide for the restorer polymorphism (SNP) (as indicated in Table 4).

In certain embodiments, the polynucleic acid comprises or is comprised in any of SEQ ID Nos: 70, 72, 76, 79, 86, 88, 92, 93, 99, 104, 105, 107, 108, 109, 115, and 134, wherein n is the corresponding nucleotide for the non-restorer/maintainer polymorphism (SNP) (as indicated in Table 4).

In certain embodiments, the polynucleic acid comprises or is comprised in any of SEQ ID NOs: 1-3, 5-7, 9-11, 13-15, 201, 203, 205, or 207, or the complement thereof, or the reverse complement thereof, or a (unique) fragment thereof. In certain embodiments, the polynucleic acid comprises or is comprised in a polynucleic acid encoding a protein of SEQ ID NOs: 4, 8, 12, 16, 202, 204, 206, 208, or the complement thereof, or the reverse complement thereof, or a (unique) fragment thereof. In certain embodiments, the polynucleic acid comprises or is comprised in any of SEQ ID NOs: 1, 5, 9, 13, 201, 203, 205, or 207, preferably SEQ ID NO: 1 or 201, or the complement thereof, or the reverse complement thereof, or a (unique) fragment thereof. In certain embodiments, the polynucleic acid comprises or is comprised in a polynucleic acid encoding a protein of SEQ ID NOs: 202, 204, 206, 208, preferably SEQ ID NO: 202, or the complement thereof, or the reverse complement thereof, or a (unique) fragment thereof. In certain embodiments, the polynucleic acid comprises or is comprised in any of SEQ ID NOs: 2-3, 6-7, 10-11, 14-15, preferably SEQ ID NO: 2, or the complement thereof, or the reverse complement thereof, or a (unique) fragment thereof. In certain embodiments, the polynucleic acid comprises or is comprised in a polynucleic acid encoding a protein of SEQ ID NOs: 4, 8, 12, 16, preferably SEQ ID NO: 4, or the complement thereof, or the reverse complement thereof, or a (unique) fragment thereof. Such polynucleic acids are suitable for identifying plants or plant parts as well as for generating plants as described herein elsewhere.

In certain embodiments, the polynucleic acid comprises at least 15 nucleotides, such as 16, 17, 18, 19, 20, 21, 22, 23, 24, or 25 nucleotides, such as at least 30, 35, 40, 45, or 50 nucleotides, such as at least 100, 200, 300, or 500 nucleotides.

In certain embodiments, the polynucleic acid comprises or consists of a polynucleic acid as defined in numbered statements 27 to 30, referred to herein elsewhere.

In certain embodiments, the polynucleic acid comprises at most 1500 nucleotides, such as 1200, 1000, 800, 600, 400, 200 nucleotides, such as at most 100, 80, 60, 50, 40, or 30 nucleotides.

In certain embodiments, the polynucleic acid comprises at least 15 nucleotides, such as 16, 17, 18, 19, 20, 21, 22, 23, 24, or 25 nucleotides, such as at least 30, 35, 40, 45, or 50 nucleotides, such as at least 100, 200, 300, or 500 nucleotides, and the polynucleic acid comprises at most 1500 nucleotides, such as 1200, 1000, 800, 600, 400, 200 nucleotides, such as at most 100, 80, 60, 50, 40, or 30 nucleotides.

In certain embodiments, the (isolated) polynucleotide has a length ranging from 15 to 500 nucleotides, preferably 15 to 100 nucleotides, preferably 15 to 50 nucleotides, more preferably 15 to 35 nucleotides.

In certain embodiments, the (isolated) polynucleotide is a primer or a probe.

In certain embodiments, the (isolated) polynucleotide is an allele-specific primer or probe.

In certain embodiments, the (isolated) is a KASP (Kompetitive allele specific PCR) primer. Primers, including KASP primers, are well-known in the art and can be designed by the skilled person according to known criteria. By means of further guidance, and without limitation, KASP is performed with two (or more) allele-specific primers (which may be the forward primers) and generally one common primer (which may be the reverse primer). The allele-specific primers are typically elongated with tail sequences (in which a different tail sequence is provided for each allele-specific primer). The tail sequences allow incorporation of a fluorescently labelled complementary sequence, to thereby fluorescently distinguish the different alleles.

In certain embodiments, the length of the tail sequence is comprised in the total primer length. In certain embodiments, the length of the tail sequence is not comprised in the total primer length.

In certain embodiments, the polynucleic acid is a (PCR) primer or (hybridization) probe. In certain embodiments, the polynucleic acid is an allele-specific primer or probe. In certain embodiments, the polynucleic acid is a KASP primer.

In an aspect, the invention relates to a (isolated) polynucleic acid comprising a (molecular) marker (allele) of the invention, or the complement or the reverse complement of a (molecular) marker (allele) of the invention. In certain embodiments, the invention relates to a polynucleic acid comprising at least 10 contiguous nucleotides, preferably at least 15 contiguous nucleotides or at least 20 contiguous nucleotides of a (molecular) marker (allele) of the invention, or the complement or the reverse complement of a (molecular) marker (allele) of the invention. In certain embodiments, the polynucleic acid is capable of discriminating between a (molecular) marker (allele) of the invention and a non-molecular marker allele, such as to specifically hybridise with a (molecular) marker allele of the invention. In certain embodiments, the polynucleic acid or the complement or reverse complement thereof does not (substantially) hybridise with or bind to (genomic) DNA originating from maize inbred line B73. In certain embodiments, the sequence of the polynucleic acid or the complement or reverse complement thereof does not occur or is not present in maize inbred line B73.

In an aspect, the invention relates to a kit comprising one or more of the polynucleotides as described herein, such as one or more of the primers or probes as described herein. The skilled person will understand that the polynucleotides may be comprised for instance in a single receptacle, such as a single vial, or in separate receptacles, such as separate vials.

It will be understood that "specifically hybridizing" means that the polynucleic acid hybridises with the (molecular) marker allele (such as under stringent hybridisation conditions, as defined herein elsewhere), but does not (substantially) hybridise with a polynucleic acid not comprising the marker allele or is (substantially) incapable of being used as a PCR primer. By means of example, in a suitable readout, the hybridization signal with the marker allele or PCR amplification of the marker allele is at least 5 times, preferably at least 10 times stronger or more than the hybridisation signal with a non-marker allele, or any other sequence.

In an aspect, the invention relates to a set of primers or probes as described above, such as a set of allele-specific primers or probes. In certain embodiments, the set may further comprise a (common) forward or reverse primer (depending on whether the allele-specific primers are reverse or forward primers).

In an aspect, the invention relates to a kit comprising such polynucleic acids, such as primers (comprising forward (such as one or more allele-specific or alternatively common primers) and/or reverse primers (such as a common or alternatively one or more allele-specific primers)) and/or probes (such as one or more allele-specific probe). The kit may further comprise instructions for use.

It will be understood that in embodiments relating to a set of forward and reverse primers, only one of both primers (forward or reverse) may need to be capable of discriminating between a (molecular) marker allele of the invention and a non-marker allele, and hence may be unique. The other primer may or may not be capable of discriminating between a (molecular) marker allele of the invention and a non-marker allele, and hence may or may not be unique.

In an aspect, the invention relates to a vector comprising a (isolated) polynucleic acid according to the invention as described herein. In certain embodiments, the vector is a (plant) expression vector. In certain embodiments, the vector is an inducible (plant) expression vector. In certain embodiments, the expression is tissue- or organ-specific. In certain embodiments, the expression is developmentally specific. In certain embodiments, the expression is tissue- or organ-specific and developmentally specific.

In certain embodiments, the vector comprises any of SEQ ID NOs: 1-3, 5-7, 9-11, 13-15, 201, 203, 205, or 207, or the complement thereof, or the reverse complement thereof, or a (unique) fragment thereof. In certain embodiments, the vector comprises a polynucleic acid encoding a protein of SEQ ID NOs: 4, 8,12,16, 202, 204, 206, 208, or the complement thereof, or the reverse complement thereof, or a (unique) fragment thereof. In certain embodiments, the vector comprises any of SEQ ID NOs: 1, 5, 9, 13, 201, 203, 205, or 207, preferably SEQ ID NO: 1 or 201, or the complement thereof, or the reverse complement thereof, or a (unique) fragment thereof. In certain embodiments, the vector comprises a polynucleic acid encoding a protein of SEQ ID NOs: 202, 204, 206, 208, preferably SEQ ID NO: 202, or the complement thereof, or the reverse complement thereof, or a (unique) fragment thereof. In certain embodiments, the vector comprises any of SEQ ID NOs: 2-3, 6-7, 10-11, 14-15, preferably SEQ ID NO: 2, or the complement thereof, or the reverse complement thereof, or a (unique) fragment thereof. In certain embodiments, the vector comprises a polynucleic acid encoding a protein of SEQ ID NOs: 4, 8, 12, 16, preferably SEQ ID NO: 4, or the complement thereof, or the reverse complement thereof, or a (unique) fragment thereof. Such polynucleic acids are suitable for identifying plants or plant parts as well as for generating plants as described herein elsewhere.

As used herein, a "vector" has its ordinary meaning in the art, and may for instance be a plasmid, a cosmid, a phage or an expression vector, a transformation vector, shuttle vector, or cloning vector; it may be double- or single-stranded, linear or circular; or it may transform a prokaryotic or eukaryotic host, either via integration into its genome or extrachromosomally. The nucleic acid according to the invention is preferably operatively linked in a vector with one or more regulatory sequences which allow the transcription, and, optionally, the expression, in a prokaryotic or eukaryotic host cell. A regulatory sequence - preferably DNA - may be homologous or heterologous to the nucleic acid according to the invention. For example, the nucleic acid is under the control of a suitable promoter or terminator. Suitable promoters may be promoters which are constitutively induced (example: 35S promoter from the "Cauliflower mosaic virus" (Odell et al., 1985); those promoters which are tissue-specific are especially suitable (example: Pollen-specific promoters, Chen et al. (2010), Zhao et al. (2006), or Twell et al. (1991)), or are development-specific (example: blossom-specific promoters). Suitable promoters may also be synthetic or chimeric promoters which do not occur in nature, are composed of multiple elements, and contain a minimal promoter, as well as - upstream of the minimum promoter - at least one cis-regulatory element which serves as a binding location for special transcription factors. Chimeric promoters may be designed according to the desired specifics and are induced or repressed via different factors. Examples of such promoters are found in Gurr & Rushton (2005) or Venter (2007). For example, a suitable terminator is the nos-terminator (Depicker et al., 1982). The vector may be introduced via conjugation, mobilization, biolistic transformation, agrobacteria-mediated transformation, transfection, transduction, vacuum infiltration, or electroporation. The vector may be a plasmid, a cosmid, a phage or an expression vector, a transformation vector, shuttle vector, or cloning vector; it may be double- or single-stranded, linear or circular. The vector may transform a prokaryotic or eukaryotic host, either via integration into its genome or extrachromosomally.

As used herein, the term "operatively linked" or "operably linked" means connected in a common nucleic acid molecule in such a manner that the connected elements are positioned and oriented relative to one another such that a transcription of the nucleic acid molecule may occur. A DNA which is operatively linked with a promoter is under the transcriptional control of this promoter.

In an aspect, the invention relates to the use of the polynucleic acid or vector according to the invention as described herein for generating a maize plant or plant part, such as a maize plant or plant part having increased pathogen resistance and/or tolerance.

In certain embodiments, the vector is an expression vector. The nucleic acid is preferably operatively linked in a vector with one or more regulatory sequences which allow the transcription, and optionally the expression, in a prokaryotic or eukaryotic host cell. A regulatory sequence may be homologous or heterologous to the nucleic acid. For example, the nucleic acid is under the control of a suitable promoter or terminator. Suitable promoters may be promoters which are constitutively induced, for example, the 35S promoter from the "Cauliflower mosaic virus" (Odell et al., 1985. Identification of DNA sequences required for activity of the cauliflower mosaic virus 35S promoter.) Tissue-specific promoters, e.g. pollen-specific promoters as described in Chen et al. (2010. Molecular Biology Reports 37(2):737-744), Zhao et al. (2006. Planta 224(2): 405-412), or Twell et al. (1991. Genes & Development 5(3): 496-507), are particularly suitable, as are development-specific promoters, e.g. blossom-specific promoters. Suitable promoters may also be synthetic or chimeric promoters which do not occur in nature, and which are composed of multiple elements. Such synthetic or chimeric promoter may contain a minimal promoter, as well as at least one cis-regulatory element which serves as a binding location for special transcription factors. Chimeric promoters may be designed according to the desired specifics and can be induced or repressed via different factors. Examples of such promoters are found in Gurr & Rushton (2005. Trends in Biotechnology 23(6): 275-282) or Venter (2007. Trends in Plant Science: 12(3):, 118-124). For example, a suitable terminator is the nos-terminator (Depicker et al., 1982. Journal of Molecular and Applied Genetics 1(6): 561-573).

In certain embodiments, the vector is a conditional expression vector. In certain embodiments, the vector is a constitutive expression vector. In certain embodiments, the vector is a tissue-specific expression vector, such as a pollen-specific expression vector. In certain embodiments, the vector is an inducible expression vector. All such vectors are well-known in the art. Methods for preparation of the described vectors are commonplace to the person skilled in the art (Sambrook et al., 2001).

Also envisaged herein is a host cell, such as a plant cell, which comprises a nucleic acid as described herein, preferably an induction-promoting nucleic acid or a nucleic acid encoding a double-stranded RNA as described herein, or a vector as described herein. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

The host cell may be a prokaryotic (for example, bacterial) or eukaryotic cell (for example, a plant cell or a yeast cell). For example, the host cell may be an agrobacterium, such as *Agrobacterium tumefaciens* or *Agrobacterium rhizogenes.* Preferably, the host cell is a plant cell.

In an aspect, the invention relates to the use of the polynucleic acid or vector according to the invention as described herein for identifying a maize plant or plant part, such as a maize plant or plant part having increased pathogen resistance and/or tolerance.

A nucleic acid described herein or a vector described herein may be introduced in a host cell via well-known methods, which may depend on the selected host cell, including, for example, conjugation, mobilization, biolistic transformation, agrobacteria-mediated transformation, transfection, transduction, vacuum infiltration, or electroporation. In particular, methods for introducing a nucleic acid or a vector in an agrobacterium cell are well-known to the skilled person and may include conjugation or electroporation methods. Also methods for introducing a nucleic acid or a vector into a plant cell are known (Sambrook et al., 2001) and may include diverse transformation methods such as biolistic transformation and agrobacterium-mediated transformation.

In particular embodiments, the present invention relates to a transgenic plant cell which comprises a nucleic acid as described herein, in particular an induction-promoting nucleic acid or a nucleic acid encoding a double-stranded RNA as described herein, as a transgene or a vector as described herein. In further embodiments, the present invention relates to a transgenic plant or a part thereof which comprises the transgenic plant cell.

For example, such a transgenic plant cell or transgenic plant is a plant cell or plant which is, preferably stably, transformed with a nucleic acid as described herein, in particular an induction-promoting nucleic acid or a nucleic acid encoding a double-stranded RNA as described herein, or a vector as described herein.

Preferably, the nucleic acid in the transgenic plant cell is operatively linked with one or more regulatory sequences which allow the transcription, and optionally the expression, in the plant cell. A regulatory sequence may be homologous or heterologous to the nucleic acid. The total structure made up of the nucleic acid according to the invention and the regulatory sequence(s) may then represent the transgene.

In an aspect, the invention relates to the use of one or more of the (molecular) marker (allele) described herein for identifying a plant or plant part having a fertility restorer (gene, locus, haplotype, genotype, or phenotype). In an aspect, the invention relates to the use of one or more of the (molecular) marker (allele) described herein which are able to detect at least one diagnostic marker allele for identifying a plant or plant part, such as having having a fertility restorer (gene, locus, haplotype, genotype, or phenotype). In an aspect, the invention relates to the detection of one or more of the (molecular) marker alleles described herein for identifying a plant or plant part having having a fertility restorer (gene, locus, haplotype, genotype, or phenotype).

In an aspect, the invention relates to a (maize) plant or plant part identified by the methods of the invention as described herein. In particular embodiments this includes plant material obtained from said plant or plant part.

In an aspect, the invention relates to a (maize) plant or plant part comprising one or more of the (molecular) markers (alleles), polynucleic acids, loci, or vectors of the invention as described herein.

In an aspect, the invention relates to a (maize) plant or plant part comprising one or more of the (molecular) marker (alleles) of Table 4 or 5.

In an aspect, the invention relates to a (maize) plant or plant part comprising one or more polynucleic acid of SEQ ID NO: 1 to 208. In an aspect, the invention relates to a (maize) plant or plant part comprising one or more polynucleic acid of SEQ ID NO: 17 to 200. In certain embodiments, the polynucleic acid of SEQ ID Nos: 17-200 corresponds to a polynucleic acid of a restorer of the invention. In certain embodiments, the polynucleic acid of SEQ ID Nos: 17-200 corresponds to a polynucleic acid of a non-restorer/maintainer of the invention. The skilled person will appreciate that the discrimination between restorer and non-restorer/maintainer can be made based on the identity of "n" in SEQ ID Nos 17-200, as also described herein elsewhere (such as for instance based on Table 4). In an aspect, the invention relates to a (maize) plant or plant part comprising one or more polynucleic acid of SEQ ID NO: 68 to 140. In an aspect, the invention relates to a (maize) plant or plant part comprising one or more polynucleic acid of SEQ ID NO: 70, 72, 76, 79, 86, 88, 92, 93, 99, 104, 105, 107, 108, 109, 115, and/or 134. In an aspect, the invention relates to a (maize) plant or plant part comprising one or more polynucleic acid of SEQ ID NO: 1, 5, 9, or 13 or one or more of SEQ ID NOs: 2, 6, 10, and 14. In an aspect, the invention relates to a (maize) plant or plant part comprising one or more polynucleic acid encoding Zm00001d043358, Zm00001d043352, Zm00001d043356, and Zm00001d043357, having respectively a coding sequence as set forth in any of SEQ ID NOs: 201, 203, 205, and 207, or having respectively a coding sequence as set forth in any of SEQ ID NOs: 3, 7, 11, and 15. In an aspect, the invention relates to a (maize) plant or plant part comprising one or more polypeptide having a sequence as set forth in any of SEQ ID NOs: 202, 204, 206, or 208, or having a sequence as set forth in any of SEQ ID NOs: 4, 8, 12, or 16. Preferably, the plant or plant part comprises a polynucleic acid encoding the polypeptide, which may be provided on a vector or may be genomically integrated.

In certain embodiments, the markers (alleles), polynucleic acids, or loci as defined herein are homozygous. Accordingly, in diploid plants the two alleles are identical (at least with respect to the particular marker (allele), polynucleic acid, or locus), in tetraploid plants the four alleles are identical, and in hexaploid plants the six alleles are identical with respect to the marker (allele), polynucleic acid, or locus. In certain embodiments, the marker (allele), polynucleic acid, or locus as defined herein is heterozygous. Accordingly, in diploid plants the two alleles are not identical, in tetraploid plants the four alleles are not identical (for instance only one, two, or three alleles comprise the specific marker (allele), polynucleic acid, or locus), and in hexaploid plants the six alleles are not identical with respect to the mutation or marker (for instance only one, two, three, four or five alleles comprise the specific marker (allele), polynucleic acid, or locus). Similar considerations apply in case of pseudopolyploid pants.

In an aspect, the invention relates to a method for generating a (maize) plant or plant part comprising introducing in (the genome of) said plant or plant part a polypeptide, polynucleic acid, locus (allele), or (molecular) marker (allele) of the invention as defined herein, or a (functional) fragment thereof. Preferably, introduction into the plant or plant part is genomic introduction. In certain embodiments however, introduction is non-genomic introduction, such as episomal introduction. In certain embodiments, introduction is achieved by means of a vector, as is known in the art and as also described herein elsewhere.

In an aspect, the invention relates to a method for generating a (maize) plant or plant part comprising introducing in (the genome of) said plant or plant part one or more polynucleic acid of SEQ ID NO: 1 to 208. In an aspect, the invention relates to a method for generating a (maize) plant or plant part comprising introducing in (the genome of) said plant or plant part one or more polynucleic acid of SEQ ID NO: 17 to 200. In certain embodiments, the polynucleic acid of SEQ ID Nos: 17-200 corresponds to a polynucleic acid of a restorer of the invention. In certain embodiments, the polynucleic acid of SEQ ID Nos: 17-200 corresponds to a polynucleic acid of a non-restorer/maintainer of the invention. The skilled person will appreciate that the discrimination between restorer and non-restorer/maintainer can be made based on the identity of "n" in SEQ ID Nos 17-200, as also described herein elsewhere (such as for instance based on Table 4). In an aspect, the invention relates to a method for generating a (maize) plant or plant part comprising introducing in (the genome of) said plant or plant part one or more polynucleic acid of SEQ ID NO: 68 to 140. In an aspect, the invention relates to a method for generating a (maize) plant or plant part comprising introducing in (the genome of) said plant or plant part one or more polynucleic acid of SEQ ID NO: 70, 72, 76, 79, 86, 88, 92, 93, 99, 104, 105, 107, 108, 109, 115, and/or 134. In an aspect, the invention relates to a method for generating a (maize) plant or plant part comprising introducing in (the genome of) said plant or plant part one or more polynucleic acid of SEQ ID NO: 1, 5, 9, or 13 or one or more of SEQ ID NOs: 2, 6, 10, and 14. In an aspect, the invention relates to a method for generating a (maize) plant or plant part comprising introducing in (the genome of) said plant or plant part one or more polynucleic acid encoding Zm00001d043358, Zm00001d043352, Zm00001d043356, and Zm00001d043357, having respectively a coding sequence as set forth in any of SEQ ID NOs: 201, 203, 205, and 207, or having respectively a coding sequence as set forth in any of SEQ ID NOs: 3, 7, 11, and 15. In an aspect, the invention relates to a method for generating a (maize) plant or plant part comprising introducing in said plant or plant part one or more polypeptide having a sequence as set forth in any of SEQ ID NOs: 202, 204, 206, or 208, or having a sequence as set forth in any of SEQ ID NOs: 4, 8, 12, or 16. Preferably, the plant or plant part comprises a polynucleic acid encoding the polypeptide, which may be provided on a vector or may be genomically integrated. In an aspect, the invention relates to a method for generating a (maize) plant or plant part comprising introducing in (the genome of) said plant or plant part one or more polynucleic acid encoding a polypeptide having a sequence as set forth in any of SEQ ID NOs: 202, 204, 206, or 208, or having a sequence as set forth in any of SEQ ID NOs: 4, 8, 12, or 16.

In certain embodiments, introducing (into the genome) as referred to herein comprises transgenesis.

In certain embodiments, introducing (into the genome) as referred to herein comprises transformation.

In certain embodiments, introducing (into the genome) as referred to herein comprises recombination, such as homologous recombination.

In certain embodiments, introducing (into the genome) as referred to herein comprises mutagenesis.

In certain embodiments, introducing (into the genome) as referred to herein comprises introgression. In certain embodiments, introducing into the genome as referred to herein does not comprise introgression.

In certain embodiments, introducing into the genome as referred to herein comprises introducing into the genome in a plant part. In certain embodiments, the plant part is a plant organ. In certain embodiments, the plant part is a plant tissue. In certain embodiments, the plant part is a plant cell. In certain embodiments, the plant part is a protoplast.

In certain embodiments, introducing into the genome as referred to herein comprises introducing into the genome in vitro. In certain embodiments, introducing into the genome as referred to herein comprises introducing into the genome in vivo.

In certain embodiments, the method for generating a maize plant or plant part comprises transforming a plant or plant part, preferably a plant cell, more preferably a protoplast, with a polynucleic acid as described herein elsewhere, and optionally regenerating a plant from said plant cell, preferably protoplast.

In certain embodiments, the transformed plant or plant part does not endogenously comprise the polynucleic acid according to the invention as described herein.

In certain embodiments, the transformed plant or plant part does not endogenously comprise the one or more molecular marker (alleles) according to the invention as described herein.

In certain embodiments, the methods for obtaining or generating plants or plant parts as described herein according to the invention involve or comprise transgenesis and/or gene editing, such as including CRISPR/Cas, TALEN, ZFN, meganucleases; (induced) mutagenesis, which may or may not be random mutagenesis, such as TILLING.

In certain embodiments, the methods for obtaining plants or plant parts as described herein according to the invention do not involve or comprise transgenesis, gene editing, and/or mutagenesis.

In certain embodiments, the methods for obtaining plants or plant parts as described herein according to the invention involve, comprise or consist of breeding and/or selection.

In certain embodiments, the methods for obtaining plants or plant parts as described herein according to the invention do not involve, comprise or consist of breeding.

In an aspect, the invention relates to a maize plant or plant part obtained or obtainable by the methods according to the invention as described herein, such as the methods for identifying a maize plant or plant part or the methods for generating a maize plant or plant part. The invention also relates to the progeny of such plants.

In an aspect, the invention relates to a maize plant or plant part comprising a polynucleic acid according to the invention as described herein. In certain embodiments, the polynucleic acid allele is homozygous. In certain embodiments, the polynucleic acid allele is heterozygous.

In an aspect, the invention relates to a maize plant or plant part comprising any one or more molecular marker (allele) according to the invention as described herein. In certain embodiments, the molecular marker (allele) is homozygous. In certain embodiments, the molecular marker (allele) allele is heterozygous.

In certain embodiments, the maize plant is not a maize variety. In certain embodiments, the plant is not exclusively obtained by means of an essentially biological process. In certain embodiments, the plant is obtained by a method which contains at least one step other than crossing, i.e. the screening for the presence of a polynucleotide as described herein.

As described herein elsewhere, in certain embodiments such (maize) plant or plant part does not comprise endogenously the recited polynucleic acids.

In certain embodiments, the maize plant or plant part is transgenic, gene-edited, or mutagenized. In certain embodiments, the maize plant or plant part is transgenic, gene-edited, or mutagenized in order to comprise the one or more molecular marker (allele), or one or more of the polynucleic acids according to the invention as described herein.

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny.

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny, wherein said first plant is a restorer (preferably a restorer of the present invention).

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny, wherein said second plant is a restorer (preferably a restorer of the present invention).

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny, wherein said first plant is a not a restorer (preferably not a restorer of the present invention).

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny, wherein said second plant is a not a restorer (preferably not a restorer of the present invention).

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny, wherein said first plant is a restorer (preferably a restorer of the present invention) and wherein said second plant is not a restorer (preferably not a restorer of the present invention).

In an aspect, the invention relates to a method for generating a (maize) plant or plant part, comprising (a) providing a first (maize) plant according to the invention or identified according to the invention or generated according to the invention, (b) crossing said first (maize) plant with a second maize plant having cytoplasmic male sterility; and optionally (d) harvesting said (maize) plant part from the progeny, wherein said first plant is a not a restorer (preferably not a restorer of the present invention) and wherein said second plant is a restorer (preferably a restorer of the present invention).

The aspects and embodiments of the invention are further supported by the following non-limiting examples. The following examples, including the experiments conducted and the results achieved, are provided for illustrative purposes only and are not constructed as limiting the present invention.

### EXAMPLES

### EXAMPLE 1: Identification of maize restorer genotype on chromosome 3

In the last years, a higher fraction of restoring lines in the female breeding pool 4 has been observed for CMSC, but only part of these lines carries the restorer RF4.

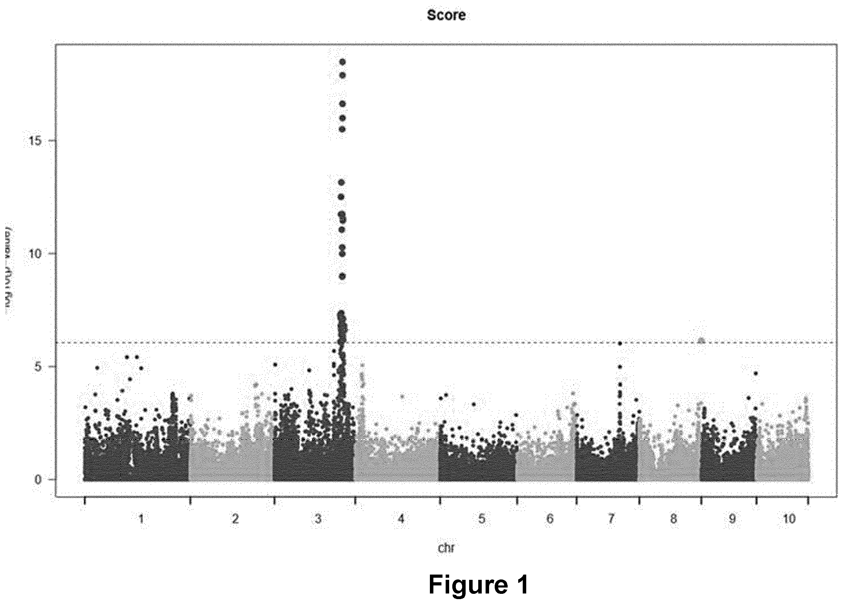

The present inventors tested 374 corn lines with phenotype of the pool 4 for the presence of RF4 und the respective restoration phenotype (Table 1). The data show that one or more other restorers seems to be present in the pool. A GWAS analysis using all pool 4 lines not carrying RF4 confirmed this assumption and showed a clear hit on chromosome 3 (Figure 1). The new restorer is named RF-03-01.

**Table 1: Analysis of presence/absence of RF4 restorer (RF4+/rf4-)**

| Haplotype | Maintainer | Restorer |
|---|---|---|
| RF4+ | 1 | 45 |
| rf4- | 174 | 93 |

Analysis of 600k data of 143 restoring and not restoring pool4 lines (all RF4 free) showed a genomic region of 0.25 Mb linked to restoration. Within the analyzed material, only two haplotypes were present, one of them explaining restoration. The haplotypes contain 64 polymorphic 600k markers (Table 4: identifiers 52, 53, 55, 57-71, 73-75, 78-88, 90, 94-124) which are in high linkage disequilibrium (LD). 14% of all pool 4 lines (including RF4 carriers) carry the RF-03-01 restorer haplotype.

**Table 2: Haplotype analysis**

| Haplotype | Maintainer | Restorer |
|---|---|---|
| A | 100 | 22 |
| B | 0 | 21 |

The restoring fraction of haplotype A can only be explained by further minor restorers which could not be identified until now.

The region on chromosome 3 contains 6 genes according the AGPv4 annotation https://www.maizegdb.org/genome/assembly/Zm-B73-REFERENCE-GRAMENE-4.0), 4 of them being expressed in pollen, one being involved in mitochondrial organization and thus representing the most prominent candidate gene (Zm00001d043358). All four genes are polymorphic between the two haplotypes.

**Table 3: List of candidate genes**

| Gene | Function | SEQ ID NO: | | | | | |
|---|---|---|---|---|---|---|---|
| | | Restorer haplotype | | | Reference B73 | | |
| | | genomic DNA | cDNA | protein | genomic DNA | cDNA | protein |
| Zm00001 d 043358 | mitochond rion organizati on | 1 | 201 | 202 | 2 | 3 | 4 |
| Zm00001 d 043352 | rRNA processin g | 5 | 203 | 204 | 6 | 7 | 8 |
| Zm00001 d 043356 | aromatic amino acid family biosyntheti c process | 9 | 205 | 206 | 10 | 11 | 12 |
| Zm00001 d 043357 | Plastocya nin-like, Electron carrier | 13 | 207 | 208 | 14 | 15 | 16 |

16 KASP markers (Table 4: identifier 54, 56, 60, 63, 70, 72, 76, 77, 83, 88, 89, 91, 92, 93, 99, and 118) were developed which can be used to detect the haplotype, partly by conversion of 600k markers, partly by using new SNPs within the candidate genes.

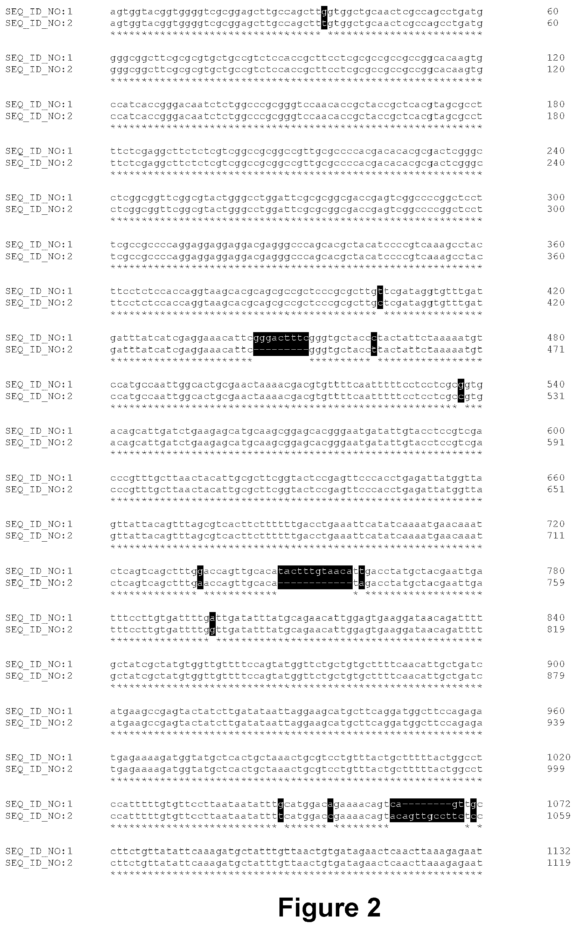

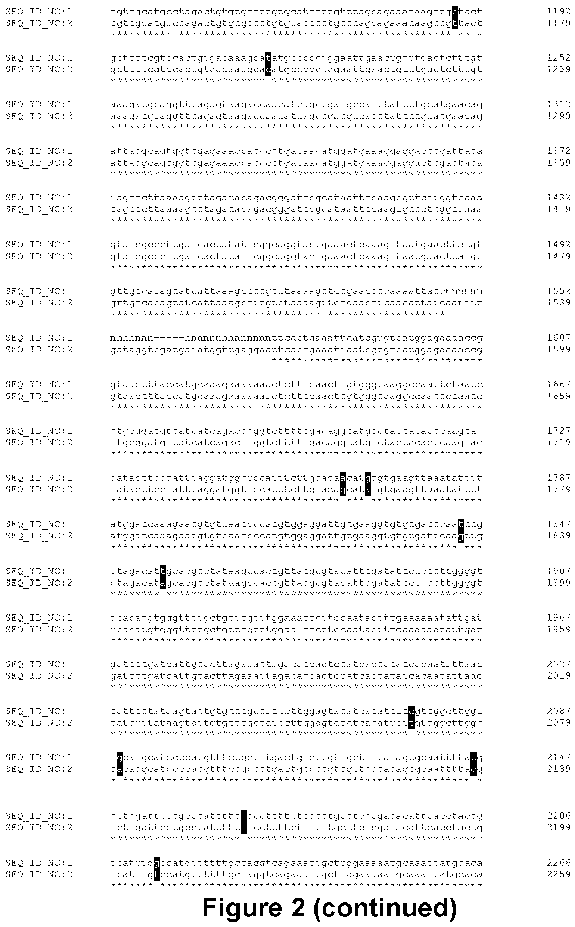

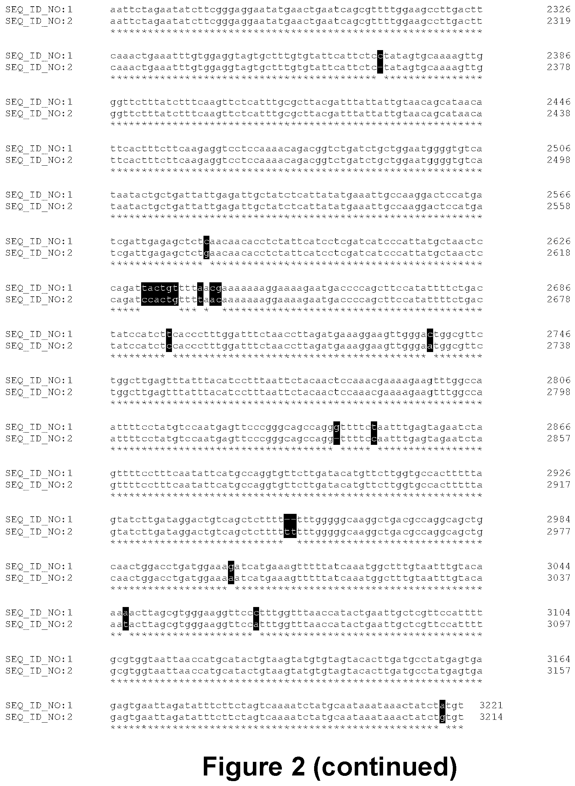

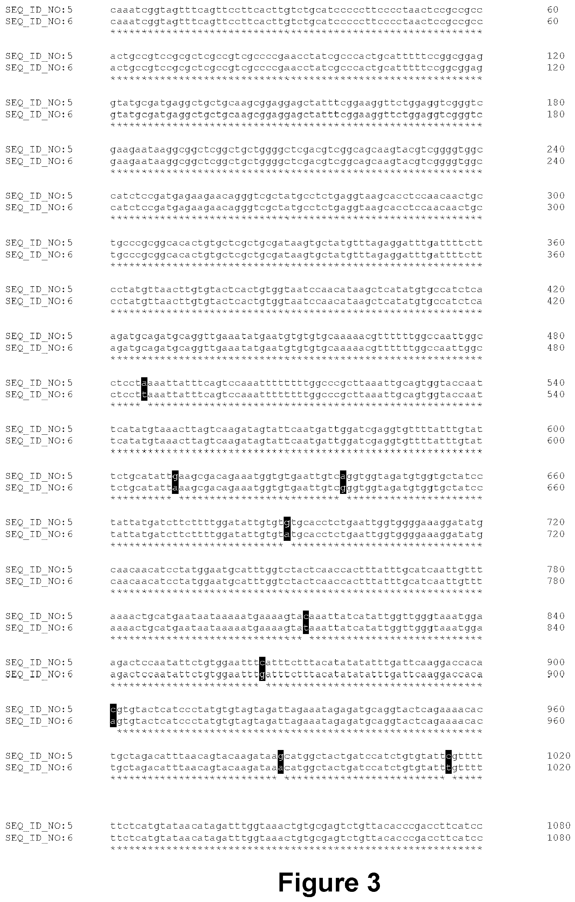

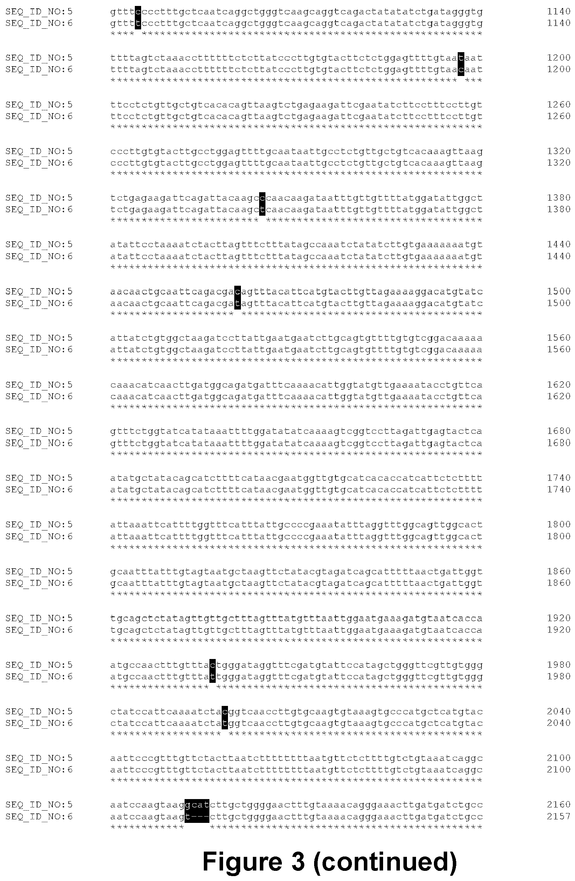

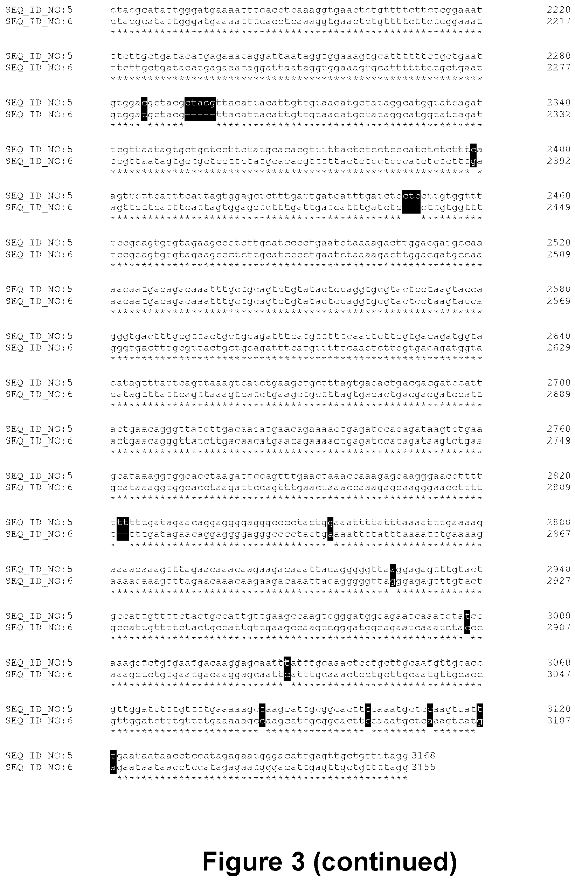

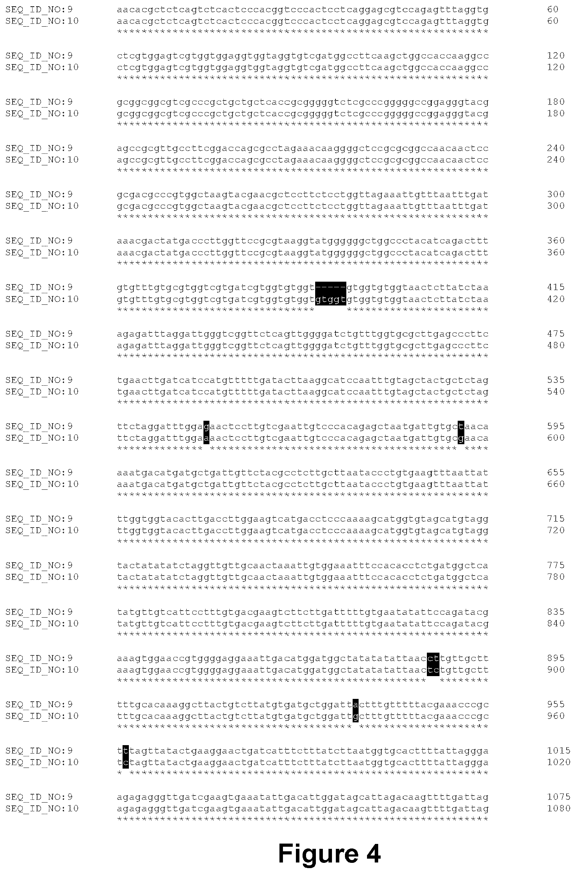

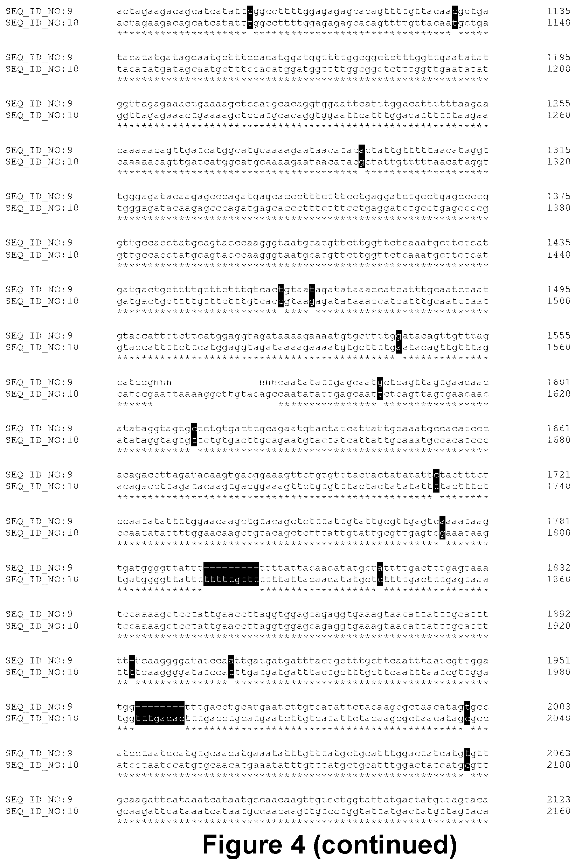

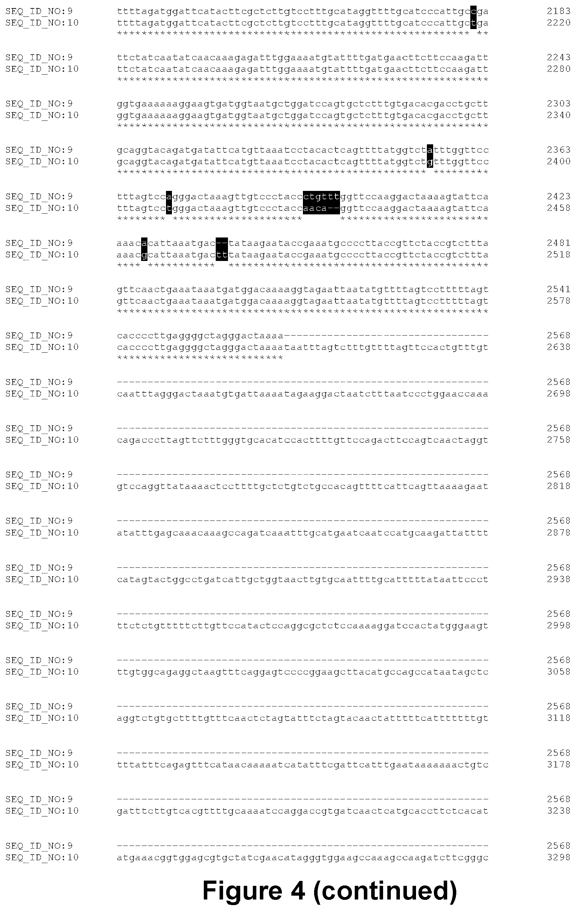

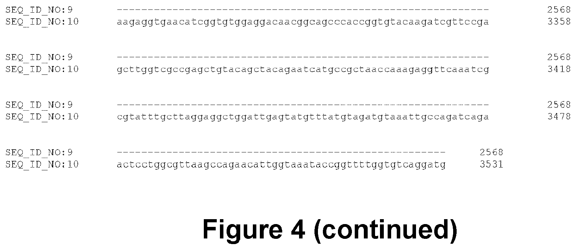

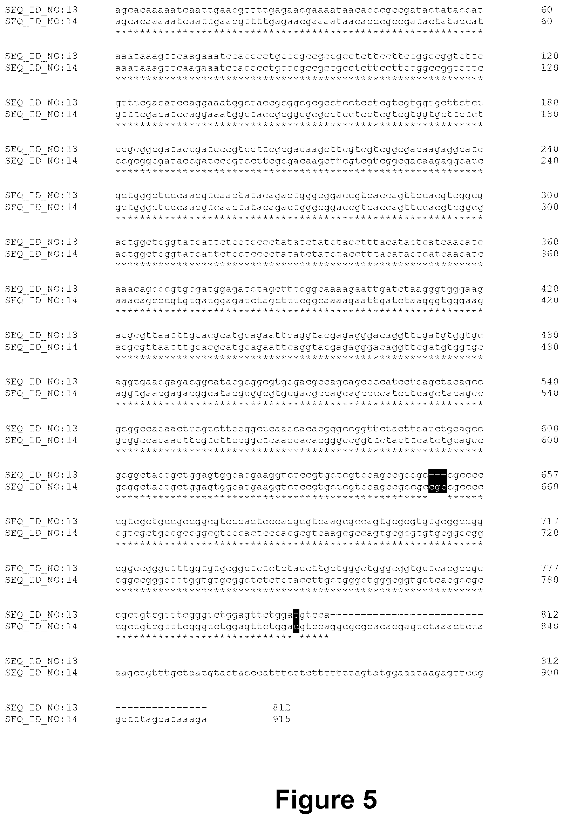

Using these markers in combination with known RF4 markers, it is possible to detect the most important restorers in pool 4 and to decide on the most reasonable conversion strategy for a given line in the process of development of hybrid corn lines. Generally all markers as listed in Table 4 can be used for this purpose. Further, Figs. 2-5 show sequence alignments of the genomic DNA of the candidate genes derived from the RF-03-01 restorer genotype and from a reference genotype (B73). Highlighted in black with white letters are polymorphisms which are additionally suitable to detect undesired restorer genotype. With regard to the list of markers according to Table 4 markers with the identifiers 52-124 showed to be 100% associated to the restorer locus on chromosome 3. Therefore, the region from position 197453646 to 197698278 referenced to B73 AGPv4 is most suitable as target site for marker-associated identification of RF-03-01 restorer genotype. Markers with identifiers 1-51 and 125-184 corresponding to regions from position 195629901 to 196989408 and from position 197708137 to 198023573 can also be used for identification, because the underlying polymorphisms can be found in most genotypes (major alleles), however it is not 100% linked.

RF-03-01 is also present in the flint pool used as male, although its impact to restoration is lower than in pool 4. Anyhow, also in this pool a good knowledge of restorer genes should be achieved, because restoring male lines are important for the usefulness of cms. In case only RF-03-01 is present, but not RF4, the restoration may be too weak or may fail in some environments, because this restorer is not as stable as RF4. Therefore, a good genotyping approach in addition to phenotyping is important to secure production.

**Table 4: List of markers; marker sequences can be found in sequence listing under respective SEQ ID NO as indicated in last column. Further, in the sequence listing under identifier <223> it is defined where in the marker sequence the polymorphism is located (number following the @ gives the position in the sequence). Position "n" thus corresponds to the polymorphism capable of discriminating between restorer and non-restorer/maintainer.**

| Identifier | Marker name | Position AGPv4 | polymorphism | | SEQ ID NO: |
|---|---|---|---|---|---|
| | | | maintainer | restorer | |
| 1 | ma0016fm86 | 195629901 | ade | gua | 17 |
| 2 | ma0016fn05 | 195639694 | thy | cyt | 18 |
| 3 | ma0016fn29 | 195677799 | gua | ade | 19 |
| 4 | ma0016fn27 | 195678356 | cyt | gua | 20 |
| 5 | ma0016fn16 | 195680790 | gua | ade | 21 |
| 6 | ma0004tk22 | 195732936 | gua | ade | 22 |
| 7 | ma0004tk05 | 195733916 | cyt | ade | 23 |
| 8 | ma0016fn55 | 195783701 | cyt | thy | 24 |
| 9 | ma0016fp29 | 196070086 | cyt | thy | 25 |
| 10 | ma0004tm29 | 196198736 | ade | gua | 26 |
| 11 | ma0004tm38 | 196244714 | thy | cyt | 27 |
| 12 | ma0016fq46 | 196653746 | cyt | thy | 28 |
| 13 | ma0000ba98 | 196693501 | cyt | ade | 29 |
| 14 | ma0000na46 | 196702811 | ade | gua | 30 |
| 15 | ma0001gr36 | 196704008 | ade | gua | 31 |
| 16 | ma0000kr37 | 196704096 | thy | cyt | 32 |
| 17 | ma0001gr37 | 196704169 | thy | cyt | 33 |
| 18 | ma0001gr41 | 196704290 | thy | cyt | 34 |
| 19 | ma0016fq61 | 196705470 | thy | ade | 35 |
| 20 | ma0022xj91 | 196706697 | gua | ade | 36 |
| 21 | ma0016fq64 | 196706755 | cyt | ade | 37 |
| 22 | ma0001jh28 | 196707190 | ade | gua | 38 |
| 23 | ma0004tn40 | 196707415 | thy | ade | 39 |
| 24 | ma0004tn32 | 196707997 | gua | gua | 40 |
| 25 | ma0016fq88 | 196773893 | cyt | thy | 41 |
| 26 | ma0004tn60 | 196774122 | cyt | thy | 42 |
| 27 | ma0016fq93 | 196774333 | gua | ade | 43 |
| 28 | ma0016fq89 | 196774502 | gua | ade | 44 |
| 29 | ma0016fq94 | 196774823 | cyt | thy | 45 |
| 30 | ma0004tn62 | 196774965 | cyt | gua | 46 |
| 31 | ma0004tn31 | 196775596 | gua | ade | 47 |
| 32 | ma0016fq50 | 196776600 | ade | gua | 48 |
| 33 | ma0004tn30 | 196776877 | cyt | thy | 49 |
| 34 | ma0000xk01 | 196840068 | cyt | ade | 50 |
| 35 | ma0011 cv03 | 196840815 | gua | cyt | 51 |
| 36 | ma0016fq79 | 196841649 | gua | cyt | 52 |
| 37 | ma0004tn51 | 196841990 | cyt | ade | 53 |
| 38 | ma0004tn53 | 196843002 | cyt | gua | 54 |
| 39 | ma0000cm74 | 196843335 | cyt | gua | 55 |
| 40 | ma0000jt04 | 196844084 | cyt | thy | 56 |
| 41 | ma0016fq81 | 196851451 | thy | cyt | 57 |
| 42 | ma0016fq76 | 196853534 | gua | cyt | 58 |
| 43 | ma0016fq97 | 196853762 | cyt | thy | 59 |
| 44 | ma0004tn72 | 196880373 | cyt | thy | 60 |
| 45 | ma0004tn96 | 196985856 | cyt | ade | 61 |
| 46 | ma0016fr46 | 196985883 | cyt | thy | 62 |
| 47 | ma0011 rt36 | 196987284 | cyt | thy | 63 |
| 48 | ma0022vg84 | 196989025 | gua | thy | 64 |
| 49 | ma0001hr21 | 196989252 | gua | ade | 65 |
| 50 | ma0012wu17 | 196989377 | gua | ade | 66 |
| 51 | ma0001hr20 | 196989408 | ade | gua | 67 |
| 52 | ma0000sa77 | 197453646 | ade | gua | 68 |
| 53 | ma0004tp73 | 197453708 | ade | gua | 69 |
| 54 | ma61758s03 | 197453708 | thy | cyt | 70 |
| 55 | ma0000qw65 | 197454448 | gua | ade | 71 |
| 56 | ma61758s02 | 197454630 | thy | cyt | 72 |
| 57 | ma0016ft38 | 197454657 | thy | cyt | 73 |
| 58 | ma0016ft37 | 197454744 | thy | gua | 74 |
| 59 | ma0000wv70 | 197454780 | cyt | gua | 75 |
| 60 | ma0004tp85 | 197454833 | ade | gua | 76 |
| 61 | ma0012sk12 | 197455007 | cyt | thy | 77 |
| 62 | ma0022mm17 | 197455034 | thy | cyt | 78 |
| 63 | ma0004tp78 | 197456922 | thy | cyt | 79 |
| 64 | ma0022mm15 | 197457134 | ade | gua | 80 |
| 65 | ma0004tp79 | 197457214 | cyt | thy | 81 |
| 66 | ma0004tp82 | 197457351 | gua | ade | 82 |
| 67 | ma0016ft40 | 197457603 | gua | ade | 83 |
| 68 | ma0016ft28 | 197459188 | thy | cyt | 84 |
| 69 | ma0004tq11 | 197487965 | gua | ade | 85 |
| 70 | ma0004tq10 | 197488751 | cyt | thy | 86 |
| 71 | ma0012aq25 | 197489067 | gua | ade | 87 |
| 72 | ma61757s01 | 197524489 | gua | thy | 88 |
| 73 | ma0016ft65 | 197524855 | cyt | thy | 89 |
| 74 | ma0004tq05 | 197525193 | gua | ade | 90 |
| 75 | ma0004tq06 | 197525365 | gua | thy | 91 |
| 76 | ma61757s03 | 197525625 | thy | cyt | 92 |
| 77 | ma61757s04 | 197525990 | cyt | thy | 93 |
| 78 | ma0004tq13 | 197526621 | ade | gua | 94 |
| 79 | ma0016ft76 | 197526690 | gua | ade | 95 |
| 80 | ma0016ft75 | 197527582 | gua | ade | 96 |
| 81 | ma0022xj97 | 197527682 | cyt | gua | 97 |
| 82 | ma0004tq07 | 197528652 | cyt | thy | 98 |
| 83 | ma0016ft73 | 197556227 | gua | ade | 99 |
| 84 | ma0004tq16 | 197609686 | gua | thy | 100 |
| 85 | ma0016ft77 | 197609730 | ade | gua | 101 |
| 86 | ma0016ft86 | 197611692 | ade | gua | 102 |
| 87 | ma0010yr95 | 197611832 | gua | ade | 103 |
| 88 | ma0011 dy20 | 197611894 | gua | ade | 104 |
| 89 | ma61755s04 | 197613135 | ade | cyt | 105 |
| 90 | ma0016ft80 | 197613658 | ade | cyt | 106 |
| 91 | ma61755s03 | 197613658 | thy | gua | 107 |
| 92 | ma61755s02 | 197615089 | gua | ade | 108 |
| 93 | ma61755s01 | 197615461 | cyt | thy | 109 |
| 94 | ma0016ft81 | 197631560 | ade | gua | 110 |
| 95 | ma0016ft78 | 197631688 | gua | ade | 111 |
| 96 | ma0016ft83 | 197632590 | gua | ade | 112 |
| 97 | ma0004tq35 | 197632706 | ade | gua | 113 |
| 98 | ma0004tq36 | 197633370 | thy | cyt | 114 |
| 99 | ma0004tq32 | 197633860 | gua | ade | 115 |
| 100 | ma0022xj98 | 197638778 | thy | cyt | 116 |
| 101 | ma0004tq24 | 197638949 | thy | cyt | 117 |
| 102 | ma0004tq26 | 197639380 | gua | ade | 118 |
| 103 | ma0004tq30 | 197652023 | cyt | thy | 119 |
| 104 | ma0016ft97 | 197652478 | ade | gua | 120 |
| 105 | ma0004tq33 | 197653125 | gua | thy | 121 |
| 106 | ma0016ft92 | 197654542 | cyt | gua | 122 |
| 107 | ma0004tq39 | 197687270 | cyt | thy | 123 |
| 108 | ma0022mm26 | 197687524 | cyt | thy | 124 |
| 109 | ma0016fu15 | 197688212 | gua | cyt | 125 |
| 110 | ma0010yr98 | 197688445 | thy | cyt | 126 |
| 111 | ma0022mm25 | 197688492 | thy | cyt | 127 |
| 112 | ma0016ft98 | 197692991 | ade | gua | 128 |
| 113 | ma0004tq38 | 197692996 | thy | cyt | 129 |
| 114 | ma0004tq54 | 197694265 | thy | cyt | 130 |
| 115 | ma0016fu20 | 197695368 | cyt | thy | 131 |
| 116 | ma0016fu17 | 197695591 | ade | gua | 132 |
| 117 | ma0004tq57 | 197695857 | thy | cyt | 133 |
| 118 | ma0016fu11 | 197696192 | thy | ade | 134 |
| 119 | ma0016fu10 | 197696732 | gua | thy | 135 |
| 120 | ma0016fu09 | 197696762 | gua | ade | 136 |
| 121 | ma0004tq43 | 197697327 | gua | ade | 137 |
| 122 | ma0016fu01 | 197697514 | cyt | thy | 138 |
| 123 | ma0004tq42 | 197698249 | gua | ade | 139 |
| 124 | ma0016fu05 | 197698278 | gua | ade | 140 |
| 125 | ma0016ft99 | 197708137 | thy | cyt | 141 |
| 126 | ma0016fu07 | 197708334 | thy | ade | 142 |
| 127 | ma0010yt02 | 197758073 | thy | gua | 143 |
| 128 | ma0000gu42 | 197760175 | gua | ade | 144 |
| 129 | ma52981s02 | 197761254 | cyt | ade | 145 |
| 130 | ma52981s01 | 197761305 | ade | cyt | 146 |
| 131 | ma0004tq83 | 197776540 | thy | cyt | 147 |
| 132 | ma0004tq66 | 197777549 | ade | gua | 148 |
| 133 | ma0016fu32 | 197777618 | thy | ade | 149 |
| 134 | ma0011zk10 | 197778110 | ade | gua | 150 |
| 135 | ma0004tq62 | 197781849 | cyt | ade | 151 |
| 136 | ma0004tq68 | 197781961 | gua | ade | 152 |
| 137 | ma0004tq78 | 197784696 | ade | gua | 153 |
| 138 | ma0004tq59 | 197785166 | cyt | thy | 154 |
| 139 | ma0022mm29 | 197785270 | cyt | gua | 155 |
| 140 | ma53009s01 | 197786148 | gua | ade | 156 |
| 141 | ma0016fu41 | 197786155 | cyt | gua | 157 |
| 142 | ma0016fu35 | 197787768 | cyt | thy | 158 |
| 143 | ma0016fu36 | 197806056 | gua | ade | 159 |
| 144 | ma0004tq69 | 197806483 | cyt | thy | 160 |
| 145 | ma0016fu28 | 197812595 | gua | ade | 161 |
| 146 | ma0016fu26 | 197813589 | thy | cyt | 162 |
| 147 | ma0010yt03 | 197814082 | gua | ade | 163 |
| 148 | ma0016fu45 | 197840802 | thy | cyt | 164 |
| 149 | ma0022mm28 | 197840951 | gua | ade | 165 |
| 150 | ma0022xj99 | 197855989 | cyt | thy | 166 |
| 151 | ma0016fu43 | 197859323 | gua | cyt | 167 |
| 152 | ma0004tq81 | 197860711 | ade | cyt | 168 |
| 153 | ma0016fu29 | 197861373 | gua | ade | 169 |
| 154 | ma0022mm31 | 197895272 | ade | gua | 170 |
| 155 | ma0010yt04 | 197902823 | gua | ade | 171 |
| 156 | ma0004tr07 | 197902855 | cyt | thy | 172 |
| 157 | ma0012fz92 | 197902923 | cyt | thy | 173 |
| 158 | ma0001ac17 | 197903119 | cyt | ade | 174 |
| 159 | ma0012pg35 | 197903264 | thy | cyt | 175 |
| 160 | ma0000en22 | 197903302 | gua | cyt | 176 |
| 161 | ma0000en21 | 197903372 | cyt | thy | 177 |
| 162 | ma0016fu62 | 197903475 | cyt | thy | 178 |
| 163 | ma0004tq90 | 197903587 | gua | thy | 179 |
| 164 | ma08364s01 | 197903629 | gua | ade | 180 |
| 165 | ma0011zt60 | 197903716 | thy | cyt | 181 |
| 166 | ma0004tq98 | 197903816 | thy | gua | 182 |
| 167 | ma0016fu52 | 197904016 | thy | thy | 183 |
| 168 | ma0016fu55 | 197904655 | cyt | ade | 184 |
| 169 | ma0011 dy21 | 197906673 | ade | gua | 185 |
| 170 | ma0004tq88 | 197907566 | gua | ade | 186 |
| 171 | ma0022xk01 | 197907617 | ade | cyt | 187 |
| 172 | ma0016fu57 | 197907653 | thy | cyt | 188 |
| 173 | ma0010yt06 | 197907842 | thy | cyt | 189 |
| 174 | ma0001dg29 | 197909824 | ade | ade | 190 |
| 175 | ma0016fu59 | 197948546 | ade | gua | 191 |
| 176 | ma0016fu58 | 197948580 | cyt | thy | 192 |
| 177 | ma0004tr05 | 197948690 | cyt | ade | 193 |
| 178 | ma0004tr10 | 197948831 | gua | gua | 194 |
| 179 | ma0004tr03 | 197948879 | ade | gua | 195 |
| 180 | ma0004tq84 | 197973632 | cyt | cyt | 196 |
| 181 | ma0016fu56 | 197974493 | ade | gua | 197 |
| 182 | ma0011jn34 | 197994208 | thy | gua | 198 |
| 183 | ma0016fu80 | 198023432 | thy | cyt | 199 |
| 184 | ma0004tr23 | 198023573 | cyt | gua | 200 |

**Table 5: List of markers based in Figs. 2-5. Nucleotide positions are indicated for the respective SEQ ID NO.**

| Identifier | SEQ ID NO: | Position B73 (AGPv4) | SEQ ID NO: | Position restorer | polymorphism | |
|---|---|---|---|---|---|---|
| | | | | | maintainer | restorer |
| 1 | 1 | 35 | 2 | 35 | g | t |
| 2 | 1 | 404 | 2 | 404 | t | c |
| 3 | 1 | 444-452 | 2 | 443-444 | gggactttc | Deletion |
| 4 | 1 | 463 | 2 | 454 | c | t |
| 5 | 1 | 537 | 2 | 528 | g | c |
| 6 | 1 | 735 | 2 | 726 | g | a |
| 7 | 1 | 748-759 | 2 | 738-739 | tactttgtaaca | Deletion |
| 8 | 1 | 761 | 2 | 740 | t | a |
| 9 | 1 | 797 | 2 | 776 | a | g |
| 10 | 1 | 1048 | 2 | 1027 | g | t |
| 11 | 1 | 1056 | 2 | 1035 | a | c |
| 12 | 1 | 1065-1066 | 2 | 1045 | tc | Insertion a |
| 13 | 1 | 1072-1073 | 2 | 1053-1059 | cc | Insertion cttctcc |
| 14 | 1 | 1071 | 2 | 1058 | g | c |
| 15 | 1 | 1188 | 2 | 1175 | c | t |
| 16 | 1 | 1218 | 2 | 1208 | t | c |
| 17 | 1 | 1765 | 2 | 1757 | a | g |
| 18 | 1 | 1769 | 2 | 1761 | g | a |
| 19 | 1 | 1844 | 2 | 1836 | t | g |
| 20 | 1 | 1856 | 2 | 1848 | t | a |
| 21 | 1 | 2076 | 2 | 2068 | c | t |
| 22 | 1 | 2089 | 2 | 2081 | g | a |
| 23 | 1 | 2146 | 2 | 2138 | t | c |
| 24 | 1 | 2168-2169 | 2 | 2161 | tt | Insertion t |
| 25 | 1 | 2214 | 2 | 2207 | g | t |
| 26 | 1 | 2370 | 2 | 2362-2363 | c | deletion |
| 27 | 1 | 2582 | 2 | 2574 | c | g |
| 28 | 1 | 2632-2637 | 2 | 2624-2629 | tactgt | ccactg |
| 29 | 1 | 2641 | 2 | 2633 | a | t |
| 30 | 1 | 2643-2644 | 2 | 2635-2636 | cg | ac |
| 31 | 1 | 2696 | 2 | 2688 | t | c |
| 32 | 1 | 2738 | 2 | 2730 | c | a |
| 33 | 1 | 2843 | 2 | 2834-2835 | g | deletion |
| 34 | 1 | 2849 | 2 | 2840 | t | c |
| 35 | 1 | 2954-2955 | 2 | 2946-2947 | tt | Insertion tt |
| 36 | 1 | 3004 | 2 | 2997 | g | a |
| 37 | 1 | 3047 | 2 | 3040 | a | t |
| 38 | 1 | 3068 | 2 | 3061 | c | a |
| 39 | 1 | 3218 | 2 | 3211 | a | g |
| 40 | 5 | 486 | 6 | 486 | a | t |
| 41 | 5 | 611 | 6 | 611 | g | a |
| 42 | 5 | 638 | 6 | 638 | a | g |
| 43 | 5 | 689 | 6 | 689 | g | a |
| 44 | 5 | 812 | 6 | 812 | c | t |
| 45 | 5 | 865 | 6 | 865 | c | g |
| 46 | 5 | 901 | 6 | 901 | c | a |
| 47 | 5 | 988 | 6 | 988 | g | a |
| 48 | 5 | 1015 | 6 | 1015 | c | t |
| 49 | 5 | 1085 | 6 | 1085 | c | t |
| 50 | 5 | 1197 | 6 | 1197 | t | c |
| 51 | 5 | 1345 | 6 | 1345 | c | t |
| 52 | 5 | 1461 | 6 | 1461 | c | t |
| 53 | 5 | 1937 | 6 | 1937 | c | t |
| 54 | 5 | 1999 | 6 | 1999 | c | t |
| 55 | 5 | 2113-2115 | 6 | 2112-2113 | gca | Deletion |
| 56 | 5 | 2286 | 6 | 2283 | c | t |
| 57 | 5 | 2293-2297 | 6 | 2289-2290 | ctacg | Deletion |
| 58 | 5 | 2399 | 6 | 2391 | c | g |
| 59 | 5 | 2448-2450 | 6 | 2439-2440 | ctc | Deletion |
| 60 | 5 | 2822-2823 | 6 | 2810-2811 | tt | Deletion |
| 61 | 5 | 2856 | 6 | 2843 | g | a |
| 62 | 5 | 2926 | 6 | 2913 | a | g |
| 63 | 5 | 2998 | 6 | 2985 | t | c |
| 64 | 5 | 3029 | 6 | 3016 | t | c |
| 65 | 5 | 3085 | 6 | 3072 | t | c |
| 66 | 5 | 3102 | 6 | 3089 | t | c |
| 67 | 5 | 3112 | 6 | 3099 | c | a |
| 68 | 5 | 3120 | 6 | 3107 | t | g |
| 69 | 5 | 3168 | 6 | 3155 | t | a |
| 70 | 9 | 392-393 | 10 | 393-397 | tg | Insertion gtggt |
| 71 | 9 | 550 | 10 | 555 | g | a |
| 72 | 9 | 591 | 10 | 596 | t | g |
| 73 | 9 | 886-887 | 10 | 891-892 | ct | tc |
| 74 | 9 | 934 | 10 | 939 | a | g |
| 75 | 9 | 957 | 10 | 962 | t | c |
| 76 | 9 | 1097 | 10 | 1102 | c | t |
| 77 | 9 | 1130 | 10 | 1135 | c | t |
| 78 | 9 | 1295 | 10 | 1300 | a | g |
| 79 | 9 | 1462 | 10 | 1467 | t | c |
| 80 | 9 | 1467 | 10 | 1472 | t | g |
| 81 | 9 | 1541 | 10 | 1546 | g | a |
| 82 | 9 | 1584 | 10 | 1603 | g | t |
| 83 | 9 | 1614 | 10 | 1633 | c | t |
| 84 | 9 | 1713 | 10 | 1732 | c | t |
| 85 | 9 | 1774 | 10 | 1793 | a | g |
| 86 | 9 | 1795-1796 | 10 | 1815-1823 | tt | Insertion tttttgttt |
| 87 | 9 | 1815 | 10 | 1843 | a | c |
| 88 | 9 | 1894-1895 | 10 | 1923 | tt | Insertion t |
| 89 | 9 | 1910 | 10 | 1939 | a | t |
| 90 | 9 | 1954-1955 | 10 | 1984-1991 | gt | Insertion tttgacac |
| 91 | 9 | 2000 | 10 | 2037 | t | c |
| 92 | 9 | 2060 | 10 | 2097 | t | c |
| 93 | 9 | 2181 | 10 | 2218 | c | t |
| 94 | 9 | 2354 | 10 | 2391 | a | g |
| 95 | 9 | 2372 | 10 | 2409 | a | t |
| 96 | 9 | 2394-2399 | 10 | 2431-2434 | ctgttt | Substitution /deletion aaca |
| 97 | 9 | 2428 | 10 | 2463 | a | g |
| 98 | 9 | 2439-2440 | 10 | 2475-2476 | ct | Insertion tt |
| 99 | 13 | 651-652 | 14 | 652-654 | cc | Insertion cgc |
| 100 | 13 | 807 | 14 | 810 | t | c |

## Claims

1. A method for identifying a maize plant or plant part, comprising screening for the presence of (a haplotype associated with) a cytoplasmic male sterility (fertility) restorer locus on chromosome 3 (RF-03-01).

2. The method according to claim 1, wherein said locus comprises or is comprised in a region on chromosome 3 corresponding to positions 195629901 to 198023573 of B73 AGPv4, preferably corresponding to positions 197453646 to 197698278 of B73 AGPv4or a fragment thereof.

3. The method according to any of claims 1 to 2, wherein said locus comprises one or more of molecular marker(s) (alleles) of Table 4 or Table 5.

4. The method according to any of claims 1 to 3, wherein said locus comprises a polynucleic acid comprising one or more of SEQ ID NOs: 1, 5, 9, and 13 or one or more of SEQ ID NOs: 2, 6, 10, and 14.

5. The method according to any of claims 1 to 4, comprising screening for the presence of any one or more of SEQ ID NOs: 17 to 200.

6. The method according to any of claims 1 to 5, comprising screening for the presence of any one or more of SEQ ID NOs: 1, 5, 9, and 13 or one or more of SEQ ID NOs: 2, 6, 10, and 14.

7. An (isolated) polynucleic acid comprising one or more molecular marker (allele) of Table 4 or Table 5, or the complement or reverse complement of said polynucleic acid.

8. An (isolated) polynucleic acid comprising one or more of SEQ ID NOs: 1, 5, 9, and 13 or one or more of SEQ ID NOs: 2, 6, 10, and 14, or a fragment thereof.

9. An (isolated) polynucleic acid comprising at least 15 contiguous nucleotides comprised in a region corresponding to a region flanked by any of the indicated 5' and 3' positions of the table below and comprising the nucleotide corresponding to the position of the indicated SNP referenced to maize chromosome 3, B73 AGPv4
| 5' (-100 nt) | 5' (-50 nt) | SNP | 3' (+50 nt) | 3' (+100 nt) |
|---|---|---|---|---|
| 195629801 | 195629851 | 195629901 | 195629951 | 195630001 |
| 195639594 | 195639644 | 195639694 | 195639744 | 195639794 |
| 195677699 | 195677749 | 195677799 | 195677849 | 195677899 |
| 195678256 | 195678306 | 195678356 | 195678406 | 195678456 |
| 195680690 | 195680740 | 195680790 | 195680840 | 195680890 |
| 195732836 | 195732886 | 195732936 | 195732986 | 195733036 |
| 195733816 | 195733866 | 195733916 | 195733966 | 195734016 |
| 195783601 | 195783651 | 195783701 | 195783751 | 195783801 |
| 196069986 | 196070036 | 196070086 | 196070136 | 196070186 |
| 196198636 | 196198686 | 196198736 | 196198786 | 196198836 |
| 196244614 | 196244664 | 196244714 | 196244764 | 196244814 |
| 196653646 | 196653696 | 196653746 | 196653796 | 196653846 |
| 196693401 | 196693451 | 196693501 | 196693551 | 196693601 |
| 196702711 | 196702761 | 196702811 | 196702861 | 196702911 |
| 196703908 | 196703958 | 196704008 | 196704058 | 196704108 |
| 196703996 | 196704046 | 196704096 | 196704146 | 196704196 |
| 196704069 | 196704119 | 196704169 | 196704219 | 196704269 |
| 196704190 | 196704240 | 196704290 | 196704340 | 196704390 |
| 196705370 | 196705420 | 196705470 | 196705520 | 196705570 |
| 196706597 | 196706647 | 196706697 | 196706747 | 196706797 |
| 196706655 | 196706705 | 196706755 | 196706805 | 196706855 |
| 196707090 | 196707140 | 196707190 | 196707240 | 196707290 |
| 196707315 | 196707365 | 196707415 | 196707465 | 196707515 |
| 196707897 | 196707947 | 196707997 | 196708047 | 196708097 |
| 196773793 | 196773843 | 196773893 | 196773943 | 196773993 |
| 196774022 | 196774072 | 196774122 | 196774172 | 196774222 |
| 196774233 | 196774283 | 196774333 | 196774383 | 196774433 |
| 196774402 | 196774452 | 196774502 | 196774552 | 196774602 |
| 196774723 | 196774773 | 196774823 | 196774873 | 196774923 |
| 196774865 | 196774915 | 196774965 | 196775015 | 196775065 |
| 196775496 | 196775546 | 196775596 | 196775646 | 196775696 |
| 196776500 | 196776550 | 196776600 | 196776650 | 196776700 |
| 196776777 | 196776827 | 196776877 | 196776927 | 196776977 |
| 196839968 | 196840018 | 196840068 | 196840118 | 196840168 |
| 196840715 | 196840765 | 196840815 | 196840865 | 196840915 |
| 196841549 | 196841599 | 196841649 | 196841699 | 196841749 |
| 196841890 | 196841940 | 196841990 | 196842040 | 196842090 |
| 196842902 | 196842952 | 196843002 | 196843052 | 196843102 |
| 196843235 | 196843285 | 196843335 | 196843385 | 196843435 |
| 196843984 | 196844034 | 196844084 | 196844134 | 196844184 |
| 196851351 | 196851401 | 196851451 | 196851501 | 196851551 |
| 196853434 | 196853484 | 196853534 | 196853584 | 196853634 |
| 196853662 | 196853712 | 196853762 | 196853812 | 196853862 |
| 196880273 | 196880323 | 196880373 | 196880423 | 196880473 |
| 196985756 | 196985806 | 196985856 | 196985906 | 196985956 |
| 196985783 | 196985833 | 196985883 | 196985933 | 196985983 |
| 196987184 | 196987234 | 196987284 | 196987334 | 196987384 |
| 196988925 | 196988975 | 196989025 | 196989075 | 196989125 |
| 196989152 | 196989202 | 196989252 | 196989302 | 196989352 |
| 196989277 | 196989327 | 196989377 | 196989427 | 196989477 |
| 196989308 | 196989358 | 196989408 | 196989458 | 196989508 |
| 197453546 | 197453596 | 197453646 | 197453696 | 197453746 |
| 197453608 | 197453658 | 197453708 | 197453758 | 197453808 |
| 197454348 | 197454398 | 197454448 | 197454498 | 197454548 |
| 197454530 | 197454580 | 197454630 | 197454680 | 197454730 |
| 197454557 | 197454607 | 197454657 | 197454707 | 197454757 |
| 197454644 | 197454694 | 197454744 | 197454794 | 197454844 |
| 197454680 | 197454730 | 197454780 | 197454830 | 197454880 |
| 197454733 | 197454783 | 197454833 | 197454883 | 197454933 |
| 197454907 | 197454957 | 197455007 | 197455057 | 197455107 |
| 197454934 | 197454984 | 197455034 | 197455084 | 197455134 |
| 197456822 | 197456872 | 197456922 | 197456972 | 197457022 |
| 197457034 | 197457084 | 197457134 | 197457184 | 197457234 |
| 197457114 | 197457164 | 197457214 | 197457264 | 197457314 |
| 197457251 | 197457301 | 197457351 | 197457401 | 197457451 |
| 197457503 | 197457553 | 197457603 | 197457653 | 197457703 |
| 197459088 | 197459138 | 197459188 | 197459238 | 197459288 |
| 197487865 | 197487915 | 197487965 | 197488015 | 197488065 |
| 197488651 | 197488701 | 197488751 | 197488801 | 197488851 |
| 197488967 | 197489017 | 197489067 | 197489117 | 197489167 |
| 197524389 | 197524439 | 197524489 | 197524539 | 197524589 |
| 197524755 | 197524805 | 197524855 | 197524905 | 197524955 |
| 197525093 | 197525143 | 197525193 | 197525243 | 197525293 |
| 197525265 | 197525315 | 197525365 | 197525415 | 197525465 |
| 197525525 | 197525575 | 197525625 | 197525675 | 197525725 |
| 197525890 | 197525940 | 197525990 | 197526040 | 197526090 |
| 197526521 | 197526571 | 197526621 | 197526671 | 197526721 |
| 197526590 | 197526640 | 197526690 | 197526740 | 197526790 |
| 197527482 | 197527532 | 197527582 | 197527632 | 197527682 |
| 197527582 | 197527632 | 197527682 | 197527732 | 197527782 |
| 197528552 | 197528602 | 197528652 | 197528702 | 197528752 |
| 197556127 | 197556177 | 197556227 | 197556277 | 197556327 |
| 197609586 | 197609636 | 197609686 | 197609736 | 197609786 |
| 197609630 | 197609680 | 197609730 | 197609780 | 197609830 |
| 197611592 | 197611642 | 197611692 | 197611742 | 197611792 |
| 197611732 | 197611782 | 197611832 | 197611882 | 197611932 |
| 197611794 | 197611844 | 197611894 | 197611944 | 197611994 |
| 197613035 | 197613085 | 197613135 | 197613185 | 197613235 |
| 197613558 | 197613608 | 197613658 | 197613708 | 197613758 |
| 197614989 | 197615039 | 197615089 | 197615139 | 197615189 |
| 197615361 | 197615411 | 197615461 | 197615511 | 197615561 |
| 197631460 | 197631510 | 197631560 | 197631610 | 197631660 |
| 197631588 | 197631638 | 197631688 | 197631738 | 197631788 |
| 197632490 | 197632540 | 197632590 | 197632640 | 197632690 |
| 197632606 | 197632656 | 197632706 | 197632756 | 197632806 |
| 197633270 | 197633320 | 197633370 | 197633420 | 197633470 |
| 197633760 | 197633810 | 197633860 | 197633910 | 197633960 |
| 197638678 | 197638728 | 197638778 | 197638828 | 197638878 |
| 197638849 | 197638899 | 197638949 | 197638999 | 197639049 |
| 197639280 | 197639330 | 197639380 | 197639430 | 197639480 |
| 197651923 | 197651973 | 197652023 | 197652073 | 197652123 |
| 197652378 | 197652428 | 197652478 | 197652528 | 197652578 |
| 197653025 | 197653075 | 197653125 | 197653175 | 197653225 |
| 197654442 | 197654492 | 197654542 | 197654592 | 197654642 |
| 197687170 | 197687220 | 197687270 | 197687320 | 197687370 |
| 197687424 | 197687474 | 197687524 | 197687574 | 197687624 |
| 197688112 | 197688162 | 197688212 | 197688262 | 197688312 |
| 197688345 | 197688395 | 197688445 | 197688495 | 197688545 |
| 197688392 | 197688442 | 197688492 | 197688542 | 197688592 |
| 197692891 | 197692941 | 197692991 | 197693041 | 197693091 |
| 197692896 | 197692946 | 197692996 | 197693046 | 197693096 |
| 197694165 | 197694215 | 197694265 | 197694315 | 197694365 |
| 197695268 | 197695318 | 197695368 | 197695418 | 197695468 |
| 197695491 | 197695541 | 197695591 | 197695641 | 197695691 |
| 197695757 | 197695807 | 197695857 | 197695907 | 197695957 |
| 197696092 | 197696142 | 197696192 | 197696242 | 197696292 |
| 197696632 | 197696682 | 197696732 | 197696782 | 197696832 |
| 197696662 | 197696712 | 197696762 | 197696812 | 197696862 |
| 197697227 | 197697277 | 197697327 | 197697377 | 197697427 |
| 197697414 | 197697464 | 197697514 | 197697564 | 197697614 |
| 197698149 | 197698199 | 197698249 | 197698299 | 197698349 |
| 197698178 | 197698228 | 197698278 | 197698328 | 197698378 |
| 197708037 | 197708087 | 197708137 | 197708187 | 197708237 |
| 197708234 | 197708284 | 197708334 | 197708384 | 197708434 |
| 197757973 | 197758023 | 197758073 | 197758123 | 197758173 |
| 197760075 | 197760125 | 197760175 | 197760225 | 197760275 |
| 197761154 | 197761204 | 197761254 | 197761304 | 197761354 |
| 197761205 | 197761255 | 197761305 | 197761355 | 197761405 |
| 197776440 | 197776490 | 197776540 | 197776590 | 197776640 |
| 197777449 | 197777499 | 197777549 | 197777599 | 197777649 |
| 197777518 | 197777568 | 197777618 | 197777668 | 197777718 |
| 197778010 | 197778060 | 197778110 | 197778160 | 197778210 |
| 197781749 | 197781799 | 197781849 | 197781899 | 197781949 |
| 197781861 | 197781911 | 197781961 | 197782011 | 197782061 |
| 197784596 | 197784646 | 197784696 | 197784746 | 197784796 |
| 197785066 | 197785116 | 197785166 | 197785216 | 197785266 |
| 197785170 | 197785220 | 197785270 | 197785320 | 197785370 |
| 197786048 | 197786098 | 197786148 | 197786198 | 197786248 |
| 197786055 | 197786105 | 197786155 | 197786205 | 197786255 |
| 197787668 | 197787718 | 197787768 | 197787818 | 197787868 |
| 197805956 | 197806006 | 197806056 | 197806106 | 197806156 |
| 197806383 | 197806433 | 197806483 | 197806533 | 197806583 |
| 197812495 | 197812545 | 197812595 | 197812645 | 197812695 |
| 197813489 | 197813539 | 197813589 | 197813639 | 197813689 |
| 197813982 | 197814032 | 197814082 | 197814132 | 197814182 |
| 197840702 | 197840752 | 197840802 | 197840852 | 197840902 |
| 197840851 | 197840901 | 197840951 | 197841001 | 197841051 |
| 197855889 | 197855939 | 197855989 | 197856039 | 197856089 |
| 197859223 | 197859273 | 197859323 | 197859373 | 197859423 |
| 197860611 | 197860661 | 197860711 | 197860761 | 197860811 |
| 197861273 | 197861323 | 197861373 | 197861423 | 197861473 |
| 197895172 | 197895222 | 197895272 | 197895322 | 197895372 |
| 197902723 | 197902773 | 197902823 | 197902873 | 197902923 |
| 197902755 | 197902805 | 197902855 | 197902905 | 197902955 |
| 197902823 | 197902873 | 197902923 | 197902973 | 197903023 |
| 197903019 | 197903069 | 197903119 | 197903169 | 197903219 |
| 197903164 | 197903214 | 197903264 | 197903314 | 197903364 |
| 197903202 | 197903252 | 197903302 | 197903352 | 197903402 |
| 197903272 | 197903322 | 197903372 | 197903422 | 197903472 |
| 197903375 | 197903425 | 197903475 | 197903525 | 197903575 |
| 197903487 | 197903537 | 197903587 | 197903637 | 197903687 |
| 197903529 | 197903579 | 197903629 | 197903679 | 197903729 |
| 197903616 | 197903666 | 197903716 | 197903766 | 197903816 |
| 197903716 | 197903766 | 197903816 | 197903866 | 197903916 |
| 197903916 | 197903966 | 197904016 | 197904066 | 197904116 |
| 197904555 | 197904605 | 197904655 | 197904705 | 197904755 |
| 197906573 | 197906623 | 197906673 | 197906723 | 197906773 |
| 197907466 | 197907516 | 197907566 | 197907616 | 197907666 |
| 197907517 | 197907567 | 197907617 | 197907667 | 197907717 |
| 197907553 | 197907603 | 197907653 | 197907703 | 197907753 |
| 197907742 | 197907792 | 197907842 | 197907892 | 197907942 |
| 197909724 | 197909774 | 197909824 | 197909874 | 197909924 |
| 197948446 | 197948496 | 197948546 | 197948596 | 197948646 |
| 197948480 | 197948530 | 197948580 | 197948630 | 197948680 |
| 197948590 | 197948640 | 197948690 | 197948740 | 197948790 |
| 197948731 | 197948781 | 197948831 | 197948881 | 197948931 |
| 197948779 | 197948829 | 197948879 | 197948929 | 197948979 |
| 197973532 | 197973582 | 197973632 | 197973682 | 197973732 |
| 197974393 | 197974443 | 197974493 | 197974543 | 197974593 |
| 197994108 | 197994158 | 197994208 | 197994258 | 197994308 |
| 198023332 | 198023382 | 198023432 | 198023482 | 198023532 |
| 198023473 | 198023523 | 198023573 | 198023623 | 198023673 |
or the complement, or reverse complement of said polynucleic acid.

10. The (isolated) polynucleic acid according to any of claims 7 to 9, comprising at most 500 nucleotides, preferably at most 200 nucleotides, more preferably at most 100 nucleotides, most preferably at most 50 nucleotides, such as at most 35 nucleotides.

11. The (isolated) polynucleic acid according to any of claims 7 to 10, which is a primer or a probe.

12. The (isolated) polynucleotide according to any of claims 7 to 11, which is an allele-specific primer or probe, preferably a KASP primer.

13. A maize plant or plant part comprising one or more molecular marker (allele) of Table 4 or Table 5, the locus as defined in any of claims 1 to 6, and/or a polynucleic acid as defined in any of claims 7 to 12.

14. A method for generating a maize plant or plant part, comprising introducing in the genome of said plant or plant part a locus as defined in any of claims 1 to 6 or a polynucleic acid as defined in any of claims 7 to 12, or a (functional) fragment thereof.

15. Use of a polynucleic acid according to any of claims 7-12 for identifying a maize plant or plant part or for generating a maize plant or plant part.
